# EUROPEAN PATENT SPECIFICATION

(11) **EP 1 931 390 B1**
(45) Date of publication and mention of the grant of the patent: **25.07.2012**
(21) Application number: 06825440.8
(22) Date of filing: 04.10.2006
(51) Int. Cl.: A61K 39/00

(54) **COMPOSITIONS AND METHODS FOR TREATMENT OF AUTOIMMUNE DISEASE**
ZUSAMMENSETZUNGEN UND VERFAHREN ZUR BEHANDLUNG VON AUTOIMMUNKRANKHEIT
COMPOSITIONS ET METHODES DE TRAITEMENT DE MALADIES AUTO-IMMUNES

(30) Priority: 05.10.2005 US 724203 P
(43) Date of publication of application: 18.06.2008
(73) Proprietor: Bayhill Therapeutics, Inc., Palo Alto, CA 94303 (US)
(72) Inventor: SOLVASON, Nanette, Palo Alto, California 94306 (US); LEVITEN, Michael, Palo Alto, California 94306 (US); GARREN, Hideki, Palo Alto, California 94306 (US)
(74) Representative: Brasnett, Adrian Hugh
(86) International application number: PCT/US2006/038776
(87) International publication number: WO 2007/044394

(56) References cited:
- WO-A-00/46147
- WO-A-03/045316
- WO-A-2005/035771
- WO-A1-00/02591
- WO-A1-03/082269
- US-A1- 2002 068 715
- US-A1- 2003 148 983
- US-B1- 6 274 136
- KARGES W ET AL: "INDUCTION OF AUTOIMMUNE DIABETES THROUGH INSULIN (BUT NOT GAD65) DNA VACCINATION IN NONOBESE DIABETIC AND IN RIP-B7.1 MICE" DIABETES, AMERICAN DIABETES ASSOCIATION, US, vol. 51, no. 11, 1 November 2002 (2002-11-01), pages 3237-3244, XP009031795 ISSN: 0012-1797
- URBANEK-RUIZ I ET AL: "Immunization with DNA encoding an immunodominant peptide of insulin prevents diabetes in NOD mice" CLINICAL IMMUNOLOGY, ACADEMIC PRESS, US, vol. 100, no. 2, 1 August 2001 (2001-08-01), pages 164-171, XP002964169 ISSN: 1521-6616
- PRUD'HOMME GÉRALD J: "Prevention of autoimmune diabetes by DNA vaccination", EXPERT REVIEW OF VACCINES, FUTURE DRUGS, LONDON, GB, vol. 2, no. 4, 1 August 2003 (2003-08-01), pages 533-540, XP009111994, ISSN: 1476-0584
- B Balasa: "Vaccination with Glutamic Acid Decarboxylase Plasmid DNA Protects Mice from Spontaneous Autoimmune Diabetes and B7/CD28 Costimulation Circumvents That Protection", Clinical Immunology, vol. 99, no. 2, 1 May 2001 (2001-05-01), pages 241-252, XP55005429, ISSN: 1521-6616, DOI: 10.1006/clim.2001.5012
- YEW N S ET AL: "OPTIMIZATION OF PLASMID VECTORS FOR HIGH-LEVEL EXPRESSION IN LUNG EPITHELIAL CELLS", HUMAN GENE THERAPY, MARY ANN LIEBERT, NEW YORK ,NY, US, vol. 8, no. 5, 20 March 1997 (1997-03-20), pages 575-584, XP000940966, ISSN: 1043-0342
- KAPPOS ET AL: "Induction of a non-encephalitogenic type 2 T helper-cell autoimmune response in multiple sclerosis after administration of an altered peptide ligand in a placebo-controlled, randomized phase II trial", NATURE MEDICINE, vol. 6, no. 10, 1 October 2000 (2000-10-01), pages 1176-1182, XP55005430, ISSN: 1078-8956, DOI: 10.1038/80525
- BIELEKOVA BIBIANA ET AL: "Encephalitogenic potential of the myelin basic protein peptide (amino acids 83-99) in multiple sclerosis: Results of a phase II clinical trial with an altered peptide ligand", NATURE MEDICINE, NATURE PUBLISHING GROUP, NEW YORK, NY, US, vol. 6, no. 10, 1 October 2000 (2000-10-01), pages 1167-1175, XP002352505, ISSN: 1078-8956, DOI: 10.1038/80516
- Claude P. Genain ET AL: Nature Medicine, vol. 6, no. 10, 1 October 2000 (2000-10-01), pages 1098-1100, XP55005431, ISSN: 1078-8956, DOI: 10.1038/80424

## Description

### BACKGROUND OF THE INVENTION

### 1. Field of the Invention.

The present invention relates to the fields of immunology and medicine. The present invention enables methods and compositions for treating or preventing disease in a subject associated with one or more self-protein(s), -polypeptide(s), or peptide(s) present in the subject and involved in a non-physiological state. More particularly the present invention relates to improved methods and compositions for treating and preventing autoimmune disease comprising DNA vaccination with modified self-vectors encoding and capable of expressing self-polypeptides comprising one or more autoantigenic epitopes associated with an autoimmune disease.

### 2. Background.

### Autoimmune Disease and Modulation of the Immune Response

Autoimmune disease is any disease caused by immune cells that become misdirected at healthy cells and/or tissues of the body. Autoimmune disease affects 3% of the U.S. population and likely a similar percentage of the industrialized world population (Jacobson et al., Clin Immunol Immunopathol 84, 223-43, 1997). Autoimmune diseases are characterized by T and B lymphocytes that aberrantly target self-proteins, -polypeptides, - peptides, and/or other self-molecules causing injury and or malfunction of an organ, tissue, or cell-type within the body (for example, pancreas, brain, thyroid or gastrointestinal tract) to cause the clinical manifestations of the disease (Marrack et al., Nat Med 7, 899-905, 2001). Autoimmune diseases include diseases that affect specific tissues as well as diseases that can affect multiple tissues. This may, in part, for some diseases depend on whether the autoimmune responses are directed to an antigen confined to a particular tissue or to an antigen that is widely distributed in the body. The characteristic feature of tissue-specific autoimmunity is the selective targeting of a single tissue or individual cell type. Nevertheless, certain autoimmune diseases that target ubiquitous self-proteins can also affect specific tissues. For example, in polymyositis the autoimmune response targets the ubiquitous protein histidyl-tRNA synthetase, yet the clinical manifestations primarily involved are autoimmune destruction of muscle.

The immune system employs a highly complex mechanism designed to generate responses to protect mammals against a variety of foreign pathogens while at the same time preventing responses against self-antigens. In addition to deciding whether to respond (antigen specificity), the immune system must also choose appropriate effector functions to deal with each pathogen (effector specificity). A cell critical in mediating and regulating these effector functions is the CD4⁺ T cell. Furthermore, it is the elaboration of specific cytokines from CD4⁺ T cells that appears to be the major mechanism by which T cells mediate their functions. Thus, characterizing the types of cytokines made by CD4⁺ T cells as well as how their secretion is controlled is extremely important in understanding how the immune response is regulated.

The characterization of cytokine production from long-term mouse CD4⁺ T cell clones was first published more than 10 years ago (Mosmann et al., J. Immunol. 136:2348-2357, 1986). In these studies, it was shown that CD4⁺ T cells produced two distinct patterns of cytokine production, which were designated T helper 1 (Th1) and T helper 2 (Th2). Th1 cells were found to predominantly produce interleukin-2 (IL-2), interferon-γ (IFN-γ) and lymphotoxin (LT), while Th2 clones predominantly produced IL-4, IL-5, IL-6, and IL-13 (Cherwinski et al., J. Exp. Med. 169:1229-1244, 1987). Somewhat later, additional cytokines, IL-9 and IL-10, were isolated from Th2 clones (Van Snick et al., J. Exp. Med. 169:363-368, 1989) (Fiorentino et al., J. Exp. Med. 170:2081-2095, 1989). Finally, additional cytokines, such as IL-3, granulocyte macrophage colony-stimulating factor (GM-CSF), and tumor necrosis factor-α (TNF-α) were found to be secreted by both Th1 and Th2 cells.

Autoimmune disease encompasses a wide spectrum of diseases that can affect many different organs and tissues within the body as outlined in Table 1 below. (*See e.g*., Paul, W.E. (1999) Fundamental Immunology, Fourth Edition, Lippincott-Raven, New York.)

**Table 1. Primary Organs and Tissues Targeted in Autoimmune Disease**

| **Primary Organ(s) Targeted** | **Disease** |
|---|---|
| Thyroid | Hashimoto's Disease |
| Thyroid | Primary myxodaema |
| Thyroid | Thyrotoxicosis |
| Stomach | Pernicious anemia |
| Stomach | Atrophic gastritis |
| Adrenal glands | Addison's disease |
| Pancreatic islets | Insulin dependent diabetes mellitus |
| Kidneys | Goodpasture's syndrome |
| Neuromuscular junction | Myasthenia gravis |
| Leydig cells | Male infertility |
| Skin | Pemphigus vulgaris |
| Skin | Pemphioid |
| Eyes | Sympathetic ophthalmia |
| Eyes | Phacogenic uveitis |
| Brain | Multiple sclerosis |
| red blood cells | Hemolytic anemia |
| Platelets | Idiopathic thrombocytopenic purpura |
| White blood cells | Idiopathic leucopenia |
| Biliary tree | Primary biliary cirrhosis |
| Bowel | Ulcerative colitis |
| Arteries | Atherosclerosis |
| Salivary and lacrimal glands | Sjogren's syndrome |
| Synovial joints | Rheumatoid arthritis |
| Muscle | Polymyositis |
| Muscle and skin | Dermatomyositis |
| Skin | Scleroderma |
| skin, joints, muscle, blood cells | Mixed connective tissue disease |
| Clotting factors | Anti-phospholipid disease |
| Skin | Discoid lupus erythematosus |
| skin, joints, kidneys, brain, blood cells | Systemic lupus erythematosus (SLE) |

Current therapies for human autoimmune disease, include glucocorticoids, cytotoxic agents, and recently developed biological therapeutics. In general, the management of human systemic autoimmune disease is empirical and unsatisfactory. For the most part, broadly immunosuppressive drugs, such as corticosteroids, are used in a wide variety of severe autoimmune and inflammatory disorders. In addition to corticosteroids, other immunosuppressive agents are used in management of the systemic autoimmune diseases. Cyclophosphamide is an alkylating agent that causes profound depletion of both T- and B-lymphocytes and impairment of cell-mediated immunity. Cyclosporine, tacrolimus, and mycophenolate mofetil are natural products with specific properties of T-lymphocyte suppression, and they have been used to treat SLE, RA and, to a limited extent, in vasculitis and myositis. These drugs are associated with significant renal toxicity. Methotrexate is also used as a "second line" agent in RA, with the goal of reducing disease progression. It is also used in polymyositis and other connective-tissue diseases. Other approaches that have been tried include monoclonal antibodies intended to block the action of cytokines or to deplete lymphocytes. (Fox, Am. J. Med; 99:82-88 1995). Treatments for multiple sclerosis (MS) include interferon (β and copolymer 1, which reduce relapse rate by 20-30% and only have a modest impact on disease progression. MS is also treated with immunosuppressive agents including methylprednisolone, other steroids, methotrexate, cladribine and cyclophosphamide. These immunosuppressive agents have minimal efficacy in treating MS. Current therapy for rheumatoid arthritis (RA) utilizes agents that non-specifically suppress or modulate immune function such as methotrexate, sulfasalazine, hydroxychloroquine, leuflonamide, prednisone, as well as the recently developed TNFα antagonists etanercept and infliximab (Moreland et al., J Rheumatol 28, 1431-52., 2001). Etanercept and infliximab globally block TNFα, making patients more susceptible to death from sepsis, aggravation of chronic mycobacterial infections, and development of demyelinating events.

In the case of organ-specific autoimmunity, a number of different therapeutic approaches have been tried. Soluble protein antigens have been administered systemically to inhibit the subsequent immune response to that antigen. Such therapies include delivery of myelin basic protein, its dominant peptide, or a mixture of myelin proteins to animals with experimental autoimmune encephalomyelitis and humans with multiple sclerosis (Brocke et al., Nature 379, 343-6, 1996; Critchfield et al., Science 263, 1139-43., 1994; Weiner et al., Annu Rev Immunol 12, 809-37, 1994), administration of type II collagen or a mixture of collagen proteins to animals with collagen-induced arthritis and humans with rheumatoid arthritis (Gumanovskaya et al., Immunology 97, 466-73., 1999); (McKown et al., Arthritis Rheum 42, 1204-8., 1999); (Trentham et al., Science 261, 1727-30., 1993), delivery of insulin to animals and humans with autoimmune diabetes (Pozzilli and Gisella Cavallo, Diabetes Metab Res Rev 16, 306-7., 2000), and delivery of S-antigen to animals and humans with autoimmune uveitis (Nussenblatt et al., Am J Ophthalmol 123, 583-92., 1997). A problem associated with this approach is T-cell unresponsiveness induced by systemic injection of antigen. Another approach is the attempt to design rational therapeutic strategies for the systemic administration of a peptide antigen based on the specific interaction between the T-cell receptors and peptides bound to MHC molecules. One study using the peptide approach in an animal model of diabetes, resulted in the development of antibody production to the peptide (Hurtenbach et al., J Exp. Med 177:1499, 1993). Another approach is the administration of T cell receptor (TCR) peptide immunization. (*See, e.g.,* Vandenbark et al., Nature 341:541, 1989). Still another approach is the induction of oral tolerance by ingestion of peptide or protein antigens. (*See, e.g.,* Weiner, Immmunol Today, 18:335 1997).

Immune responses are currently altered by delivering polypeptides, alone or in combination with adjuvants (immunostimulatory agents). For example, the hepatitis B virus vaccine contains recombinant hepatitis B virus surface antigen, a non-self antigen, formulated in aluminum hydroxide, which serves as an adjuvant. This vaccine induces an immune response against hepatitis B virus surface antigen to protect against infection. An alternative approach involves delivery of an attenuated, replication deficient, and/or non-pathogenic form of a virus or bacterium, each non-self antigens, to elicit a host protective immune response against the pathogen. For example, the oral polio vaccine is composed of a live attenuated virus, a non-self antigen, which infects cells and replicates in the vaccinated individual to induce effective immunity against polio virus, a foreign or non-self antigen, without causing clinical disease. Alternatively, the inactivated polio vaccine contains an inactivated or 'killed' virus that is incapable of infecting or replicating and is administered subcutaneously to induce protective immunity against polio virus.

### DNA Vaccination/Polynucleotide Therapy

Polynucleotide therapy, or DNA vaccination, is an efficient method to induce immunity against foreign pathogens (Davis, 1997; Hassett and Whitton, 1996; and Ulmer et al., 1996) and cancer antigens (Stevenson et al., 2004) and to modulate autoimmune processes (Waisman et al., 1996). Following intramuscular injection, plasmid DNA is taken up by, for example, muscle cells allowing for the expression of the encoded polypeptide (Wolff et al., 1992) and the mounting of a long-lived immune response to the expressed proteins (Hassett et al., 2000). In the case of autoimmune disease, the effect is a shift in an ongoing immune response to suppress autoimmune destruction and is believed to include a shift in self-reactive lymphocytes from a Th1 to a Th2-type response. The modulation of the immune response may not be systemic but occur only locally at the target organ under autoimmune attack.

Administration of a polynucleotide encoding a self protein, polypeptide or peptide formulated in precipitation- and/or transfection-facilitating agents or using viral vectors differs from "gene therapy." Gene therapy is the delivery of a polynucleotide to provide expression of a protein or peptide, to replace a defective or absent protein or peptide in the host and/or to augment a desired physiologic function. Gene therapy includes methods that result in the integration of DNA into the genome of an individual for therapeutic purposes. Examples of gene therapy include the delivery of DNA encoding clotting factors for hemophilia, adenosine deaminase for severe combined immunodeficiency, low-density lipoprotein receptor for familial hypercholesterolemia, glucocerebrosidase for Gaucher's disease, α₁-antitrypsin for α₁-antitrypsin deficiency, α- or β-globin genes for hemoglobinopathies, and chloride channels for cystic fibrosis (Verma and Somia, Nature 389, 239-42, 1997).

Investigators have described DNA therapies encoding immune molecules to treat autoimmune diseases. Such DNA therapies include DNA encoding the antigen-binding regions of the T cell receptor to alter levels of autoreactive T cells driving the autoimmune response (Waisman et al., Nat Med 2:899-905, 1996) (U.S. Patent 5,939,400). DNA encoding autoantigens were attached to particles and delivered by gene gun to the skin to prevent multiple sclerosis and collagen induced arthritis. (International Patent Application Publication Nos WO 97/46253; Ramshaw et al. Immunol. and Cell Bio. 75:409-413, 1997). DNA encoding adhesion molecules, cytokines (*e.g.,* TNFα), chemokines (e.g., C-C chemokines), and other immune molecules (e.g., Fas-ligand) have been used in animal models of autoimmune disease (Youssef et al., J Clin Invest 106:361-371, 2000); (Wildbaum et al:, J Clin Invest 106:671-679, 2000); (Wildbaum et al, J Immunol 165:5860-5866, 2000); (Wildbaum et al., J Immunol 161:6368-7634, 1998); (Youssef et al., J Autoimmun 13:21-9, 1999).

Methods for treating autoimmune disease by administering a nucleic acid encoding one or more autoantigens are described in International Patent Application Nos. WO 00/53019, WO 2003/045316, and WO 2004/047734. While these methods have been successful, further improvements are still needed.
Karges et al, Diabetes, vol 51, no 11,1 November 2002, pages 3237-3244 relates to the induction of autoimmune diabetes through insulin (but not GAD65) DNA vaccination in nonobese diabetic and in RIP-B7 1 mice. WO00/46147 relates to DNA vaccines and adjuvants for these vaccines. Balasa et al, Clinical Immunology, vol. 99 no 2, pages 241-252, relates to vaccination of mice with GAD plasmid DNA. WO03/082269 relates to the use of statins and a second immunomodulatory agent for treating an autoimmune disease. WO00/02591 relates to the use of calcium phosphate as an adjuvant for DNA vaccines for the treatment of autoimmune diseases.

### SUMMARY OF THE INVENTION

The present invention provides a high expression self-vector (HESV) comprising in operative combination: (a) CMV promoter and early enhancer; (b) a β-globin/lg chimeric intron; (c) a polynucleotide encoding one or more autoantigens targeted in insulin dependent diabetes mellitus (IDDM), said autoantigen being selected from insulin, proinsulin and preproinsulin; (d) an immune modulatory sequence selected from the group consisting of 5'-Purine-Pyrimidine-[X]-[Y]-Pyrimidine-Pyrimidine-3' or 5'-Purine-Purine-[X]-[Y]-Pyrimidine-Pyrimidine-3', wherein X and Y are any naturally occurring or synthetic nucleotide, except that X and Y cannot be cytosine-guanine; and (e) a bovine growth hormone polyadenylation signal sequence and transcription terminator, for use in a method of treating IDDM in a subject with clinical or diagnostic symptoms of IDDM.

Further embodiments of the invention are defined by the accompanying claims.

The self-vector may allow for an extracellular or secreted autoantigen associated with the autoimmune disease to be encoded and expressed as an intracellular and non-secreted polypeptide.

In non-mutually exclusive embodiments, the improved use of the invention includes administering to a subject an effective amount of the HESV. A modified self-vector that does not secrete a self-protein(s), -polypeptide(s), or -peptide(s) associated with an extracellular or secreted autoantigens is referred to herein as a non-secreted self-vector (N-SSV).
In preferred embodiments the non-secreted version of a secreted self-polypeptide encoded by a N-SSV is lacking a signal sequence. In certain embodiments the N-SSV encodes a a non-secreted version of preproinsulin, proinsulin (e.g., SEQ ID NO: 2), that is lacking a signal sequence.
A modified self-vector that expresses a secreted or non-membrane bound self-protein(s), -polypeptide(s), or - peptide(s) associated with a membrane associated or intracellular autoantigen is referred to herein as a secreted self-vector (SSV). A SSV comprises a polynucleotide encoding and capable of expressing a membrane associated or intracellular self-polypeptide associated with an autoimmune disease and a modification to express the secreted or non-membrane bound self-polypeptide from a host cell.

The present invention may be used to provide methods and compositions for treating insulin-dependent diabetes mellitus,
In some embodiments the HESV is generated by one or more of the following modifications: using a more ideal consensus kozak sequence, optimizing codon usage, inclusion of introns or combinations of two or more of the foregoing modifications.

In some embodiments the self-polypeptide encoded by a N-SSV is selected from the group consisting of preproinsulin, proinsulin (e.g., SEQ ID NO: 2) and insulin. In preferred embodiments the N-SSV administered to treat or prevent IDDM encodes a non-secreted version of preproinsulin (i.e., proinsulin, SEQ ID NO: 2) in which the signal sequence of preproinsulin is removed.

In certain variations, the uses and compositions for treating or preventing an autoimmune disease further comprise the administration of the modified self-vector in combination with other substances, such as, for example, polynucleotides comprising an immune modulatory sequence, pharmacological agents, adjuvants, cytokines, or vectors encoding cytokines.
In another embodiment delivery of a modified self-vector is coupled with co-administration of an expression vector encoding a Th2 cytokine selected from the group consisting of IL-4, IL-10, and IL-13.

### DEFINITIONS

Unless defined otherwise, all technical and scientific terms used herein have the same meaning as commonly understood by those of ordinary skill in the art to which this invention belongs. The following references provide one of skill with a general definition of many of the terms used herein: Hale and Margham, The Harper Collins Dictionary of Biology (HarperPerennial, 1991); King and Stansfield, A Dictionary of Genetics (Oxford University Press, 4th ed. 1990); Stedman's Medical Dictionary (Lippincott Williams & Wilkins, 27th ed. 2000); and Hawley's Condensed Chemical Dictionary (John Wiley & Sons, 13th ed. 1997). As used herein, the following terms and phrases have the meanings ascribed to them unless specified otherwise.

The terms "a," "an," and "the" include plural referents unless the context clearly dictates otherwise.

The terms "polynucleotide" and "nucleic acid" refer to a polymer composed of a multiplicity of nucleotide units (ribonucleotide or deoxyribonucleotide or related structural variants) linked via phosphodiester bonds. A polynucleotide or nucleic acid can be of substantially any length, typically from about six (6) nucleotides to about 10⁹ nucleotides or larger. Polynucleotides and nucleic acids include RNA, DNA, synthetic fonns, and mixed polymers, both sense and antisense strands, double- or single-stranded, and can also be chemically or biochemically modified or can contain non-natural or derivatized nucleotide bases, as will be readily appreciated by the skilled artisan. Such modifications include, for example, labels, methylation, substitution of one or more of the naturally occurring nucleotides with an analog, internucleotide modifications such as uncharged linkages (*e.g*., methyl phosphonates, phosphotriesters, phosphoamidates, carbamates, and the like), charged linkages (*e.g*., phosphorothioates, phosphorodithioates, and the like), pendent moieties (*e.g*., polypeptides), intercalators (*e.g*., acridine, psoralen, and the like), chelators, alkylators, and modified linkages (*e.g*., alpha anomeric nucleic acids, and the like). Also included are synthetic molecules that mimic polynucleotides in their ability to bind to a designated sequence via hydrogen bonding and other chemical interactions. Such molecules are known in the art and include, for example, those in which peptide linkages substitute for phosphate linkages in the backbone of the molecule.

The terms "intron" or "intronic sequence" as used herein refers to intervening polynucleotide sequences within a gene or portion of a gene present in a self-vector that is situated upstream of or between "exons", polynucleotide sequences that are retained during RNA processing and most often code for a polypeptide. Introns do not function in coding for protein synthesis and are spliced out of a RNA before it is translated into a polypeptide.

"Splicing" refers to the mechanism by which a single functional RNA molecule is generated by the removal of introns and juxtaposition of exons during processing of the primary transcript, or preRNA. Consensus sequences are present at intron-exon junctions that define the 5' end, or donor site, of an intron and the 3' end, or acceptor site, and at a branchpoint site located approximately 20-50 basepairs upstream of the acceptor site within the intron sequence. Most introns start from the sequence GU and end with the sequence AG (in the 5' to 3' direction) with a branchpoint site approximating CU(A/G)A(C/U), where A is conserved in all genes. These sequences signal for the looping out of the intron and its subsequent removal.

The term "promoter" is used here to refer to the polynucleotide region recognized by RNA polymerases for the initiation of RNA synthesis, or "transcription". Promoters are one of the functional elements of self-vectors that regulate the efficiency of transcription and thus the level of protein expression of a self-polypeptide encoded by a self-vector. Promoters can be "constitutive", allowing for continual transcription of the associated gene, or "inducible", and thus regulated by the presence or absence of different substances in the environment. Additionally, promoters can also either be general, for expression in a broad range of different cell types, or cell-type specific, and thus only active or inducible in a particular cell type, such as a muscle cell. Promoters controlling transcription from vectors maybe obtained from various sources, for example, the genomes of viruses such as: polyoma, simian virus 40 (SV40), adenovirus, retroviruses, hepatitis B virus and preferably cytomegalovirus, or from heterologous mammalian promoters, e.g. b-actin promoter. The early and late promoters of the SV 40 virus are conveniently obtained as is the immediate early promoter of the human cytomegalovirus.

"Enhancer" refers to cis-acting polynucleotide regions of about from 10-300 basepairs that act on a promoter to enhance transcription from that promoter. Enhancers are relatively orientation and position independent and can be placed 5' or 3' to the transcription unit, within introns, or within the coding sequence itself.

A "terminator sequence" as used herein means a polynucleotide sequence that signals the end of DNA transcription to the RNA polymerase. Often the 3' end of a RNA generated by the terminator sequence is then processed considerably upstream by polyadenylation. "Polyadenylation" is used to refer to the non-templated addition of about 50 to about 200 nucleotide chain of polyadenylic acid (polyA) to the 3' end of a transcribed messenger RNA. The "polyadenylation signal" (AAUAAA) is found within the 3' untranslated region (UTR) of a mRNA and specifies the site for cleavage of the transcript and addition of the polyA tail. Transcription termination and polyadenylation are functionally linked and sequences required for efficient cleavage/polyadenylation also constitute important elements of termination sequences (Connelly and Manley, 1988).

"Oligonucleotide," as used herein refers, to a subset of polynucleotides of from about 6 to about 175 nucleotides or more in length. Typical oligonucleotides are up to about 100 nucleotides in length. Oligonucleotide refers to both oligoribonucleotides and to oligodeoxyribonucleotides, hereinafter referred to ODNs. ODNs include oligonucleosides and other organic base containing polymers. Oligonucleotides can be obtained from existing nucleic acid sources, including genomic DNA, plasmid DNA, viral DNA and cDNA, but are typically synthetic oligonucleotides produced by oligonucleotide synthesis. Oligonucleotides can be synthesized on an automated oligonucleotide synthesizer (for example, those manufactured by Applied BioSystems (Foster City, Calif.)) according to specifications provided by the manufacturer.

The terms "DNA vaccination", "DNA immunization", and "polynucleotide therapy" are used interchangeably herein and refer to the administration of a polynucleotide to a subject for the purpose of modulating an immune response. "DNA vaccination" with plasmids expressing foreign microbial antigens is a well known method to induce protective antiviral or antibacterial immunity (Davis, 1997; Hassett and Whitton, 1996; and Ulmer et al., 1996). For the purpose of the present invention, "DNA vaccination", "DNA immunization", or "polynucleotide therapy" refers to the administration of polynucleotides encoding one or more self-polypeptides that include one or more autoantigenic epitopes associated with a disease. The "DNA vaccination" serves the purpose of modulating an ongoing immune response to suppress autoimmune destruction for the treatment or prevention of an autoimmune disease. Modulation of an immune response in reaction to "DNA vaccination" may include shifting self-reactive lymphocytes from a Th1 to a Th2-type response. The modulation of the immune response may occur systemically or only locally at the target organ under autoimmune attack.

"Self-vector" means one or more vector(s) which taken together include a polynucleotide encoding one or more self-protein(s), -polypeptide(s), or -peptide(s). The self-protein(s), -polypeptide(s), or -peptide(s) coding sequence is inserted into an appropriate expression vector. Once the polynucleotide encoding the self-protein(s), -polypeptide(s), or - peptide(s) is inserted into the expression vector, the vector is then referred to as a "self-vector." In the case where polynucleotides encoding more than one self-polypeptide are to be administered, a single self-vector may encode multiple self-polypeptides, or alternatively, each self-polypeptide may be encoded on a separate DNA expression vector. Multiple self-polypeptides contained within a single self-vector may be arranged in any manner that allows for their expression as, for example, by using internal ribosomal entry sequences (IRES) or designing fusion proteins.

A "modified self-vector" refers to a self-vector that is modified to alter the expression of the self-protein(s), -polypeptide(s), or peptide(s). Alterations in the expression of the self-protein(s), -polypeptide(s), or -peptide(s) may either be to increase or lower the expression of the self-protein(s), -polypeptide(s), or peptide(s) compared to an unmodified self vector. Alternatively a modified self-vector includes a modification to the coding sequence to change a secreted or membrane bound self-protein(s), -polypeptide(s), or -peptide(s) to a non-secreted or non-membrane bound self-protein(s), -polypeptide(s), or - peptide(s). A modified self-vector also includes an alteration to increase the expression of self-protein(s), -polypeptide(s), or peptide(s) combined with a modification to change a secreted or membrane bound self-protein(s), -polypeptide(s), or -peptide(s) to a non-secreted or non-membrane bound self-protein(s), -polypeptide(s), or -peptide(s). A modified self-vector is administered to a subject to modulate an immune response.

A "high expression self-vector" or "HESV" refers herein to a modified self-vector that is altered to increase expression of an encoded self-protein(s), -polypeptide(s), or - peptide(s) relative to an unmodified self-vector encoding the same self-protein(s), - polypeptide(s), or -peptide(s). A HESV comprises a polynucleotide encoding and capable of expressing a self-polypeptide associated with an autoimmune disease and a modification to generate increased expression of the self-polypeptide relative to the same self-vector unmodified. A HESV further comprises in operative combination: a promoter; a polynucleotide encoding a self-polypeptide that includes at least one autoantigenic epitope associated with the autoimmune disease; a transcription terminator; and at least one modification for generating increased expression of the self-polypeptide in a host cell, in which the increased expression is relative to an unmodified self-vector comprising the promoter, polynucleotide, and transcription terminator. Modifications of a self-vector to generate a HESV with increased expression of a self-polypeptide are selected from alterations that increase: transcription initiation, transcription termination, mRNA stability, translation efficiency, and/or protein stability. More specifically, modifications of a self-vector to increase expression of a self-polypeptide are selected from the group consisting of: using a stronger promoter region, addition of enhancer regions, using a more efficient transcription terminator sequence, addition of polyadenylation signals, using a more ideal consensus kozak sequence, optimizing codon usage, inclusion of introns or combinations of the foregoing modifications. Single or multiple modifications may be incorporated into a self-vector to generate a HESV.

A "non-secreted self-vector" or "N-SSV" refers herein to a modified self-vector that contains a polynucleotide encoding for an intracellular or non-secreted self-polypeptide version of a extracellular or secreted autoantigen (*e.g*., a transmembrane protein or secreted soluble factor) associated with a disease. A N-SSV comprises a polynucleotide encoding and capable of expressing a secreted self-polypeptide associated with an autoimmune disease and a modification to express a non-secreted or non-membrane bound self-polypeptide from a host cell. A N-SSV further comprises in operative combination: a promoter; a polynucleotide encoding an extracellular or secreted self-polypeptide that includes at least one autoantigenic epitope associated with the autoimmune disease; a transcription terminator; and at least one modification to prevent secretion of the self-polypeptide from a host cell relative to an unmodified self-vector comprising the promoter, polynucleotide, and transcription terminator. Modifications to a self-vector to generate a N-SSV encoding and expressing a non-secreted or non-membrane bound version of a secreted or membrane bound self-polypeptide include but are not limited to eliminating the signal sequence, mutating the signal sequence, and adding alternative protein localization (ER retention, plasma membrane attachment, etc.) protein degradation signals or modifying or deleting, transmembrane domains or hydrophobic regions of the self-polypeptide.

A "non-secreted high expression self-vector" or "N-SHESV" refers to a modified self-vector that is altered to increase expression of an encoded intracellular or non-secreted version of an extracellular or secreted self-polypeptide or non-membrane bound version of a membrane bound self-polypeptide in which expression and secretion is relative to an unmodified self-vector. A N-SHESV comprises a polynucleotide encoding and capable of expressing a secreted or membrane bound self-polypeptide associated with an autoimmune disease and a modification to generate increased expression of the self-polypeptide in a non-secreted or non-membrane bound form relative to the unmodified self-vector. A N-SHESV further comprises in operative combination: a promoter; a polynucleotide encoding a extracellular or secreted self-polypeptide that includes at least one autoantigenic epitope associated with the autoimmune disease; a transcription terminator; and at least one modification for generating increased expression of the self-polypeptide and at least one modification to express the non-secreted or non-membrane bound self-polypeptide from a host cell where both modifications are relative to an unmodified self-vector comprising the promoter, polynucleotide, and transcription terminator.

A "secreted self-vector" or "SSV" refers herein to a modified self-vector that contains a polynucleotide encoding a secreted self-polypeptide version of a membrane associated or intracellular autoantigen associated with a disease. A SSV comprises a polynucleotide encoding and capable of expressing a membrane associated or intracellular self-polypeptide associated with an autoimmune disease and a modification to allow secretion of the self-polypeptide from a host cell. A SSV comprises a polynucleotide encoding and capable of expressing a membrane associated or intracellular self-polypeptide associated with an autoimmune disease and a modification to allow secretion of the self-polypeptide from a host cell. A SSV further comprises in operative combination: a promoter; a polynucleotide encoding a membrane associated or intracellular self-polypeptide that includes at least one autoantigenic epitope associated with the autoimmune disease; a transcription terminator; and at least one modification to permit secretion of the self-polypeptide from a host cell compared to an unmodified self-vector comprising the promoter, polynucleotide, and transcription terminator. Modifications to a self-vector to generate a SSV encoding and expressing a secreted version of an intracellular self-polypeptide include, but are not limited to, addition of a signal sequence. Additionally, the modification may further include signals for membrane association including, for example, a transmembrane domain or a GPI anchor so that intracellular epitope(s) are presented extracellularly. Modifications to a self-vector to generate a SSV encoding and expressing a secreted version of a membrane associated self-polypeptide include but are not limited to: removal of a transmembrane domain; removal of a GPI linkage, removal of an extracellular and transmembrane domain with addition of a signal sequence to an intracellular domain; and removal of a transmembrane domain and intracellular domain.

A "secreted high expression self-vector" or "SHESV" as used herein refers to a modified self-vector that is altered to increase expression of an encoded secreted version of a membrane associated or intracellular self-polypeptide in which expression and secretion is relative to an unmodified self-vector. A SHESV comprises a polynucleotide encoding and capable of expressing a membrane associated or intracellular self-polypeptide associated with an autoimmune disease and a modification to generate increased expression of the self-polypeptide in a secreted or extracellular membrane associated form relative to the same self-vector unmodified. A SHESV further comprises in operative combination: a promoter; a polynucleotide encoding a membrane associate or intracellular self-polypeptide that includes at least one autoantigenic epitope associated with the autoimmune disease; a transcription terminator; and at least one modification for generating increased expression of the self-polypeptide and at least one modification to allow secretion of the self-polypeptide from a host cell where both modifications are relative to an unmodified self-vector comprising the promoter, polynucleotide, and transcription terminator.

"Plasmids" and "vectors" are designated by a lower case p followed by letters and/or numbers. The starting plasmids are commercially available, publicly available on an unrestricted basis, or can be constructed from available plasmids in accord with published procedures. In addition, equivalent plasmids to those described are known in the art and will be apparent to the ordinarily skilled artisan. A "vector" or "plasmid" refers to any genetic element that is capable of replication by comprising proper control and regulatory elements when present in a host cell. For purposes of this invention examples of vectors or plasmids include, but are not limited to, plasmids, phage, transposons, cosmids, virus, and the like.

"Transfection" means introducing DNA into a host cell so that the DNA is expressed, whether functionally expressed or otherwise; the DNA may also replicate either as an extrachromosomal element or by chromosomal integration. Transfection may be accomplished by any method known in the art suitable for introducing an extracellular nucleic acid into a host cell, including but not limited to, the use of transfection facilitating agents or processes such as calcium phosphate co-precipitation, viral transduction, protoplast fusion, DEAE-dextran-mediated transfection, polybrene-mediated transfection, liposome fusion, microinjection, microparticle bombardment or electroporation. In preferred embodiments the nucleic acid of interest is formulated with calcium for injection into an animal for uptake by the host cells of the animal. In preferred embodiments the nucleic acid to be transfected is formulated with calcium at a concentration between about 0.05 mM to about 2 M; in more preferred embodiments the calcium concentration is between about 0.9 mM (1x) to about 8.1 mM (9x); in most preferred embodiments the calcium concentration is between about 0.9 mM (1x) to about 5.4 mM (6x).

"Antigen," as used herein, refers to any molecule that can be recognized by the immune system that is by B cells or T cells, or both.

"Autoantigen," as used herein, refers to an endogenous molecule, typically a protein or fragment thereof, that elicits a pathogenic immune response. When referring to the autoantigen or epitope thereof as "associated with an autoimmune disease," it is understood to mean that the autoantigen or epitope is involved in the pathophysiology of the disease either by inducing the pathophysiology (*i.e*., associated with the etiology of the disease), mediating or facilitating a pathophysiologic process; and/or by being the target of a pathophysiologic process. For example, in autoimmune disease, the immune system aberrantly targets autoantigens, causing damage and dysfunction of cells and tissues in which the autoantigen is expressed and/or present. Under normal physiological conditions, autoantigens are ignored by the host immune system through the elimination, inactivation, or lack of activation of immune cells that have the capacity to recognize the autoantigen through a process designated "immune tolerance."

As used herein the term "epitope" is understood to mean a portion of a polypeptide having a particular shape or structure that is recognized by either B-cells or T-cells of the animal's immune system. "Autoantigenic epitope" or "pathogenic epitope" refers to an epitope of an autoantigen that elicits a pathogenic immune response.

The terms "polypeptide," "peptide," and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymers.

"Self-protein," "self-polypeptide," or self-peptide" are used herein interchangeably and refer to insulin, proinsulin or preproinsulin. These are encoded within the genome of the animal; produced or generated in the animal; may be modified posttranslationally at some time during the life of the animal; and, present in the animal non-physiologically. The term "non-physiological" or "non-physiologically" when used to describe the self-protein(s), -polypeptide(s), or -peptide(s) of this invention means a departure or deviation from the normal role or process in the animal for that self-protein, - polypeptide, or -peptide. When referring to the self-protein, -polypeptide or -peptide as "associated with a disease" or "involved in a disease" it is understood to mean that the self-protein, -polypeptide, or peptide may be modified in form or structure and thus be unable to perform its physiological role or process or may be involved in the pathophysiology of the condition or disease either by inducing the pathophysiology; mediating or facilitating a pathophysiologic process; and/or by being the target of a pathophysiologic process. For example, in autoimmune disease, the immune system aberrantly attacks self-proteins causing damage and dysfunction of cells and tissues in which the self-protein is expressed and/or present. Alternatively, the self-protein, -polypeptide or -peptide can itself be expressed at non-physiological levels and/or function non-physiologically. For example in neurodegenerative diseases self-proteins are aberrantly expressed, and aggregate in lesions in the brain thereby causing neural dysfunction. In other cases, the self-protein aggravates an undesired condition or process. For example in osteoarthritis, self-proteins including collagenases and matrix metalloproteinases aberrantly degrade cartilage covering the articular surface of joints. Examples of posttranslational modifications of self-protein(s), - polypeptide(s) or peptide(s) are glycosylation, addition of lipid groups, reversible phosphorylation, addition of dimethylarginine residues, citrullination, and proteolysis, and more specifically citrullination of fillagrin and fibrin by peptidyl arginine deiminase (PAD), alpha β-crystallin phosphorylation, citrullination of MBP, and SLE autoantigen proteolysis by caspases and granzymes. Immunologically, self-protein, -polypeptide or peptide would all be considered host self-antigens and under normal physiological conditions are ignored by the host immune system through the elimination, inactivation, or lack of activation of immune cells that have the capacity to recognize self-antigens through a process designated "immune tolerance." A self-protein, -polypeptide, or peptide does not include immune proteins, polypeptides, or peptides which are molecules expressed physiologically exclusively by cells of the immune system for the purpose of regulating immune function. The immune system is the defense mechanism that provides the means to make rapid, highly specific, and protective responses against the myriad of potentially pathogenic microorganisms inhabiting the animal's world. Examples of immune protein(s), polypeptide(s) or peptide(s) are proteins comprising the T-cell receptor, immunoglobulins, cytokines including the type I interleukins, and the type II cytokines, including the interferons and IL-10, TNF, lymphotoxin, and the chemokines such as macrophage inflammatory protein -1 alpha and beta, monocyte-chemotactic protein and RANTES, and other molecules directly involved in immune function such as Fas-ligand. There are certain immune protein(s), polypeptide(s) or peptide(s) that are included in the self-protein, -polypeptide or peptide of the invention and they are: class I MHC membrane glycoproteins, class II MHC glycoproteins and osteopontin. Self-protein, -polypeptide or -peptide does not include proteins, polypeptides, and peptides that are absent from the subject, either entirely or substantially, due to a genetic or acquired deficiency causing a metabolic or functional disorder, and are replaced either by administration of said protein, polypeptide, or peptide or by administration of a polynucleotide encoding said protein, polypeptide or peptide (gene therapy). Examples of such disorders include Duchenne' muscular dystrophy, Becker's muscular dystrophy, cystic fibrosis, phenylketonuria, galactosemia, maple syrup urine disease, and homocystinuria. Self-protein, -polypeptide or -peptide does not include proteins, polypeptides, and peptides expressed specifically and exclusively by cells which have characteristics that distinguish them from their normal counterparts, including: (1) clonality, representing proliferation of a single cell with a genetic alteration to form a clone of malignant cells, (2) autonomy, indicating that growth is not properly regulated, and (3) anaplasia, or the lack of normal coordinated cell differentiation. Cells have one or more of the foregoing three criteria are referred to either as neoplastic, cancer or malignant cells.

"Modulation of," "modulating", or "altering an immune response" as used herein refers to any alteration of an existing or potential immune responses against self-molecules, including, *e.g*., nucleic acids, lipids, phospholipids, carbohydrates, self-polypeptides, protein complexes, or ribonucleoprotein complexes, that occurs as a result of administration of a polynucleotide encoding a self-polypeptide. Such modulation includes any alteration in presence, capacity, or function of any immune cell involved in or capable of being involved in an immune response. Immune cells include B cells, T cells, NK cells, NK T cells, professional antigen-presenting cells, non-professional antigen-presenting cells, inflammatory cells, or any other cell capable of being involved in or influencing an immune response. "Modulation" includes any change imparted on an existing immune response, a developing immune response, a potential immune response, or the capacity to induce, regulate, influence, or respond to an immune response. Modulation includes any alteration in the expression and/or function of genes, proteins and/or other molecules in immune cells as part of an immune response.

"Modulation of an immune response" includes, for example, the following: elimination, deletion, or sequestration of immune cells; induction or generation of immune cells that can modulate the functional capacity of other cells such as autoreactive lymphocytes, antigen presenting cells (APCs), or inflamatory cells; induction of an unresponsive state in immune cells (*i.e*., anergy); increasing, decreasing, or changing the activity or function of immune cells or the capacity to do so, including but not limited to altering the pattern of proteins expressed by these cells. Examples include altered production and/or secretion of certain classes of molecules such as cytokines, chemokines, growth factors, transcription factors, kinases, costimulatory molecules, or other cell surface receptors; or any combination of these modulatory events.

For example, a polynucleotide encoding a self-polypeptide can modulate an immune response by eliminating, sequestering, or inactivating immune cells mediating or capable of mediating an undesired immune response; inducing, generating, or turning on immune cells that mediate or are capable of mediating a protective immune response; changing the physical or functional properties of immune cells; or a combination of these effects. Examples of measurements of the modulation of an immune response include, but are not limited to, examination of the presence or absence of immune cell populations (using flow cytometry, immunohistochemistry, histology, electron microscopy, polymerase chain reaction (PCR)); measurement of the functional capacity of immune cells including ability or resistance to proliferate or divide in response to a signal (such as using T cell proliferation assays and pepscan analysis based on ³H-thymidine incorporation following stimulation with anti-CD3 antibody, anti-T cell receptor antibody, anti-CD28 antibody, calcium ionophores, PMA, antigen presenting cells loaded with a peptide or protein antigen; B cell proliferation assays); measurement of the ability to kill or lyse other cells (such as cytotoxic T cell assays); measurements of the cytokines, chemokines, cell surface molecules, antibodies and other products of the cells (*e.g*., by flow cytometry, enzyme-linked immunosorbent assays, Western blot analysis, protein microarray analysis, immunoprecipitation analysis); measurement of biochemical markers of activation of immune cells or signaling pathways within immune cells (*e.g*., Western blot and immunoprecipitation analysis of tyrosine, serine or threonine phosphorylation, polypeptide cleavage, and formation or dissociation of protein complexes; protein array analysis; DNA transcriptional, profiling using DNA arrays or subtractive hybridization); measurements of cell death by apoptosis, necrosis, or other mechanisms (*e.g*., annexin V staining, TUNEL assays, gel electrophoresis to measure DNA laddering, histology; fluorogenic caspase assays, Western blot analysis of caspase substrates); measurement of the genes, proteins, and other molecules produced by immune cells (*e.g*., Northern blot analysis, polymerase chain reaction, DNA microarrays, protein microarrays, 2-dimentional gel electrophoresis, Western blot analysis, enzyme linked immunosorbent assays, flow cytometry); and measurement of clinical symptoms or outcomes such as improvement of autoimmune, neurodegenerative, and other diseases involving self proteins or self polypeptides (clinical scores, requirements for use of additional therapies, functional status, imaging studies) for example, by measuring relapse rate or disease severity (using clinical scores known to the ordinarily skilled artisan) in the case of multiple sclerosis, measuring blood glucose in the case of type I diabetes, or joint inflammation in the case of rheumatoid arthritis.

"Immune Modulatory Sequences (IMSs)" as used herein refers to compounds consisting of deoxynucleotides, ribonucleotides, or analogs thereof that modulate an autoimmune and/or inflammatory response. IMSs are typically oligonucleotides or a sequence of nucleotides incorporated in a vector (*e.g*., single-strand or double-stranded DNA, RNA, and/or oligonucleosides).

"Subjects" shall mean any animal, such as, for example, a human, non-human primate, horse, cow, dog, cat, mouse, rat, guinea pig or rabbit.

"Treating," "treatment," or "therapy" of a disease or disorder shall mean slowing, stopping or reversing the disease's progression, as evidenced by decreasing, cessation or elimination of either clinical or diagnostic symptoms, by administration of a polynucleotide encoding a self-polypeptide, either alone or in combination with another compound as described herein. "Treating," "treatment," or "therapy" also means a decrease in the severity of symptoms in an acute or chronic disease or disorder or a decrease in the relapse rate as for example in the case of a relapsing or remitting autoimmune disease course or a decrease in inflammation in the case of an inflammatory aspect of an autoimmune disease. In the preferred embodiment, treating a disease means reversing or stopping or mitigating the disease's progression, ideally to the point of eliminating the disease itself. As used herein, ameliorating a disease and treating a disease are equivalent.

"Preventing," "prophylaxis," or "prevention" of a disease or disorder as used in the context of this invention refers to the administration of a polynucleotide encoding a self-polypeptide, either alone or in combination with another compound as described herein, to prevent the occurrence or onset of a disease or disorder or some or all of the symptoms of a disease or disorder or to lessen the likelihood of the onset of a disease or disorder.

A "therapeutically or prophylactically effective amount" of a self-vector refers to an amount of the self-vector that is sufficient to treat or prevent the disease as, for example, by ameliorating or eliminating symptoms and/or the cause of the disease. For example, therapeutically effective amounts fall within broad range(s) and are determined through clinical trials and for a particular patient is determined based upon factors known to the skilled clinician, including, *e.g*., the severity of the disease, weight of the patient, age, and other factors.

### BRIEF DESCRIPTION OF THE DRAWINGS

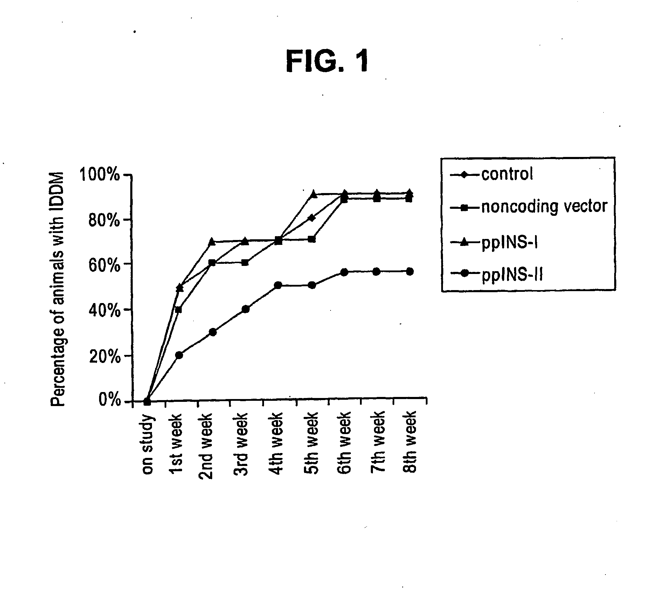

Figure 1: Treatment of established hyperglycermia with DNA vaccination using a self-vector encoding preproinsulin II. Female NOD mice were treated with bi-weekly intramuscular DNA vaccines after the onset of hyperglycemia (190-250 mg/dl) at treatment week 0. Fifty µg of each DNA plasmid was administered per animal. The DNA administered included vaccines that encoded either murine preproinsulin I (ppINS-I), murine preproinsulin II (ppINS-II) or a non-coding vector as indicated in the legend. Progression to diabetes was defined as two consecutive blood glucose measurements greater than 250 mg/dl on weekly monitoring. The time at which the glucose measurements were made is indicated on the x-axis, and the percentage of mice defined as diabetic is indicated on the y-axis.

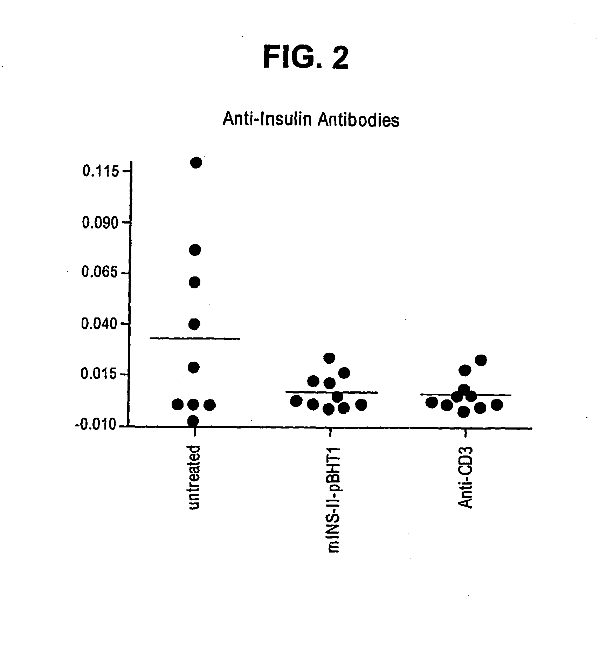

Figure 2: Correlation of DNA vaccination effect and anti-insulin autoantibody titers. Sera from animals involved in the study described in Figure 1 were obtained at the completion of the study. Anti-insulin autoantibody titers were measured by radioimmunoassay. Individual dots represent the insulin autoantibody indices of individual mice. The horizontal bar indicates the mean value of all mice within the group.

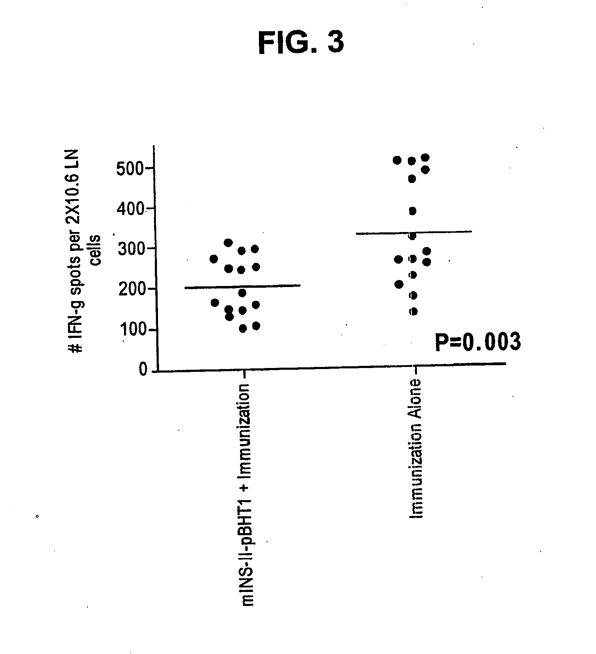

Figure 3: Effect of DNA vaccination using a self-vector encoding preproinsulin II on subsequent immune response. Female NOD mice were treated bi-weeldy for a total of 3 injections with intramuscular DNA vaccines of mINS-II-pBHT1 at 6 weeks of age. Fifty µg of each DNA plasmid was administered per animal per injection. Two weeks after the last DNA injection DNA vaccinated and control animals were immunized with insulin II 9-23 peptide. Ten days after the immunization, the number of lymph node cells responding to restimulation with insulin II 9-23 by INF-gamma secretion was determined by ELISpots. Shown are the numbers of IFN-gamma secreting cells for each treated or control animal.

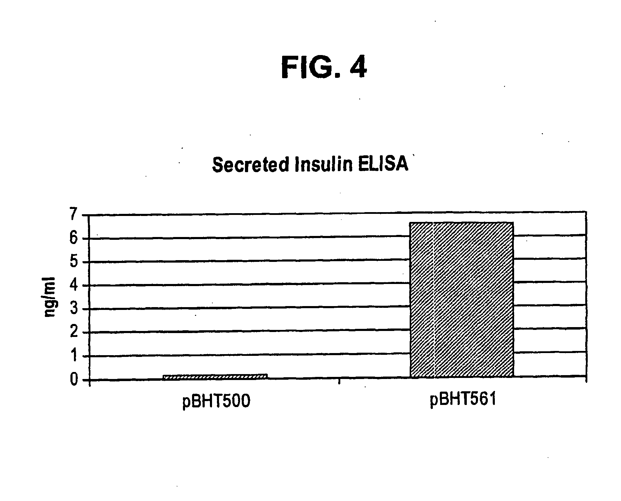

Figure 4: Increased insulin expression by high expression self-vector encoding proinsulin. HEK293 transfected with the insulin expressing plasmids mINS-II-pBHT1 (pBHT500) and mINS-II-HESV (pBHT561) were incubated for 24 hours and insulin protein levels in the supernatant were analyzed at by ELISA. The amount of protein detected in the supernatant (ng/mL) is graphically represented for both insulin expressing vectors.

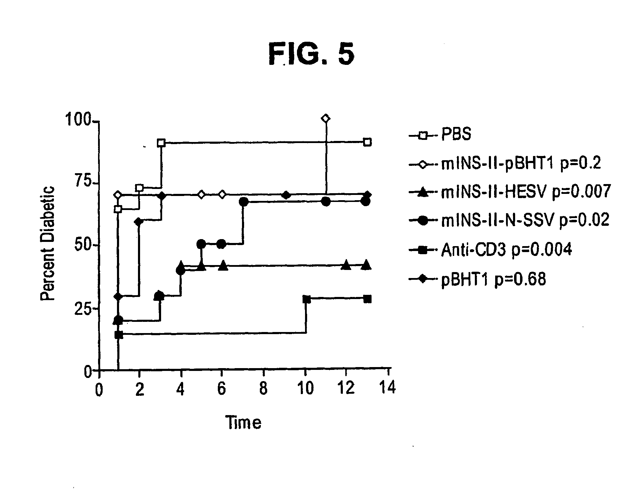

Figure 5: Treatment of established hyperglycemia with DNA vaccination using modified self-vectors encoding insulin II. Female NOD mice (n=10 per group) were treated with weekly intramuscular DNA vaccines after the onset of hyperglycemia (190-250 mg/dl) at treatment week 0. Fifty µg of each DNA plasmid was administered per animal. The DNA vaccines administered included pBHT1 empty vector, mINS-II-pBHT1, mINS-II-HESV, or mINS-II-N-SSV. Anti-CD3 was administered at 5ug/animal by IV injection for 5 consecutive days. Animals were monitored weekly for IDDM onset and were considered diabetic on the first of 2 consecutive weeks with blood glucose levels greater than 250mg/dl. Shown are the percentages of diabetic animals over time. KM plots were generated using GraphPad Prism. LogRank tests were performed by Prism to determine statistical significance.

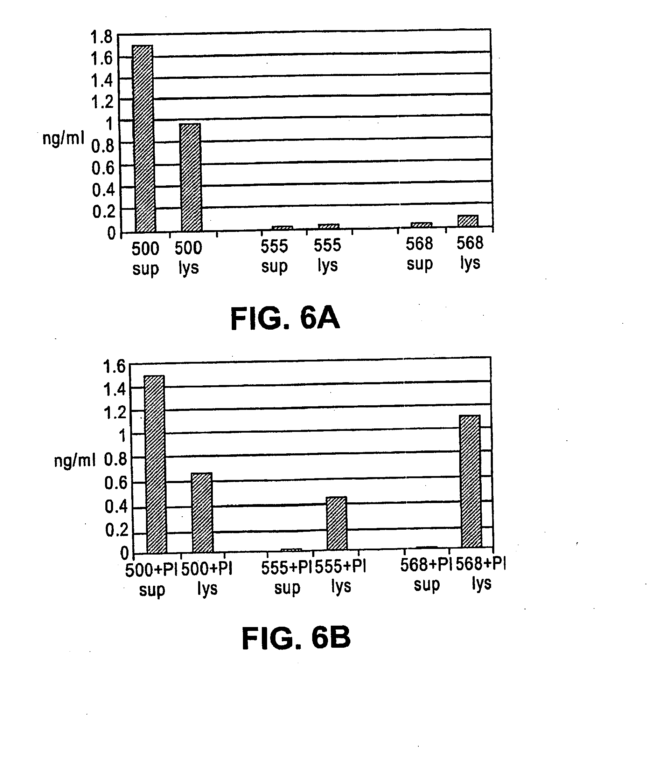

Figure 6: Proinsulin expressed by non-secreted self-vectors is intracellular. A) HEK293 transfected with the insulin expressing plasmids mINS-II-pBHT1 (pBHT500), mINS-II-N-SSV (pBHT555), and mINS-II-N-SHESV (pBHT568) were incubated for 48 hours and insulin protein levels in the supernatant and cell lysates were analyzed at by ELISA. The amount of protein detected in supernatant and cell lysates is shown for each insulin expressing vector. B) Alternatively transfected cells were incubated for 24 hrs in normal media and then for 24 hrs in the presence of the proteasome inhibitor lactacystin. Insulin protein levels at 48 hrs were measured by ELISA. The amount of protein detected in supernatant and cell lysates is shown for each insulin expressing vector.

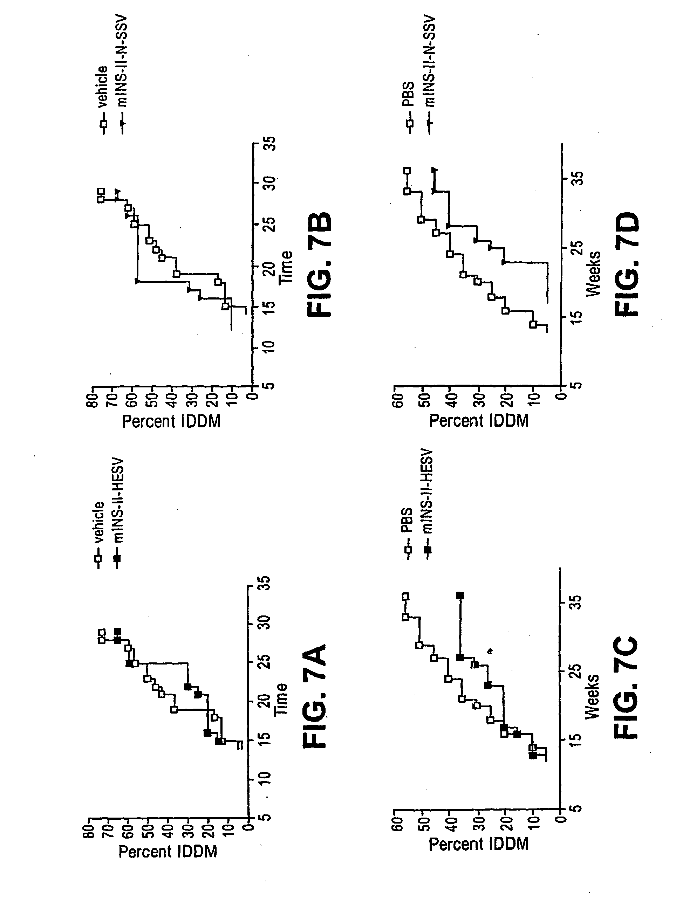

Figure 7: Weekly versus bi-weekly dosing of DNA vaccines using modified self-vectors to treat diabetes. Female NOD mice (n=20 per group) were treated with weekly (A, B) or bi-weekly (C, D) intramuscular injections of mINS-II-HESV (A, C) and mINS-N-SSV (B, D) DNA vaccines at 10 weeks of age. Fifty µg of each DNA plasmid was administered per animal. Animals were monitored weekly for diabetes onset and were considered diabetic on the first of 2 consecutive weeks with blood glucose levels greater than 250mg/dl. Shown are the percentages of diabetic animals over time compared to vehicle control. KM plots were generated using GraphPad Prism.

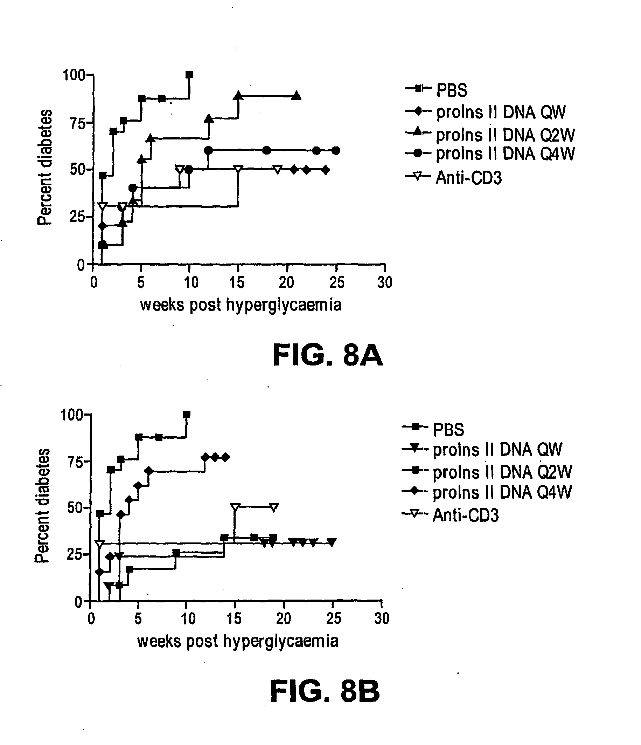

Figure 8: Treatment of established hyperglycemia with DNA vaccination using a non-secreted high expression self-vector (N-SHESV) encoding mouse proinsulin II. Female NOD mice (n=15 per group) were treated with weekly (QW), every other week (Q2W), or every fourth week (Q4W) intramuscular DNA vaccinations after the onset of hyperglycemia (190-250 mg/dl) at treatment week 0. Fifty µg of each DNA plasmid was administered per animal. The DNA vaccines administered included: mINS-II-N-SSV and mINS-N-SHESV. Anti-CD3 was administered at 5ug/animal by IV injection for 5 consecutive days. Animals were monitored weekly for IDDM onset and were considered diabetic on the first of 2 consecutive weeks with blood glucose levels greater than 250mg/dl. Shown are the percentages of diabetic animals treated with mINS-II-N-SSV (A) versus mINS-II-N-SHESV over time.

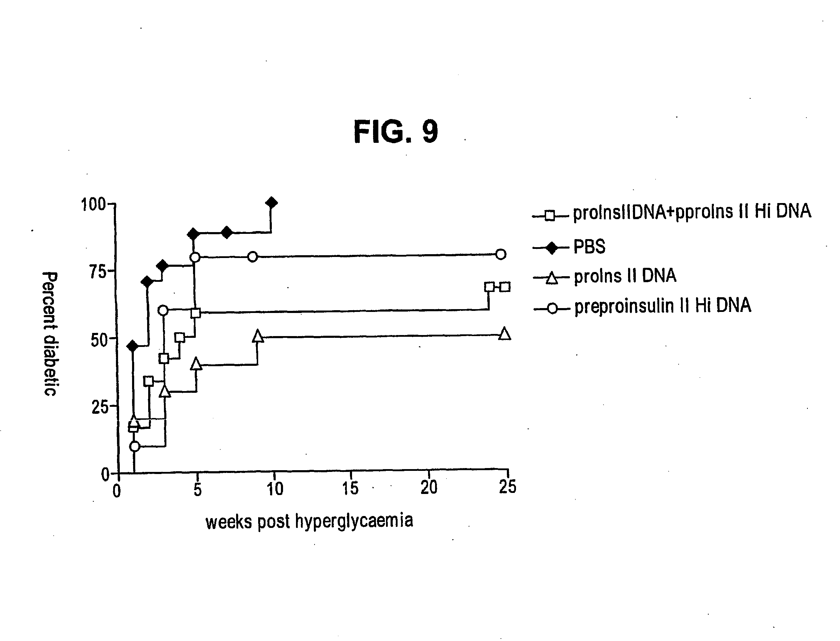

Figure 9: Treatment of established hyperglycemia with DNA vaccination using a combination of a non-secreted self-vector (N-SSV) and a high expression self-vector (HESV). Female NOD mice were treated with weekly intramuscular DNA vaccinations after the onset of hyperglycemia (190-250 mg/dl) at treatment week 0. Fifty µg of each DNA plasmid was administered per animal. The DNA vaccines administered included: mINS-II-N-SSV, mINS-N-HESV, and a combination of these two vectors. Animals were monitored weekly for IDDM onset and were considered diabetic on the first of 2 consecutive weeks with blood glucose levels greater than 250mg/dl. Shown are the percentages of diabetic animals treated over time. KM plots were generated using GraphPad Prism.

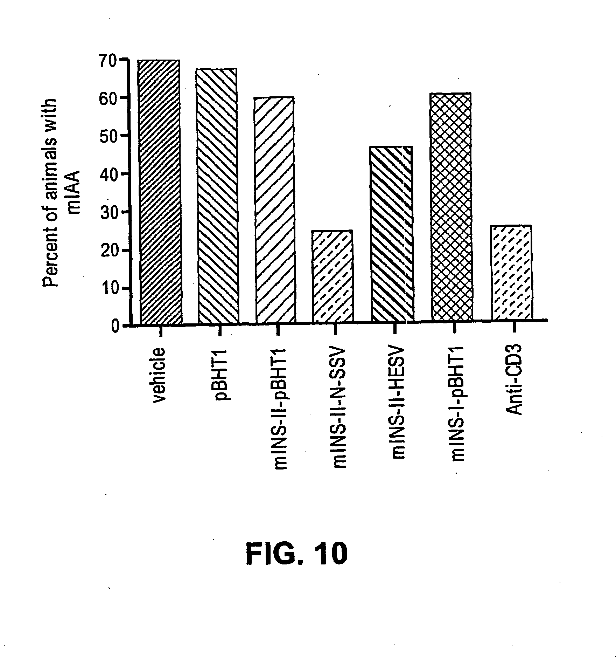

Figure 10: Prevention of autoantibody production in NOD mice by DNA vaccination using modified DNA self-vectors encoding preproinsulin II. Female NOD mice (n=20 per group) were treated with weekly intramuscular DNA vaccines at five weeks of age. Fifty µg of each DNA plasmid was administered per animal. The DNA vaccines administered included pBHT1 empty vector, mINS-I-pBHT1, mINS-II-pBHT1, mINS-II-HESV, and mINS-II-N-SSV. Anti-CD3 was administered at 5ug/animal by IV injection for 5 consecutive days. Two weeks after the last DNA injection, sera was collected and screened in a blinded fashion for antibodies to insulin by radioimmunoassay at the Barbara Davis Center for Diabetes. The percentage of animals with a mouse insulin autoantibody (mIAA) index above 0.01 for each treatment group is graphed here.

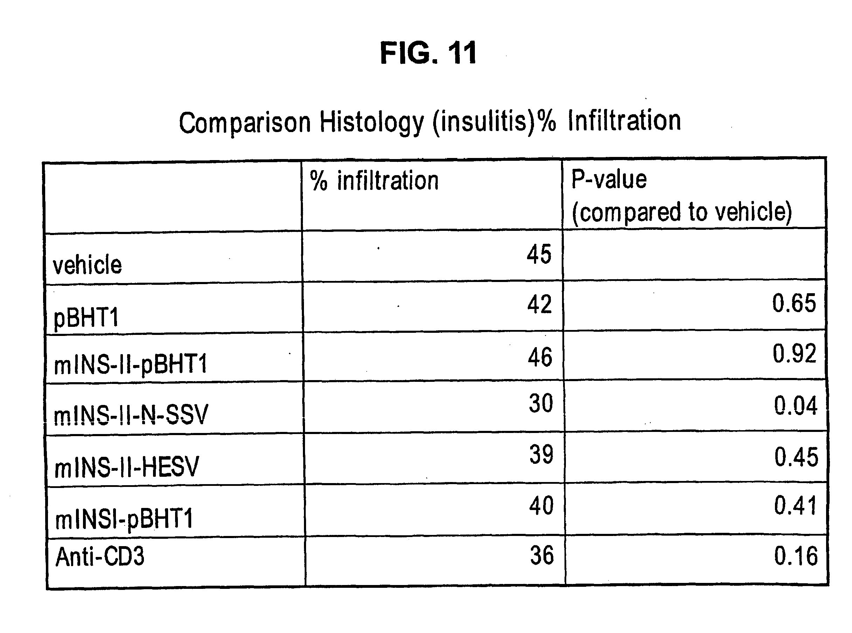

Figure 11: Prevention of insulitis in NOD mice by DNA vaccination using modified DNA self-vectors encoding preproinsulin II. Female NOD mice (n=20 per group) were treated with weekly intramuscular DNA vaccines at five weeks of age. Fifty ug of each DNA plasmid was administered per animal. The DNA vaccines administered included: pBHT1 empty vector, mINS-1-pBHT1, mINS-II-pBHT1, mINS-II-HESV, and mINS-II-N-SSV. Anti-CD3 was administered at 5ug/animal by IV injection for 5 consecutive days. Two weeks after the last DNA injection the pancreas of each treated mouse was harvested and fixed in formalin for H&E evaluation of the extent of insulitis. The percentage infiltration and the P-value compared to PBS control are shown for each treatment condition.

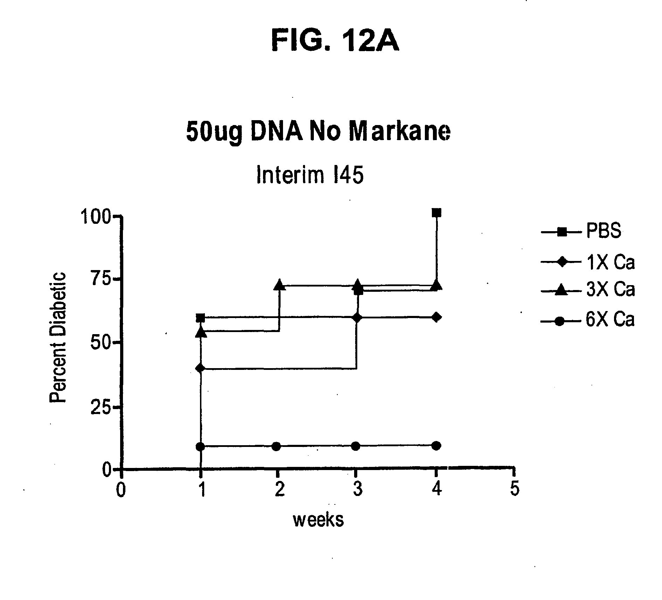

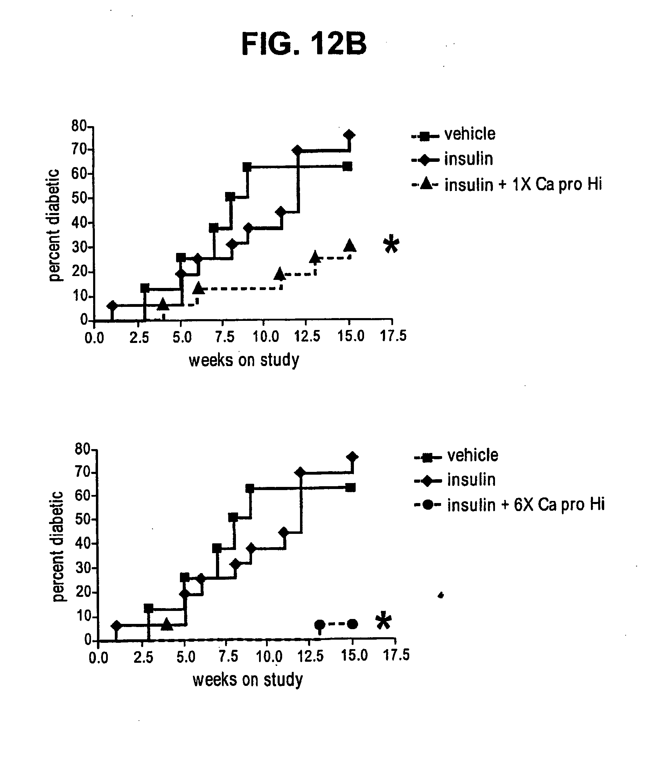

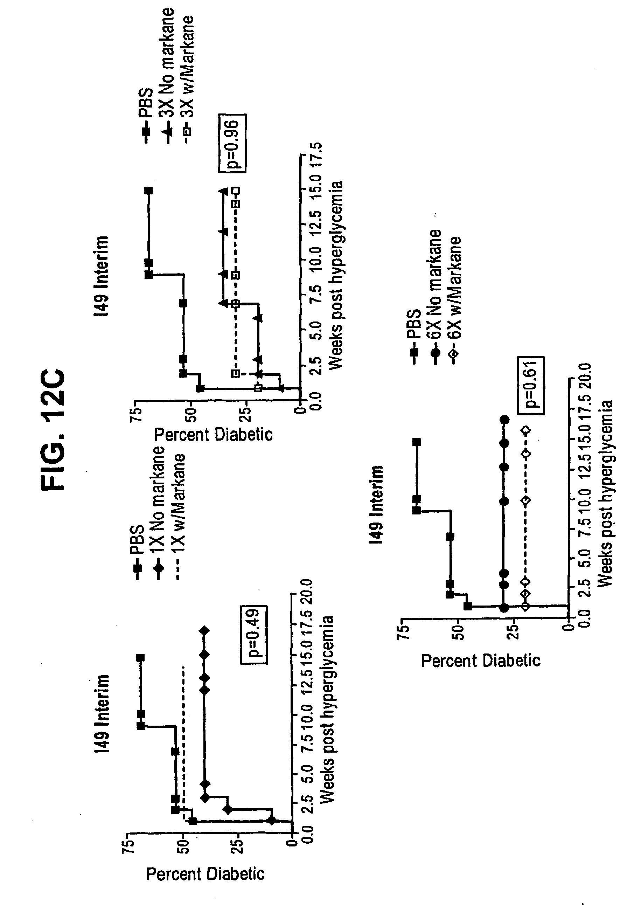

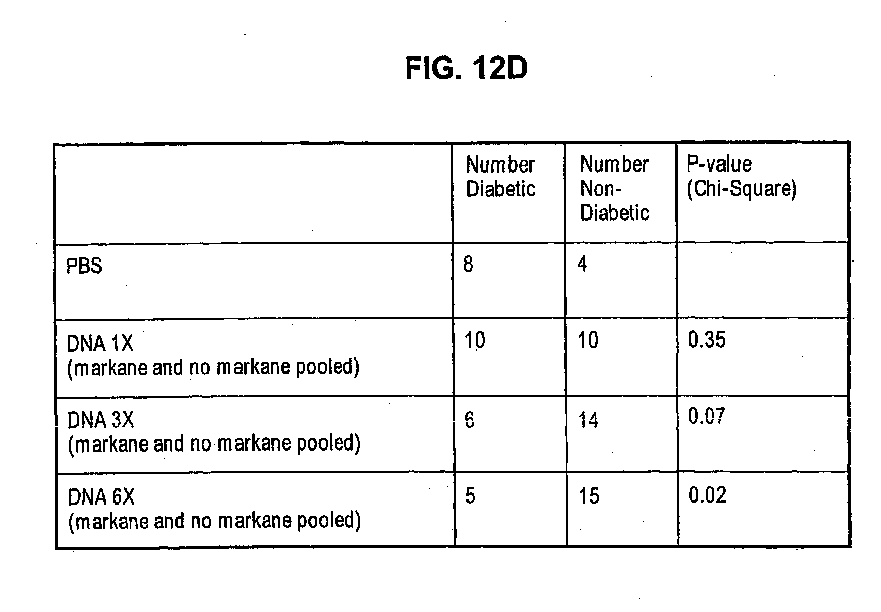

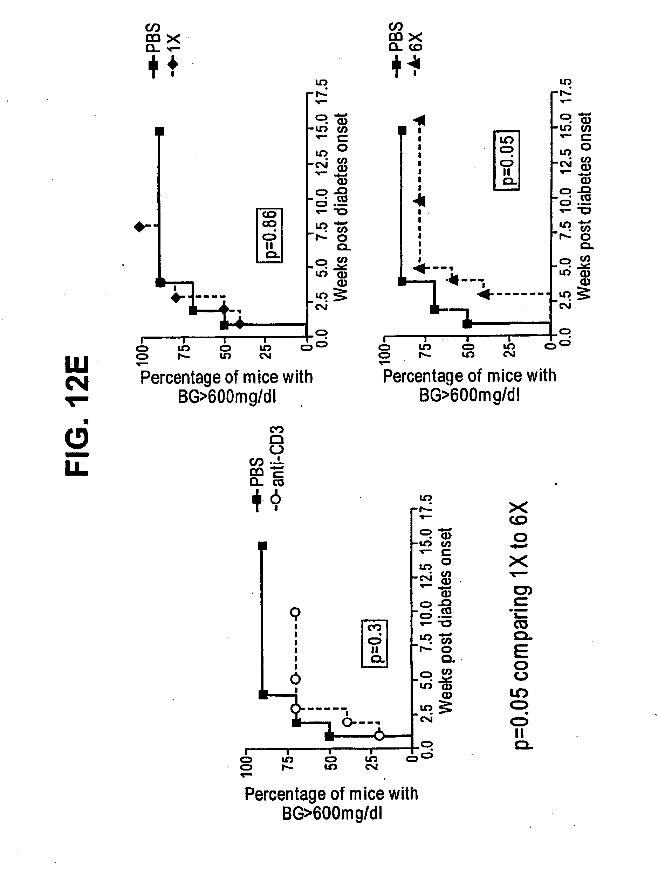

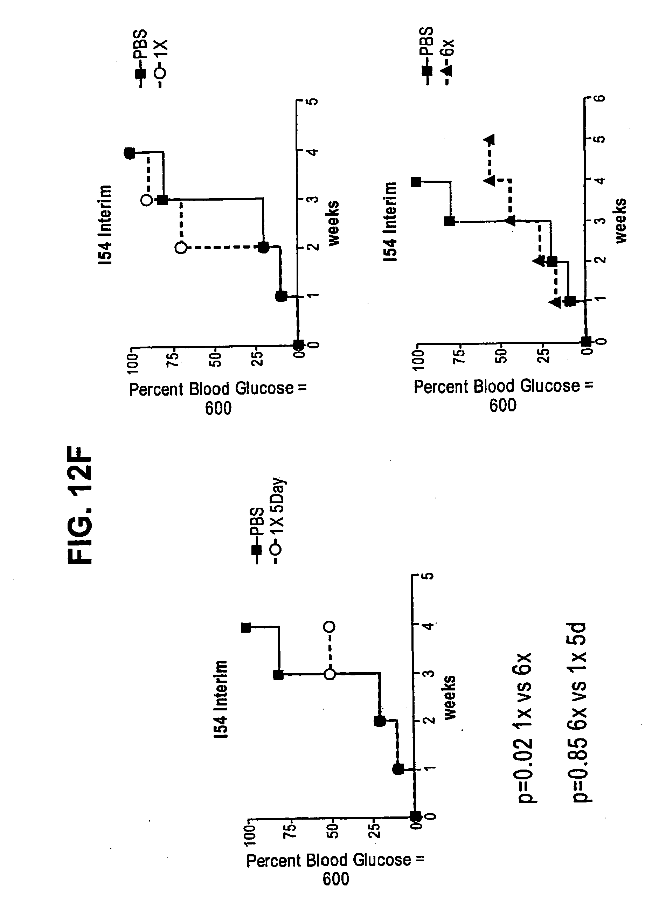

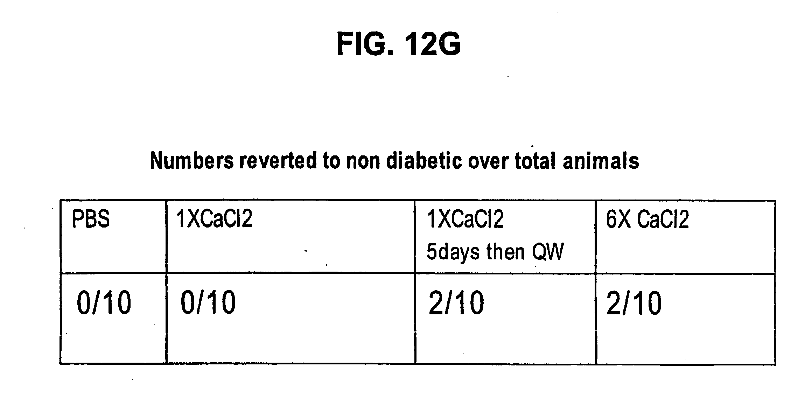

Figure 12: Treatment of established hyperglycemia with DNA vaccination using a high expression self-vector (HESV) formulated with different Ca++ concentrations. Female NOD mice were treated with weekly intramuscular DNA vaccinations after the onset of hyperglycemia (190-250 mg/dl) at treatment week 0. Fifty µg of each DNA plasmid was administered per animal. The DNA vaccine mINS-N-HESV (pBHT-3021) was injected at different Ca++ concentrations including: 0.9 mM (1x), 2.7 mM (3x) and 5.4 mM (6x). Animals were monitored weekly for IDDM onset and were considered diabetic on the first of 2 consecutive weeks with blood glucose levels greater than 250 mg/dl. Shown are the percentages of diabetic animals treated over time. KM plots were generated using GraphPad Prism. A) Treatment with pBHT-3021 in different calcium concentrations without Markane revealed that a 6x calcium formulation significantly increased the efficacy of DNA vaccination to protect against progression to diabetes. B) Treatment with pBHT-3021 at different calcium concentrations in combination with co-administration of insulin similarly revealed an increased efficacy against diabetes progression for a formulation utilizing 6x calcium. C) Treatment with pBHT-3021 at different calcium concentrations and with Markane revealed a slight increase in efficacy at 3x and 6x calcium formulations. D) Summary of results from experiments with pBHT-3021 self vector formulated with increasing concentrations of calcium with and without Markane. E) Treatment of post-diabetic animals with pBHT-3021 formulated with 1x or 6x calcium revealed a delay and reduction in the percentage of animals with high blood glucose levels with 6x calcium (lower right graph, dashed line with triangles), similar to anti-CD3 treated positive controls (left graph), compared to 1x calcium (upper right graph, dashed line with diamonds) that showed no difference from PBS treated controls (solid lines with squares). F) Treatment of post-diabetic animals with pBHT-3021 formulated with 6x calcium (lower right graph, dashed line with triangles) or formulated with 1x calcium injected for 5 days (left graph, dashed line with open circles) reduced blood glucose levels compared to PBS treated controls (solid line with squares), an effect not seen with 1x calcium formulation alone (upper right graph, dashed line with diamonds). G) One-fifth of animals treated with pBHT-3021 self-vector formulated with 6x calcium or formulated with 1x calcium injected for 5 days reverted to non-diabetic status as compared to no reversion in animals treated with 1x calcium or PBS.

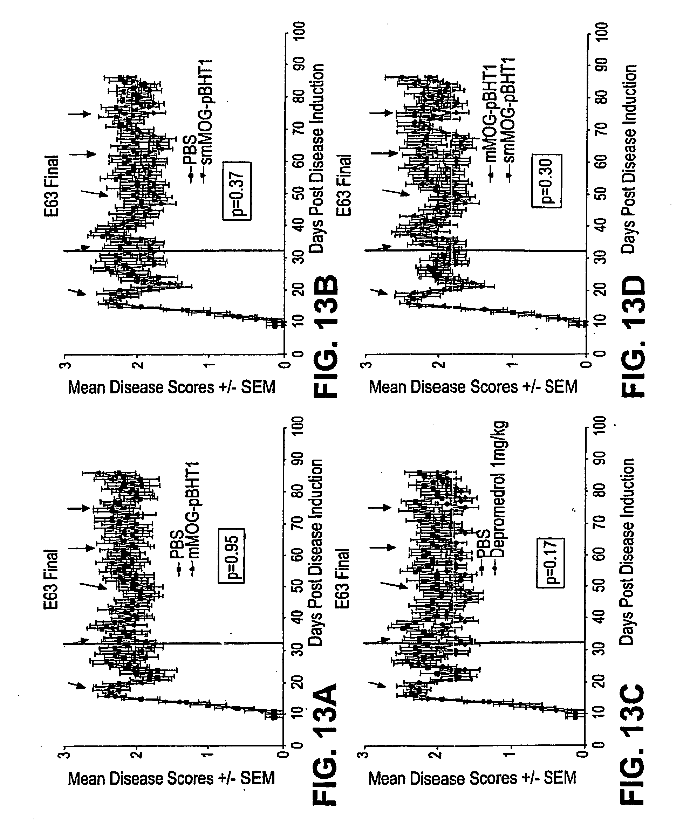

Figure 13: Treatment of EAE in mice by DNA vaccination using a secreted self vector (SSV) encoding the extracellular region of myelin oligodendrocyte glycoprotein (MOG). EAE was induced in mice using MOG peptide 35-55 and 16 days later were treated with bi-weekly intramuscular DNA vaccines as indicated by arrows in each panel. Fifty µg of each DNA plasmid was administered per animal. The DNA vaccines administered included mMOG-pBHT1 and mMOG-SSV. Depromedrol was injected as a positive control. Mean disease score for each treatment group is plotted over time post EAE induction. A) Mean disease score of mMOG-pBHT1 treated mice compared to PBS injected controls. B) Mean disease score of mMOG-SSV treated animals compared to PBS injected controls. C) Mean disease score of depromedrol treated animals compared to PBS injected controls. D) Mean disease score of mMOG-pBHT1 unmodified vector treated mice compared to mMOG-SSV treated animals.

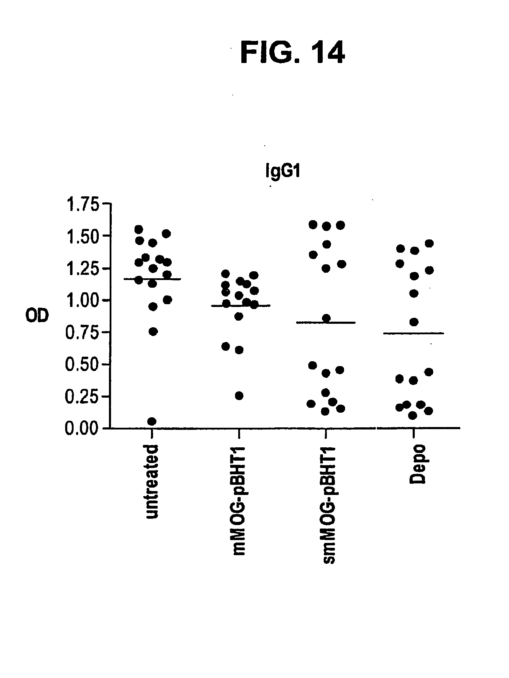

Figure 14: Immune response to treatment of EAE by DNA vaccination using a secreted self-vector (SSV) encoding the extracellular region of MOG. EAE was induced in mice using MOG peptide 35-55 and 16 days later were treated with bi-weekly intramuscular DNA vaccines. Fifty µg of each DNA plasmid was administered per animal. The DNA vaccines administered included mMOG-pBHT1 and mMOG-SSV. Depromedrol was injected as a positive control. At the conclusion of the study, the immune response of DNA vaccinated and control animals to the extracellular domain of MOG was examined. Sera from treated mice was collected and analyzed by ELISA for IgG1 anti-MOG specific antibodies. The optical density (OD) of the ELISA from each animal is plotted by treatment group. The mean OD for each group is indicated by a horizontal line.

### DETAILED DESCRIPTION OF THE INVENTION

The present invention relates to methods and compositions for treating or preventing IDDM in a subject.

### Autoimmune Disease

As used herein, "autoimmune disease" refers to IDDM.

*Insulin Dependent Diabetes Mellitus.* Human type I or insulin-dependent diabetes mellitus (IDDM) is characterized by autoimmune destruction of the β cells in the pancreatic islets of Langerhans. The depletion of β cells results in an inability to regulate levels of glucose in the blood. Overt diabetes occurs when the level of glucose in the blood rises above a specific level, usually about 250 mg/dl. In humans a long presymptomatic period precedes the onset of diabetes. During this period there is a gradual loss of pancreatic beta cell function. The development of disease is implicated by the presence of autoantibodies against insulin, glutamic acid decarboxylase and the tyrosine phosphatase IA2 (IA2).

Markers that may be evaluated during the presymptomatic stage are the presence of insulitis in the pancreas, the level and frequency of islet cell antibodies, islet cell surface antibodies, aberrant expression of Class II MHC molecules on pancreatic beta cells, glucose concentration in the blood, and the plasma concentration of insulin. An increase in the number of T lymphocytes in the pancreas, islet cell antibodies and blood glucose is indicative of the disease, as is a decrease in insulin concentration.

The Non-Obese Diabetic (NOD) mouse is an animal model with many clinical, immunological, and histopathological features in common with human IDDM. NOD mice spontaneously develop inflammation of the islets and destruction of the β cells, which leads to hyperglycemia and overt diabetes. Both CD4⁺ and CD8⁺ T cells are required for diabetes to develop, although the roles of each remain unclear. It has been shown that administration of insulin or GAD, as proteins, under tolerizing conditions to NOD mice prevents disease and down-regulates responses to the other autoantigens.

The presence of combinations of autoantibodies with various specificities in serum are highly sensitive and specific for human type I diabetes mellitus. For example, the presence of autoantibodies against GAD and/or IA-2 is approximately 98% sensitive and 99% specific for identifying type I diabetes mellitus from control serum. In non-diabetic first degree relatives of type I diabetes patients, the presence of autoantibodies specific for two of the three autoantigens including GAD, insulin and IA-2 conveys a positive predictive value of >90% for development of type IDM within 5 years.

Autoantigens targeted in human insulin dependent diabetes mellitus may include, for example, tyrosine phosphatase IA-2; IA-2β; glutamic acid decarboxylase (GAD) both the 65 kDa and 67 kDa forms; carboxypeptidase H; insulin; proinsulin (*e.g*., SEQ ID NOs:1 and 2) ; heat shock proteins (HSP); glima 38; islet cell antigen 69 KDa (ICA69); p52; two ganglioside antigens (GT3 and GM2-1); islet-specific glucose-6-phosphatase-related protein (IGRP); and an islet cell glucose transporter (GLUT 2).

Human IDDM is currently treated by monitoring blood glucose levels to guide injection, or pump-based delivery, of recombinant insulin. Diet and exercise regimens contribute to achieving adequate blood glucose control.

### Compositions and Methods for Treatment

The present invention provides improved methods and compositions for treating the clinical or diagnostic symptoms of IDDM comprising DNA vaccination with a modified self-vector encoding a self-protein(s), polypeptide(s), or -peptide(s), as defined in the claims.

Methods for the treatment or prevention of autoimmune disease by administration of a self-vector encoding one or more self-polypeptides, and which are amenable to the improvements set forth herein, are generally known in the art. Exemplary methods to which the modifications of the present invention may be applied are described in, e.g., International Patent Application Nos. WO 00/53019, WO 2003/045316, and WO 2004/047734.

### High expression self-vector (HESV)

Prior to the instant invention described herein it has been generally believed, with respect to the administration of a vector encoding an autoantigen or pathogenic epitope thereof for treating an autoimmune disease, that lower levels of autoantigen or epitope expression are more efficacious in the treatment of an autoimmune disease. Surprisingly, the studies set forth herein demonstrate that, contrary to this understanding in the art, increasing expression of a polypeptide comprising a pathogenic epitope associated with an autoimmune disease increases efficacy of such treatment.

Modifications for generating increased expression of an encoded polypeptide from a plasmid vector are well known in the art (Azevedi et al., 1999). Also envisioned are alterations to a self-vector to produce a HESV that generates a polyribonucleotide, or mRNA, with increased stability relative to the unmodified self-vector. In other embodiments a self-vector is modified to produce a HESV that generates mRNAs with increased translational efficiency compared to the unmodified self-vector. Additionally, a self-vector may be modified to generate a HESV by changes that increase the stability of an encoded self-polypeptide compared to the stability of the same self-polypeptide encoded by the unmodified self-vector. In particular embodiments of the present invention, modifications of a self-vector to increase expression of a self-polypeptide associated with an autoimmune disease are selected from: using a more ideal consensus Kozak sequence, optimizing codon usage, inclusion of introns, etc or combinations of the modifications.

Multiple modifications may be made to a self-vector to increase expression of an encoded self-polypeptide relative to the unmodified self-vector in order to generate a HESV.

A self-vector can also be modified to a HESV by the inclusion of changes that enhance the translation efficiency of an mRNA generated by transcription of a self-polynucleotide. Sequences flanking the start codon (AUG) of a mRNA influence translation initiation by eukaryotic ribosomes with the Kozak consensus sequence ⁹GCCGCC(A/G)CCAUGG⁺⁴ defining the preferred initiation signal. However, efficient translation can be obtained as long as the -3 position relative to the AUG contains a purine base (A/G) or a guanine is at the +4 position. Thus modifying a self-vector by site-directed mutagenesis, for example, to more closely approximate the Kozak sequence can be used to generate a HESV. Translation efficiency can also be increased by optimizing codon usage based on known codon biases in different species (Gustafsson et al., 2004). Codon bias is the unequal use of synonymous codons, codons encoding the same amino acid. The frequency with which different codes are used varies between different species and between proteins expressed at different levels in the same species. Modifications of a self-polynucleotide to include preferred codons over less preferred codons of a host cell can increase protein expression.

Other envisioned modifications to generate HESVs include the introduction of changes that alter the encoded self-polypeptide to increase its stability compared to the unmodified self-polypeptide. Proteins can be stabilized in a number of ways including but not limited to: the addition of carbohydrate moieties to extracellular proteins; the elimination of signals for protein degradation such as ubiquitination; entropic stabilization as by the introduction of prolines residues, disulfide bridges, etc; and reductions in water-accessible hydrophobic surfaces.

A self-vector can also be modified to a HESV by the inclusion of sequences into the vector that promote plasmid DNA nuclear localization. Such sequences are ligated into the plasmid backbone at any location that does not interfere with expression of the encoded self polypeptide. In certain embodiments the simian virus 40 (SV40) early promoter and enhancer regions that mediate plasmid nuclear transport activity (Dean et al., 1999, Curr. Eye Res. 19:66-75) are incorporated into a self-vector to generate increased protein expression from a HESV.

The β-globin/Ig chimeric intron may be obtained from the commercially available vector pTarget (Promega, Madison, WI) and placed downstream of the promoter region and immediately upstream from the start codon in the 5' untranslated region (UTR) of the self-polynucleotide. The chimeric intron is composed of the 5' donor site from the first intron of the human β-globin gene and the branch and 3' acceptor site from the intron of an immunoglobulin gene heavy chain variable region with the donor, acceptor, and branchpoint site sequences altered to match the consensus sequences for splicing (Bothwell et al., 1981). In transient transfections of human embryonic kidney cells the presence of this chimeric intron can increase expression of an encoded gene 20-fold (Brondyk, 1994).

Increased expression of a self-polypeptide associated with a HESV relative to an unmodified self-vector can be determined in host cells *in vitro* using well-known methods for host cell transfection and expression of recombinant proteins. Typically, a host cell population is transfected with either the unmodified self-vector or a HESV containing at least one modification for generating increased expression of the self-polypeptide. The host cells are then cultured in conditions that allow for expression of the self-polypeptide. Typically, the host cells and culture conditions are designed to mimic or approximate physiological conditions *in vivo.* Relative levels of expression of the self-polypeptide between host cell populations transfected with the HESV versus the unmodified self-vector are then determined using known methods such as, for example, Western blot analysis, ELISA, or FACS.

The present invention may be used to provide improved methods for treating or preventing the autoimmune disease insulin-dependent diabetes mellitus (IDDM) comprising administering to a subject a modified self-vector encoding and capable of expressing a self-polypeptide that includes one or more autoantigenic epitopes associated with IDDM. The improved method for treating or preventing IDDM includes administering to a subject an effective amount of a modified self-vector that is altered to increase expression of an encoded self-protein(s), -polypeptide(s), or -peptide(s) associated with IDDM relative to an unmodified self-vector encoding the same self-protein(s), - polypeptide(s), or -peptide(s).
Multiple HESVs encoding different self-protein(s), -polypeptide(s), or - peptide(s) may be administered. In preferred embodiments the HESV administered to treat or prevent IDDM contains a β-globin/Ig chimeric upstream of the start codon of a polynucleotide that encodes the self-polypeptide preproinsulin or proinsulin (e.g., SEQ ID NO: 2).

### Non-secreted self-vector (N-SSV)

In non-mutually exclusive uses of the invention, the improved method for treating or preventing IDDM includes administering to a subject an effective amount of a modified self-vector that contains a polynucleotide encoding for an intracellular or non-secreted non-membrane bound self-polypeptide version of an extracellular or secreted or membrane bound autoantigen (*e.g*., a transmembrane protein or secreted soluble factor) associated with the disease. A modified self-vector altered to encode an intracellular or non-secreted non-membrane bound form of an extracellular or secreted or membrane bound self-polypeptide is referred to herein as a non-secreted self-vector (N-SSV). A N-SSV comprises a polynucleotide encoding and capable of expressing a secreted self-polypeptide associated with an autoimmune disease and a modification to prevent secretion of the self-polypeptide from a host cell.

In certain variations, the non-secreted or non-membrane bound self-polypeptide encoded by a N-SSV is an altered form of a secreted or membrane bound self-protein(s), - polypeptide(s), or peptide(s) that includes a modification preventing secretion of the self-polypeptide from a host cell or incorporation or the self-polypeptide into the membrane of a host cell. In other variations the non-secreted self-polypeptide encoded by a N-SSV is an altered form of an extracellular self-polypeptide that includes a modification to encode an intracellular version of the extracellular region of, for example, a transmembrane or GPI-linked protein or in the case of a non-membrane bound self-polypeptide encoded by a N-SSV is an altered from of a membrane bound self-polypeptide that alters or deletes a transmembrane or hydrophobic region of the self-polypeptide. One particularly suitable modification for generating a N-SSV is the elimination of the signal sequence from the extracellular or secreted self-polypeptide. Alternatively the signal sequence can be mutated so that the associated protein is no longer targeted for secretion. Other non-mutually exclusive modifications that can prevent secretion and retain a protein intracellularly are also envisioned and include signals that localize the protein to particular intracellular regions such as membrane anchors (transmembrane domains, lipid modifications, etc.), nuclear localization signals (NLS), ER retention signals, lysosomal targeting sequences, etc. Alternatively, protein degradation signals, such as ubiquitination, can be added to the protein so that a significant fraction of the protein is targeted for degradation and cleaved within the cell as opposed to being secreted.
The N-SSV administered to treat IDDM may include polynucleotides that encode one or more self-polypeptides associated with IDDM such as: preproinsulin, proinsulin (e.g., SEQ ID NO: 2) and insulin. Alternatively multiple N-SSVs encoding different self-polypeptides may be administered. In preferred embodiments the N-SSV administered encodes a non-secreted version of preproinsulin, proinsulin (*e.g.*, SEQ ID NO: 2), in which the signal sequence of preproinsulin is eliminated.

### Non-secreted high expression self-vector (N-SHESV)

In other uses of the present invention, the improved method for treating or preventing an autoimmune disease includes administering to a subject an effective amount of a modified self-vector that is altered to increase expression of an intracellular or non-secreted version of an extracellular or secreted self-polypeptide associated with IDDM where both expression and secretion are relative to the unmodified self-vector. A modified self-vector that is altered to increase expression of an encoded intracellular or non-secreted version of an extracellular or secreted self-polypeptide is referred to as a non-secreted high expression self-vector (N-SHESV). A N-SHESV comprises a polynucleotide encoding and capable of expressing a secreted self-polypeptide associated with IDDM and a modification to generate increased expression of the self-polypeptide in a non-secreted form relative to the unmodified self-vector.

In certain embodiments of the present invention improved methods for treating or preventing an autoimmune disease such as insulin-dependent diabetes mellitus (IDDM) are provided comprising administering to a subject a modified self-vector that is altered to both increase expression of an encoded self-polypeptide associated with IDDM relative to an unmodified self-vector encoding the same self-polypeptide and to express a non-secreted form of a secreted autoantigen associated with IDDM such that secretion is prevented from a host cell relative to an unmodified self-vector. In some embodiments the modified self-vector that increases expression of a non-secreted self-polypeptide is a N-SHESV that contains an intron downstream of the promoter region and immediately upstream of the start codon of the encoded non-secreted form of a secreted self-polypeptide associated with IDDM. The N-SHESV administered to treat or prevent IDDM may include polynucleotides that encode one or more of proinsulin (*e.g*., SEQ ID NO: 2) and insulin Alternatively multiple N-SHESVs encoding different self-polypeptides may be administered. In preferred embodiments the N-SHESV administered to treat or prevent IDDM contains
a β-globin/Ig chimeric intron upstream of the start codon of a polynucleotide that encodes the self-polypeptide proinsulin (SEQ ID NO: 2), lacking the signal sequence of preproinsulin. In a more preferred embodiment the N-SHESV administered to treat or prevent IDDM contains the β-globin/Ig chimeric intron upstream of the start codon of a polynucleotide that encodes the self-polypeptide proinsulin (*e.g.*, SEQ ID NO: 2), lacking the signal sequence of preproinsulin.

### Secreted self-vector (SSV)

In other non-mutually exclusive embodiments, the improved method for treating or preventing IDDM includes administering to a subject an effective amount of a modified self-vector that contains a polynucleotide encoding a secreted form of a self protein, -polypeptide, or -peptide that is typically not secreted.
A modified self-vector altered to encode a secreted form of a self-protein, -polypeptide, or -peptide that is not secreted, e.g. a transmembrane or intracellular self-polypeptide, is referred to herein as a secreted self-vector (SSV).

In certain variations, the secreted self-polypeptide encoded by a SSV is an altered form of an intracellular self-protein(s), -polypeptide(s), or-peptide(s) that includes a modification allowing secretion of the self-polypeptide from a host cell. One particularly suitable modification for generating a SSV is the addition of an N-terminal signal sequence to the intracellular self-polypeptide to allow for secretion from the host cell. The signal sequence from any endogenously secreted protein may be used, or chimeric and/or consensus versions thereof. In addition to the signal sequence, the modification may further include signals for membrane association including, for example, a transmembrane domain or a GPI anchor so that intracellular epitope(s) are presented extracellularly. (A GPI anchor (phosphatidyl-inositol glycane) is a common modification of the C-terminus of membrane-attached proteins. It is composed of a hydrorophobic *phosphatidyl inositol* group linked through a carbohydrate containing linker (glucosamine and mannose linked to phosphoryl ethanolamine residue) to the C-terminal amino acid of a mature protein. The two fatty acids within the hydrophobic inositol group anchor the protein to the membrane.) In other variations the secreted self-polypeptide encoded by a SSV is an altered form of a transmembrane self-polypeptide that includes a modification to allow for secretion of the intracellular portion of the self-polypeptide with or without the extracellular portion of the self-polypeptide. One suitable modification includes removal of the transmembrane domain. Alternatively the extracellular portion of the transmembrane self-polypeptide is removed and a signal sequence is added to the N-terminus of the intracellular portion. In other variations the secreted self-polypeptide encoded by a SSV includes a modification to allow secretion of the extracellular portion of a transmembrane or membrane bound (i.e. GPI linked) self polypeptide in soluble form with or without the intracellular portion. One suitable modification includes removal of the transmembrane domain or the GPI linkage. In preferred embodiments the transmembrane and intracellular domains are removed to allow secretion of the extracellular portion of the self-polypeptide in soluble form.

In certain embodiments the present invention provides improved methods for treating or preventing an autoimmune disease such as insulin-dependent diabetes mellitus (IDDM) comprising administering to a subject a modified self-vector encoding and capable of expressing a self-polypeptide that includes one or more autoantigenic epitopes associated with IDDM. The improved method for treating or preventing IDDM includes administering to a subject an effective amount of a modified self-vector that encodes a secreted self-polypeptide version of a membrane associated or intracellular self-protein(s), -polypeptide(s), or -peptide(s) associated with IDDM.

### Secreted high expression self-vector (SHESV)

In other embodiments of the present invention, the improved method for treating or preventing an autoimmune disease includes administering to a subject an effective amount of a modified self-vector that is altered to increase expression of a secreted version of a membrane associated or intracellular self-polypeptide associated with an autoimmune disease where both expression and secretion are relative to the unmodified self-vector. A modified self-vector that is altered to increase expression of an encoded secreted version of a membrane associated or intracellular self-polypeptide is referred to as a secreted high expression self-vector (SHESV). A SHESV comprises a polynucleotide encoding and capable of expressing a membrane associated or intracellular self-polypeptide associated with an autoimmune disease and a modification to generate increased expression of the self-polypeptide in a secreted form compared to the unmodified self-vector. A SHESV further comprises in operative combination: a promoter; a polynucleotide encoding a membrane associated or intracellular self-polypeptide that includes at least one autoantigenic epitope associated with the autoimmune disease; a transcription terminator; and at least one modification for generating increased expression of the self-polypeptide and at least one modification to allow secretion of the self-polypeptide from a host cell where both modifications are relative to an unmodified self-vector comprising the promoter, polynucleotide, and transcription terminator.

Modifications to a self-vector to alter the expression level and/or the secretion of encoded self-protein(s), -polypeptide(s), or -peptide(s) relative to an unmodified self-vector can be determined *in vitro* using well-known methods for expression of recombinant proteins in host cells. Host cells such as primary mammalian cells or cell lines including, for example, HEK293, COS, or CHO cells are transfected with either the unmodified self-vector or with the modified self-vector having one or more of the following alternations: 1) at least one modification to generate increased expression of the self-polypeptide, 2) at least one modification to express a non-secreted or non-membrane bound form of a secreted or membrane bound self-polypeptide; or 3) at least one modification to allow secretion of a non-secreted self-polypeptide. The transfected host cells are then cultured in conditions that allow for expression of the self-polypeptide. Typically, the host cells and culture conditions are designed to mimic or approximate physiological conditions *in vivo*. Relative protein expression levels of the encoded self-polypeptide in host cells transfected with an unmodified self-vector verses a modified self-vector with at least one alteration to increase expression of the encoded self-polypeptide are then determined using known methods such as, for example, Western blot analysis, ELISA, or FACS. In preferred embodiments the protein expression of a HESV is between approximately 2- and 50-fold higher than the unmodified self-vector; in more preferred embodiments the increased expression is between approximately 5- and 35-fold higher than the unmodified self-vector; and in most preferred embodiments the increased expression is between approximately 10- and 30-fold higher than the unmodified self-vector as determined by ELISA. In addition the secretion versus non-secretion of a self-polypeptide can be determined by comparing protein levels in the culture medium, or supernatant, and protein levels in cell lysates. Furthermore, relative protein localization may be determined, for example, using immunohistochemistry or immunofluorescence. In preferred embodiments the non-secretion of a secreted self-polypeptide encoded by a N-SSV is virtually complete so that protein levels in the supernatant of transfected cells are not significantly above background as determined by ELISA. In alternative preferred embodiments the secretion of a non-secreted self-polypeptide encoded by a SSV is found in the supernatant at levels above background as determined by ELISA.

The IMSs used in accordance with the present invention comprise the following core hexamer:
5'-purine-pyrimidine-[X]-[Y]-pyrimidine-pyrimidine-3'
   or
5'-purine-purine-[X]-[Y]-pyrimidine-pyrimidine-3';
wherein X and Y are any naturally occurring or synthetic nucleotide, except that X and Y cannot be cytosine-guanine.

The core hexamer of IMSs can be flanked 5' and/or 3' by any composition or number of nucleotides or nucleosides. Preferably, IMSs range between 6 and 100 base pairs in length, and most preferably 16-50 base pairs in length. IMSs can also be delivered as part of larger pieces of DNA, ranging from 100 to 100,000 base pairs. IMSs can be incorporated in, or already occur in, DNA plasmids, viral vectors and genomic DNA. Most preferably IMSs can also range from 6 (no flanking sequences) to 10,000 base pairs, or larger, in size. Sequences present which flank the hexamer core can be constructed to substantially match flanking sequences present in any known immunoinhibitory sequences (IIS). For example, the flanking sequences TGACTGTG-Pu-Pu-X-Y-Pyr-Pyr-AGAGATGA, where TGACTGTG and AGAGATGA are flanking sequences. Another preferred flanking sequence incorporates a series of pyrimidines (C, T, and U), either as an individual pyrimidine repeated two or more times, or a mixture of different pyrimidines two or more in length. Different flanking sequences have been used in testing inhibitory modulatory sequences. Further examples of flanking sequences for inhibitory oligonucleotides are contained in the following references: U.S. Patents, number 6,225,292 and 6,339,068, Zeuner et al., Arthritis and Rheumatism, 46:2219-24, 2002.

Particular IMSs suitable for administration with modified self-vectors of the invention include oligonucleotides containing the following hexamer sequences:
1. 5'-purine-pyrimidine-[X]-[Y]-pyrimidine-pyrimidine-3' IMSs containing GG dinucleotide cores: GTGGTT, ATGGTT, GCGGTT, ACGGTT, GTGGCT, ATGGCT, GCGGCT, ACGGCT, GTGGTC, ATGGTC, GCGGTC, ACGGTC, and so forth;
2. 5'-purine-pyrimidine-[X]-[Y]-pyrimidine-pyrimidine-3' IMSs containing GC dinucleotides cores: GTGCTT, ATGCTT, GCGCTT, ACGCTT, GTGCCT, ATGCCT, GCGCCT, ACGCCT, GTGCTC, ATGCTC, GCGCTC, ACGCTC, and so forth;
Guanine and inosine can substitue for adenine and/or uridine can substitute for cytosine or thymine and those substitutions can be made as set forth based on the guidelines above.

In certain embodiments of the present invention, the core hexamer region of the IMS is flanked at either the 5' or 3' end, or at both the 5' and 3' ends, by a polyG region. A "polyG region" or "polyG motif" as used herein means a nucleic acid region consisting of at least two (2) contiguous guanine bases, typically from 2 to 30 or from 2 to 20 contiguous guanines. In some embodiments, the polyG region has from 2 to 10, from 4 to 10, or from 4 to 8 contiguous guanine bases. In certain preferred embodiments, the flanking polyG region is adjacent to the core hexamer. In yet other embodiments, the polyG region is linked to the core hexamer by a non-polyG region (non-polyG linker); typically, the non-polyG linker region has no more than 6, more typically no more than 4 nucleotides, and most typically no more than 2 nucleotides.

IMSs also include suppressive oligonucleotides of at least eight nucleotides in length, wherein the oligonucleotide forms a G-tetrad with a circular dichroism (CD) value of greater than about 2.9 and the number of guanosines is at least two (International Patent Application No. WO 2004/012669). CD is defined as the differential absorption of left and right hand circularly polarized light. G-tetrads are G-rich DNA segments that allow complex secondary and/or tertiary structures. More specifically a G-tetrad 1) involves the planar association of four guanosines in a cyclic hydrogen bonding arrangement involving non-Watson Crick base-pairing and 2) requires two of more contiguous guanosines or a hexameric region in which over 50% of the bases are guanosines. Examples include an oligonucleotide with at least one and preferrably between two and twenty TTAGGG motifs. Other useful suppressive oligonucleotides include but are not limited to those that conform to one of the following: (TGGGCGGT)ₓ where x is preferrably between 2 and 100 and more preferrably between 2 and 20; GGGTGGGTGGGTATTACCATTA;TTAGGGTTAGGGTCAACCTTCA; or (G)GG(C/G)AAGCTGGACCTTGGGGG(G)

IMSs are preferentially oligonucleotides that contain unmethylated GpG oligonucleotides. Alternative embodiments include IMSs in which one or more adenine or cytosine residues are methylated. In eukaryotic cells, typically cytosine and adenine residues can be methylated.

IMSs can be stabilized and/or unstabilized oligonucleotides. Stabilized oligonucleotides mean oligonucleotides that are relatively resistant to *in vivo* degradation by exonucleases, endonucleases and other degradation pathways. Preferred stabilized oligonucleotides have modified phosphate backbones, and most preferred oligonucleotides have phophorothioate modified phosphate backbones in which at least one of the phosphate oxygens is replaced by sulfur. Backbone phosphate group modifications, including methylphosphonate, phosphorothioate, phophoroamidate and phosphorodithionate internucleotide linkages, can provide antimicrobial properties on IMSs. The IMSs are preferably stabilized oligonucleotides, preferentially using phosphorothioate stabilized oligonucleotides.

Alternative stabilized oligonucleotides include: alkylphosphotriesters and phosphodiesters, in which the charged oxygen is alkylated; arylphosphonates and alkylphosphonates, which are nonionic DNA analogs in which the charged phosphonate oxygen is replaced by an aryl or alkyl group; or/and oligonucleotides containing hexaethyleneglycol or tetraethyleneglycol, or another diol, at either or both termini. Alternative steric configurations can be used to attach sugar moieties to nucleoside bases in IMSs.

The nucleotide bases of the IMS which flank the modulating dinucleotides may be the known naturally occurring bases or synthetic non-natural bases. Oligonucleosides may be incorporated into the internal region and/or termini of the IMS using conventional techniques for use as attachment points, that is as a means of attaching or linking another molecule, including, for example, a lipid, protein, peptide, glycolipid, carbohydrate, glycoprotein, or an additional immune modulatory therapeutic. The bases, sugar moieties, phosphate groups, and/or termini of the IMS may also be modified in any manner known to those of ordinary skill in the art to construct an IMS having properties desired in addition to the modulatory activity of the IMS. For example, sugar moieties may be attached to nucleotide bases of IMS in any steric configuration.

The techniques for making these phosphate group modifications to oligonucleotides are known in the art and do not require detailed explanation. For review of one such useful technique, the intermediate phosphate triester for the target oligonucleotide product is prepared and oxidized to the naturally occurring phosphate triester with aqueous iodine or with other agents, such as anhydrous amines. The resulting oligonucleotide phosphoramidates can be treated with sulfur to yield phophorothioates. The same general technique (excepting the sulfur treatment step) can be applied to yield methylphosphoamidites from methylphosphonates. Further details concerning phosphate group modification techniques are described in, *e.g.,* U.S. Patent Nos. 4,425,732, 4,458,066, 5,218,103, and 5,453,496; Tetrahedron Lett. at 21:4149 25 (1995), 7:5575 (1986), and 25:1437 (1984); and Journal Am. ChemSoc., 93:6657 (1987),

A particularly useful phosphate group modification is the conversion to the phosphorothioate or phosphorodithioate forms of the IMS oligonucleotides. Phosphorothioates and phosphorodithioates are more resistant to degradation *in vivo* than their unmodified oligonucleotide counterparts, making the IMS oligonucleotides of the invention more available to the host.

IMS oligonucleotides can be synthesized using techniques and nucleic acid synthesis equipment which are well-known in the art. (*See, e.g.,* Ausubel, et al., Current Protocols in Molecular Biology, Chs. 2 and 4 (Wiley Interscience, 1989); Maniatis, et al., Molecular Cloning: A Laboratory Manual (Cold Spring Harbor Lab., New York, 1982); U.S. Patent No. 4,458,066; and U.S. Patent No. 4,650,675,

Alternatively, immune inhibitory oligonucleotides can be obtained by mutation of isolated microbial immune stimulatory oligonucleotide to substitute a competing dinucleotide for the naturally occurring CpG motif within the flanking nucleotides. Screening procedures which rely on nucleic acid hybridization make it possible to isolate any polynucleotide sequence from any organism provided the appropriate probe or antibody is available. Oligonucleotide probes, which correspond to a part of the sequence encoding the protein in question, can be synthesized chemically. This requires that short, oligo-peptide stretches of amino acid sequence be known. The DNA sequence encoding the protein can also be deduced from the genetic code, though the degeneracy of the code must be taken into account.

For example, a cDNA library believed to contain an ISS-containing polynucleotide can be screened by injecting various mRNA derived from cDNAs into oocytes, allowing sufficient time for expression of the cDNA gene products to occur, and testing for the presence of the desired cDNA expression product, for example, by using antibody specific for a peptide encoded by the polynucleotide of interest or by using probes for the repeat motifs and a tissue expression pattern characteristic of a peptide encoded by the polynucelotide of interest. Alternatively, a cDNA library can be screened indirectly for expression of peptides of interest having at least one epitope using antibodies specific for the peptides. Such antibodies can be either polyclonally or monoclonally derived and used to detect expression product indicative of the presence of cDNA of interest.

Once the immune stimulatory sequence-containing polynucleotide has been obtained, it can be shortened to the desired length by, for example, enzymatic digestion using conventional techniques. The CpG motif in the immune stimulatory sequence oligonucleotide product is then mutated to substitute an "inhibiting" dinucleotide - identified using the methods of this invention- for the CpG motif. Techniques for making substitution mutations at particular sites in DNA having a known sequence are well known, for example M13 primer mutagenesis through PCR. Because the IMS is non-coding, there is no concern about maintaining an open reading frame in making the substitution mutation. However, for *in vivo* use, the polynucleotide starting material, immune stimulatory sequence intermediate, or IMS mutation product should be rendered substantially pure (*i.e.,* as free of naturally occurring contaminants and LPS as is possible using available techniques known to and chosen by one of ordinary skill in the art).

The IMS of the invention may be used alone or may be incorporated into a recombinant self-vector (plasmid, cosmid, virus or retrovirus) which may in turn code for a polypeptide deliverable by a recombinant expression vector. Incorporation may be accomplished using conventional techniques as known to one of ordinary skill in the art. (*See generally, e.g.,* Ausubel, *Current Protocols in Molecular Biology, supra. See also* Sambrook and Russell, Molecular Cloning, A Laboratory Manual (3rd ed. 2001); Sambrook et al., Molecular Cloning, A Laboratory Manual (2nd ed. 1989).)

Techniques for construction of vectors and transfection of cells are well-known in the art, and the skilled artisan will be familiar with the standard resource materials that describe specific conditions and procedures. The self-vector encoding a self-polypeptide is prepared and isolated using commonly available techniques for isolation of nucleic acids. The vector is purified free of bacterial endotoxin for delivery to humans as a therapeutic agent.

Construction of the vectors of the invention employs standard ligation and restriction techniques that are well-known in the art (*see generally, e.g.,* Ausubel *et al., supra;* Sambrook and Russell, *supra;* Sambrook, *supra).* Isolated plasmids, DNA sequences, or synthesized oligonucleotides are cleaved, tailored, and relegated in the form desired. Sequences of DNA constructs can be confirmed using, *e.g.*, standard methods for DNA sequence analysis (*see, e.g.,* Sanger et al. (1977) Proc. Natl. Acad. Sci., 74, 5463-5467).

One particularly suitable nucleic acid vector useful in accordance with the methods provided herein is a nucleic acid expression vector in which a non-CpG dinucleotide is substituted for one or more CpG dinucleotides of the formula 5'-purine-pyrimidine-C-G-pyrimidine-pyrimidine-3' or 5'-purine-purine-C-G-pyrimidine-pyrimidine-3', thereby producing a vector in which immunostimulatory activity is reduced. For example, the cytosine of the CpG dinucleotide can be substituted with guanine, thereby yielding an IMS region having a GpG motif of the formula 5'-purine-pyrimidine-G-G-pyrimidine-pyrimidine-3' or 5'-purine-purine-G-G-pyrimidine-pyrimidine-3'. The cytosine can also be substituted with any other non-cytosine nucleotide. The substitution can be accomplished, for example, using site-directed mutagenesis. Typically, the substituted CpG motifs are those CpGs that are not located in important control regions of the vector (*e.g.*, promoter regions). In addition, where the CpG is located within a coding region of an expression vector, the non-cytosine substitution is typically selected to yield a silent mutation or a codon corresponding to a conservative substitution of the encoded amino acid.

For example, in certain embodiments, the vector used for construction of the self-vector is a modified pVAX1 vector in which one or more CpG dinucleotides of the formula 5'-purine-pyrimidine-C-G-pyrimidine-pyrimidine-3' is mutated by substituting the cytosine of the CpG dinucleotide with a non-cytosine nucleotide. The pVAX1 vector is known in the art and is commercially available from Invitrogen (Carlsbad, CA). In one exemplary embodiment, the modified pVAX1 vector has the following cytosine to non-cytosine substitutions within a CpG motif: cytosine to guanine at nucleotides 784, 1161, 1218, and 1966; cytosine to adenine at nucleotides 1264, 1337, 1829, 1874, 1940, and 1997; and cytosine to thymine at nucleotides 1963 and 1987; with additional cytosine to guanine mutations at nucleotides 1831, 1876, 1942, and 1999. (The nucleotide number designations as set forth above are according to the numbering system for pVAX1 provided by Invitrogen.) The vector thus constructed was named pBHT1.

Nucleotide sequences selected for use in the self-vector can be derived from known sources, for example, by isolating the nucleic acid from cells containing a desired gene or nucleotide sequence using standard techniques. Similarly, the nucleotide sequences can be generated synthetically using standard modes of polynucleotide synthesis that are well known in the art. *See, e.g.,* Edge et al., Nature 292:756, 1981; Nambair et al., Science 223:1299, 1984; Jay et al., J. Biol. Chem. 259:6311, 1984. Generally, synthetic oligonucleotides can be prepared by either the phosphotriester method as described by Edge *et al.* (supra) and Duckworth et al. (Nucleic Acids Res. 9:1691, 1981); or the phosphoramidite method as described by Beaucage et al. (Tet. Letts. 22:1859, 1981) and Matteucci et al. (J. Am. Chem. Soc. 103:3185, 1981). Synthetic oligonucleotides can also be prepared using commercially available automated oligonucleotide synthesizers. The nucleotide sequences can thus be designed with appropriate codons for a particular amino acid sequence. In general, one will select preferred codons for expression in the intended host. The complete sequence is assembled from overlapping oligonucleotides prepared by standard methods and assembled into a complete coding sequence. *See, e.g.,* Edge *et al.* (*supra*); Nambair *et al.* (*supra*) and Jay *et al.* (*supra*).

Another method for obtaining nucleic acid sequences for use herein is by recombinant means. Thus, a desired nucleotide sequence can be excised from a plasmid carrying the nucleic acid using standard restriction enzymes and procedures. Site specific DNA cleavage is performed by treating with the suitable restriction enzymes and procedures. Site specific DNA cleavage is performed under conditions which are generally understood in the art, and the particulars of which are specified by manufacturers of commercially available restriction enzymes. If desired, size separation of the cleaved fragments may be performed by polyacrylamide gel or agarose gel electrophoreses using standard techniques.

Yet another convenient method for isolating specific nucleic acid molecules is by the polymerase chain reaction (PCR) (Mullis et al., Methods Enzymol. 155:335-350, 1987) or reverse transcription PCR (RT-PCR). Specific nucleic acid sequences can be isolated from RNA by RT-PCR. RNA is isolated from, for example, cells, tissues, or whole organisms by techniques known to one skilled in the art. Complementary DNA (cDNA) is then generated using poly-dT or random hexamer primers, deoxynucleotides, and a suitable reverse transcriptase enzymes. The desired polynucleotide can then be amplified from the generated cDNA by PCR. Alternatively, the polynucleotide of interest can be directly amplified from an appropriate cDNA library. Primers that hybridize with both the 5' and 3' ends of the polynucleotide sequence of interest are synthesized and used for the PCR. The primers may also contain specific restriction enzyme sites at the 5' end for easy digestion and ligation of amplified sequence into a similarly restriction digested plasmid vector.

The expression cassette of the modified self-vector will employ a promoter that is functional in host cells. In general, vectors containing promoters and control sequences that are derived from species compatible with the host cell are used with the particular host cell. Promoters suitable for use with prokaryotic hosts illustratively include the beta-lactamase and lactose promoter systems, alkaline phosphatase, the tryptophan (trp) promoter system and hybrid promoters such as tac promoter. However, other functional bacterial promoters are suitable. In addition to prokaryotes, eukaryotic microbes such as yeast cultures may also be used. *Saccharomyces cerevisiae,* or common baker's yeast is the most commonly used eukaryotic microorganism, although a number of other strains are commonly available. Promoters controlling transcription from vectors in mammalian host cells may be obtained from various sources, for example, the genomes of viruses such as: polyoma, simian virus 40 (SV40), adenovirus, retroviruses, hepatitis B virus and preferably cytomegalovirus (CMV), or from heterologous mammalian promoters, *e.g*. β-actin promoter. The early and late promoters of the SV 40 virus are conveniently obtained as an SV40 restriction fragment which also contains the SV40 viral origin of replication. The immediate early promoter of the human cytomegalovirus is conveniently obtained as a HindIII restriction fragment. Of course, promoters from the host cell or related species also are useful herein.

In one embodiment, DNA encoding two or more self-protein(s), -polypeptide(s), or -peptide(s) are encoded sequentially in a single self-vector utilizing internal ribosomal re-entry sequences (IRES) or other elements for expression of multiple proteins from a single DNA molecule.

The vectors used herein may contain a selection gene, also termed a selectable marker. A selection gene encodes a protein, necessary for the survival or growth of a host cell transformed with the vector. Examples of suitable selectable markers for mammalian cells include the dihydrofolate reductase gene (DHFR), the ornithine decarboxylase gene, the multi-drug resistance gene (mdr), the adenosine deaminase gene, and the glutamine synthase gene. When such selectable markers are successfully transferred into a mammalian host cell, the transformed mammalian host cell can survive if placed under selective pressure. There are two widely used distinct categories of selective regimes. The first category is based on a cell's metabolism and the use of a mutant cell line which lacks the ability to grow independent of a supplemented media. The second category is referred to as dominant selection which refers to a selection scheme used in any cell type and does not require the use of a mutant cell line. These schemes typically use a drug to arrest growth of a host cell. Those cells which have a novel gene would express a protein conveying drug resistance and would survive the selection. Examples of such dominant selection use the drugs neomycin (Southern and Berg (1982) J. Molec. Appl. Genet. 1, 327), mycophenolic acid (Mulligan and Berg (1980) Science 209, 1422), or hygromycin (Sugden et al. (1985) Mol. Cell. Bio. 5, 410-413). The three examples given above employ bacterial genes under eukaryotic control to convey resistance to the appropriate drug neomycin (G418 or genticin), xgpt (mycophenolic acid) or hygromycin, respectively.

Alternatively the vectors used herein are propagated in a host cell using antibiotic-free selection based on repressor titration (Cranenburgh et al., 2001). The vectors are modified to contain the *lac* operon either as part of the *lac* promoter or with the *lacO₁* and *lacO₃* operators with the optimal spacing found in the pUC series of plasmid vectors. Alternatively the *lacO₁* operator or palindromic versions of the *lacO* can be used in isolation as single or multiple copies (Cranenburgh et al., 2004). The *lac* operon sequence may be incorporated at single or multiple sites anywhere within the vector so as not to interfere with other functional components of the vector. In preferred embodiments a synthetic *Escherichia coli lac* operon dimer operator (Genbank Acc. Num. K02913) is used. The *lac* operon may be added to a vector that lacks a suitable selective marker to provide selection, be added in addition to another selectable marker, or used to replace a selectable marker, especially an antibiotic resistance marker, to make the vector more suitable for therapeutic applications. Vectors containing the *lac* operon can be selected in genetically modified *E. coli* with an essential gene, including *dapD,* under the control of the *lac* promoter (*lacOP*) thus allowing the modified host cell to survive by titrating the *lac* repression from the *lacOP* and allowing expression of *dapD.* Suitable *E. coli* stains include DH1*lacdapD* and DH1*lacP2dapD*

### (Cranenburgh et al., 2001)

For *in vitro* evaluation, host cells may be transformed with the modified self-vector and cultured in conventional nutrient media modified as is appropriate for inducing promoters, selecting transformants or amplifying genes. One suitable method for transfection of the host cells is the calcium phosphate co-precipitation method of Graham and van der Eb (1973) Virology 52, 456-457. Alternative methods for transfection are electroporation, the DEAE-dextran method, lipofection and biolistics (Kriegler (1990) Gene Transfer and Expression: A Laboratory Manual, Stockton Press). Culture conditions, such as temperature, pH and the like, that are suitable for host cell expression are generally known in the art and will be apparent to the skilled artisan.

If a recombinant expression vector is utilized as a carrier for the IMS-ODN of the invention, plasmids and cosmids are particularly preferred for their lack of pathogenicity. However, plasmids and cosmids are subject to degradation *in vivo* more quickly than viruses and therefore may not deliver an adequate dosage of IMS-ODN to prevent or treat an inflammatory or autoimmune disease.

Modified self-vectors of this invention can be formulated as polynucleotide salts for use as pharmaceuticals. Polynucleotide salts can be prepared with non-toxic inorganic or organic bases. Inorganic base salts include sodium, potassium, zinc, calcium, aluminum, magnesium, etc. Organic non-toxic bases include salts of primary, secondary and tertiary amines, etc. Such self-DNA polynucleotide salts can be formulated in lyophilized form for reconstitution prior to delivery, such as sterile water or a salt solution. Alternatively, self-DNA polynucleotide salts can be formulated in solutions, suspensions, or emulsions involving water- or oil-based vehicles for delivery. In one preferred embodiment, the DNA is lyophilized in phosphate buffered saline with physiologic levels of calcium (0.9 mM) and then reconstituted with sterile water prior to administration. Alternatively the DNA is formulated in solutions containing higher quantities of Ca⁺⁺, between 1 mM and 2M. The DNA can also be formulated in the absence of specific ion species.

A wide variety of methods exist to deliver polynucleotide to subjects, as defined herein. For example, the polynucleotide encoding a self-polypeptide can be formulated with cationic polymers including cationic liposomes. Other liposomes also represent effective means to formulate and deliver self-polynucleotide. Alternatively, the self DNA can be incorporated into a viral vector, viral particle, or bacterium for pharmacologic delivery. Viral vectors can be infection competent, attenuated (with mutations that reduce capacity to induce disease), or replication-deficient. Methods utilizing self-DNA to prevent the deposition, accumulation, or activity of pathogenic self proteins may be enhanced by use of viral vectors or other delivery systems that increase humoral responses against the encoded self-protein. In other embodiments, the DNA can be conjugated to solid supports including gold particles, polysaccharide-based supports, or other particles or beads that can be injected, inhaled, or delivered by particle bombardment (ballistic delivery). Methods for delivering nucleic acid preparations are known in the art. *See, e.g*.; U.S. Patent Nos. 5,399,346, 5,580,859, and 5,589,466. A number of viral based systems have been developed for transfer into mammalian cells. For example, retroviral systems have been described (U.S. Patent No. 5,219,740; Miller et al., Biotechniques 7:980-990, 1989; Miller, Human Gene Therapy 1:5-14, 1990; Scarpa et al., Virology 180:849-852, 1991; Bums et al., Proc. Natl. Acad. Sci. USA 90:8033-8037, 1993; and, Boris-Lawrie and Temin, Cur. Opin. Gent. Develop. 3:102-109, 1993). A number of adenovirus vectors have also been described, *see e.g.,* (Haj-Ahmad et al., J. Virol. 57:267-274, 1986; Bett et al., J. Virol. 67:5911-5921, 1993; Mittereder et al., Human Gene Therapy 5:717-729, 1994; Seth et al., J. Virol 68:933-940, 1994; Barr et al., Gene Therapy 1:51-58, 1994; Berkner, BioTechniques 6:616-629, 1988; and, Rich et al., Human Gene Therapy 4:461-476, 1993). Adeno-associated virus (AAV) vector systems have also been developed for nucleic acid delivery. AAV vectors can be readily constructed using techniques well known in the art. *See, e.g.,* U.S. Patent Nos. 5,173,414 and 5,139,941; International Publication Nos. WO 92/01070 and WO 93/03769; Lebkowski et al., Molec. Cell. Biol. 8:3988-3996, 1988; Vincent et al., Vaccines 90 (Cold Spring Harbor Laboratory Press) 1990; Carter, Current Opinion in Biotechnology 3:533-539, 1992; Muzyczka, Current Topics in Microbiol. And Immunol. 158:97-129, 1992; Kotin, Human Gene Therapy 5:793-801,1994; Shelling et al., Gene Therapy 1:165-169, 1994; and, Zhou et al., J. Exp. Med. 179:1867-1875, 1994).

The polynucleotide of this invention can also be delivered without a viral vector. For example, the molecule can be packaged in liposomes prior to delivery to the subject. Lipid encapsulation is generally accomplished using liposomes which are able to stably bind or entrap and retain nucleic acid. For a review of the use of liposomes as carriers for delivery of nucleic acids, *see, e.g.,* Hug et al., Biochim. Biophys. Acta. 1097:1-17, 1991; Straubinger et al., in Methods of Enzymology, Vol. 101, pp. 512-527, 1983.

Therapeutically effective amounts of self-vector are in the range of about 0.001 mg to about 1 g. A preferred therapeutic amount of self-vector is in the range of about 10 ng to about 10 mg. A most preferred therapeutic amount of self-vector is in the range of about 0.025 mg to 6 mg. In certain embodiments, the self-vector is administered monthly for 6-12 months, and then every 3-12 months as a maintenance dose. Alternative treatment regimens may be developed and may range from daily, to weekly, to every other month, to yearly, to a one-time administration depending upon the severity of the disease, the age of the patient, the self-polypeptide or polypeptides being administered, and such other factors as would be considered by the ordinary treating physician.

In one embodiment, the polynucleotide is delivered by intramuscular injection. In other variations, the polynucleotide is delivered intranasally, orally, subcutaneously, intradermally, intravenously, mucosally, impressed through the skin, or attached to gold particles delivered to or through the dermis (*see, e.g.,* WO 97/46253). Alternatively, nucleic acid can be delivered into skin cells by topical application with or without liposomes or charged lipids (*see e.g.* U.S. Patent No. 6,087,341). Yet another alternative is to deliver the nucleic acid as an inhaled agent. The polynucleotide is formulated in phosphate buffered saline with physiologic levels of calcium (0.9 mM). Alternatively, the polynucleotide is formulated in solutions containing higher quantities of Ca⁺⁺, between 1 mM and 2M. The polynucleotide may be formulated with other cations such as zinc, aluminum, and others. Alternatively, or in addition, the polynucleotide may be formulated either with a cationic polymer, cationic liposome-forming compounds, or in non-cationic liposomes. Examples of cationic liposomes for DNA delivery include liposomes generated using 1,2-bis(oleoyloxy)-3-(trimethylammionio) propane (DOTAP) and other such molecules.

Prior to delivery of the polynucleotide, the delivery site can be preconditioned by treatment with bupivicane, cardiotoxin or another agent that may enhance the subsequent delivery of the polynucleotide. Such preconditioning regimens are generally delivered 12 to 96 hours prior to delivery of therapeutic polynucleotide; more frequently 24 to 48 hours prior to delivery of the therapeutic polynucleotide. Alternatively, no preconditioning treatment is given prior to polynucleotide therapy.

In alternative variations, the method for treating autoimmune disease further includes the administration of an adjuvant for modulating the immune response comprising a CpG oligonucleotide in order to enhance the immune response. CpG oligonucleotides or stimulatory IMSs have been shown to enhance the antibody response of DNA vaccinations (Krieg et al., Nature 374:546-9, 1995). The CpG oligonucleotides will consist of a purified oligonucleotide of a backbone that is resistant to degradation *in vivo* such as a phosphorothioated backbone. The stimulatory IMS useful in accordance with the present invention comprise the following core hexamer:
5'-purine-pyrimidine-[C]-[G]-pyrimidine-pyrimidine-3'
   or
5'-purine-purine-[C]-[G]-pyrimidine-pyrimidine-3';

The core hexamer of immune stimulatory IMSs can be flanked 5' and/or 3' by any composition or number of nucleotides or nucleosides. Preferably, stimulatory IMSs range between 6 and I00 base pairs in length, and most preferably 16-50 base pairs in length. Stimulatory IMSs can also be delivered as part of larger pieces of DNA, ranging from 100 to 100,000 base pairs. Stimulatory IMSs can be incorporated in, or already occur in, DNA plasmids, viral vectors and genomic DNA. Most preferably stimulatory IMSs can also range from 6 (no flanking sequences) to 10,000 base pairs, or larger, in size. Sequences present which flank the hexamer core can be constructed to substantially match flanking sequences present in any known immunostimulatory sequences (ISS). For example, the flanking sequences TGACTGTG-Pu-Pu-C-G-Pyr-Pyr-AGAGATGA, where TGACTGTG and AGAGATGA are flanking sequences. Another preferred flanking sequence incorporates a series of pyrimidines (C, T, and U), either as an individual pyrimidine repeated two or more times, or a mixture of different pyrimidines two or more in length. Different flanking sequences have been used in testing inhibitory modulatory sequences and can be adapted to stimulatory modulatory sequences. Further examples of flanking sequences are contained in the following references: U.S. Patents, number 6,225,292 and 6,339,068, Zeuner et al., Arthritis and Rheumatism, 46:2219-24, 2002.

Particular stimulatory inhibitory IMSs suitable for administration with modified self-vectors of the invention include oligonucleotides containing the following hexamer sequences:
1. 5'-purine-pyrimidine-[X]-[Y]-pyrimidine-pyrimidine-3' IMSs containing CG dinucleotide cores: GTCGTT, ATCGTT, GCCGTT, ACCGTT, GTCGCT, ATCGCT, GCCGCT, ACCGCT, GTCGTC, ATCGTC, GCCGTC, ACCGTC, and so forth;
Guanine and inosine can substitue for adenine and/or uridine can substitute for cytosine or thymine and those substitutions can be made as set forth based on the guidelines above. Alternatively ISS-ODNs can be included into self-vectors as described in detail for IMSs above. A particularly useful ISS includes the mouse optimal CpG element AACGTT. A single ISS or multiple ISSs can be added to a modified self-vector at a single or at multiple sites in the vector as long as other functional electors are not disrupted. In one exemplary example the ISS added to a modified self-vector include a cluster of five mouse optimal CpG elements (AACGTT) immediately upstream of the promoter.

The self-vector can be administered in combination with other substances, such as, for example, pharmacological agents, adjuvants, cytokines, or vectors encoding cytokines. Furthermore, to avoid the possibility of eliciting unwanted anti-self cytokine responses when using cytokine codelivery, chemical immunomodulatory agents such as the active form of vitamin D3 can also be used. In this regard, 1,25-dihydroxy vitamin D3 has been shown to exert an adjuvant effect via intramuscular DNA immunization.

A polynucleotide coding for a protein known to modulate a host's immune response (e.g., an cytokine) can be coadministered with the self vector. Accordingly, a gene encoding an immunomodulatory cytokine (*e.g*., an interleukin, interferon, or colony stimulating factor), or a functional fragment thereof, may be used in accordance with the instant invention. Gene sequences for a number of these cytokines are known. Thus, in one embodiment of the present invention, delivery of a self-vector is coupled with coadministration of at least one of the following immunomodulatory proteins, or a polynucleotide encoding the protein(s): IL-4; IL-10; IL-13; and IFN-γ.

### EXAMPLES

The following examples are specific embodiments for carrying out the present invention. The examples are offered for illustrative purposes only, and are not intended to limit the scope of the present invention in any way.

### Example 1

### Treatment of established hyperglycemia in NOD mice by DNA vaccination with a self vector encoding preproinsulin II

Non-obese diabetic (NOD) mice develop spontaneous autoimmune diabetes that shares many clinical, immunological, and histopathological features with human insulin-dependent diabetes mellitus (IDDM). Diabetes onset in the NOD mouse can be prevented by DNA vaccination with a self-vector encoding a peptide fragment of insulin B (Urbanek-Ruiz et al., 2001). In this study, the ability of DNA vaccination with self-vectors encoding murine preproinsulin I or preproinsulin II to prevent diabetes in NOD mice with established hyperglycemia was investigated.

Self-vectors encoding murine preproinsulin I and preproinsulin II were generated. Polynucleotide encoding full-length preproinsulin I (ppINS-I) or preproinsulin II (ppINS-II) were amplified by PCR and ligated into the multicloning site of pBHT1 to generate mINS-I-pBHT1 and mINS-II-pBHT1 (pBHT500) respectively. pBHT1 is a modified pVAX1 mammalian expression vector that retains the main structural features of its parent plasmid (Invitrogen, Carlsbad, CA) including a CMV immediate-early promoter/enhancer, a bovine growth hormone polyadenylation sequence, a kanamycin resistance gene for bacterial selection, and a pUC origin of replication. pVAX1 was modified to pBHT1 by the elimination of 12 out of 29 immunostimulatory CpG sequences (sequences with the consensus motif: RYCpGYY; R=A or G, Y=C or T) in the polynucleotide sequence of the vector using site-directed mutagenesis techniques. A non-coding pBHT1 vector was used as a control DNA for vaccination. The DNA was purified using Qiagen Endo-free Mega-preps (Qiagen, Valencia, CA) according to the manufacturer's protocol to provide a substantially endotoxin free self-vector for administration.

Treatment of NOD mice began only after the mice became hyperglycemic with blood glucose levels reaching 190-250 mg/dl (typically at 15-18 weeks of age) as determined by plasma glucose measurements using the One Touch II meter (Johnson & Johnson, Milpitas, CA). Mice with such overt clinical pre-diabetes were then injected in each quadricep with 0.05 ml of 0.25% bupivicaine-HCL (Sigma, St. Louis, MO). Two days later, the mice were administered intramuscularly ppINS-I, ppINS-II, or non-coding pBHT1 vector in phosphate buffered saline (PBS) with 0.9 mM calcium in each quadricep for a total of 50 ug/animal. DNA injections were continued bi-weekly for 8 weeks. As a positive control anti-CD3 antibodies were administered at 5 ug/animal by IV injection for 5 consecutive days (Bisikirska & Herold, 2004). Mice were tested weekly for glucosuria by Chemstrip (Boehringer Mannheim Co., Indianapolis, IN) and diabetes was confirmed by plasma glucose measurement using the One Touch II meter (Johnson & Johnson, Milpitas, CA). Progression of diabetes was defined as two consecutive blood glucose measurements greater than 250 mg/dl.

The results shown in Figure 1 revealed that DNA vaccination with a self-vector encoding murine preproinsulin II reduced the progression of diabetes in hyperglycemic NOD mice significantly compared to PBS injected controls. Only slightly over 50% of mice injected with ppINS-II progressed to diabetes. In contrast, mice injected with a self-vector encoding either murine preproinsulin I or a non-coding pBHT1 vector progressed to diabetes at the same rate as control PBS injected animals with over 80% of animals progressing to diabetes by the 8^{th} week. Furthermore, a correlation between the clinical effect and a reduction in insulin autoantibody titers was observed as shown in Figure 2. Sera from mice were taken at the end of the study, and anti-insulin autoantibody titers measured by radioimmunoassay. Mice vaccinated with a self-vector encoding murine preproinsulin II showed reduced mean insulin autoantibody indices as compared to untreated mice, similar to anti-CD3 positive control treated animals.

### Example 2

### Effect of DNA vaccination with a self-vector encoding preproinsulin II on subsequent immune response

This study investigated whether DNA vaccination with a self-vector affects a subsequent immune response. NOD mice were vaccinated with a self-vector encoding murine preproinsulin II before being challenged with a peptide fragment of insulin II (insulin II 9-23). The subsequent immune response to restimulation with the insulin 9-23 peptide was then examined.

Female NOD mice were treated at 6 weeks of age before signs of hyperglycemia. Mice were injected in each quadricep with 0.05 ml of 0.25% bupivicaine-HCL (Sigma, St. Louis, MO). Two days later the mice were administered intramuscularly 0.10 ml of mINS-II-pBHT1 at 250 ug/ml in PBS with 0.9 mM calcium in each quadricep for a total of 50 ug/animal. Animals were treated bi-weekly for a total of three injections. Two weeks after the last DNA injection DNA vaccinated and control animals were immunized with insulin II 9-23 peptide emulsified in incomplete Freund's adjuvant. Ten days after the immunization, the draining lymph nodes were harvested and ELISpots were performed. Cells that secreted interferon (IFN)-gamma in response to stimulation with insulin II 9-23 peptide were visualized using a Cellular Technology ELISpot Plate reader. A Student's t-test was performed to determine statistical significance. Administration of DNA encoding preproinsulin II significantly decreased the number cell that produced IFN-gamma upon restimulation with insulin II 9-23 peptide (Fig. 3) thus preventing progression to diabetes.

### Example 3

### Treatment of established hyperglycemia in NOD mice by DNA vaccination using a high expression self-vector (HESV) encoding preproinsulin II

This study investigated whether modifying the preproinsulin II DNA self-vector described in Example 1 to a HESV affects treatment efficacy of DNA vaccination to slow or prevent progression to diabetes. A high expression self-vector (HESV) was constructed that contains a β-globin/Ig chimeric intron upstream of a polynucleotide encoding preproinsulin II. This modified self-vector was then used to vaccinate NOD mice with established hyperglycemia.

A HESV encoding preproinsulin II was generated that differs from the self-vector encoding preproinsulin II (mINS-II-pBHT1) used in the study described in Example 1 in that it contains a chimeric β-globin/Ig intron downstream of the promoter region and immediately upstream of the preproinsulin start codon. As described above, full-length preproinsulin II was cloned into a modified pVAX1 plasmid vector, pBHT1, to generate the plasmid pBHT500. A plasmid derived from pBHT1 was constructed containing a chimeric intron from the commercially available vector pTarget (Promega, Madison, WI) downstream of the CMV promoter/enhancer region (pBHT520). The preproinsulin II coding sequence from pBHT500 was isolated by restriction nuclease digestion with HindIII and Xbal and ligated into pBHT520 resulting in the plasmid vector pBHT561, generating a HESV and referred to as mINS-II- HESV. The chimeric intron is composed of the 5' donor site from the first intron of the human β-globin gene and the branch and 3' acceptor site from the intron of an immunoglobulin gene heavy chain variable region. The donor, acceptor, and branchpoint site sequences were altered to match the consensus sequences for splicing (Bothwell et al., 1981).

Next it was demonstrated that the introduction of an intron to generate the high expression self-vector mINS-II-HESV (pBHT561) resulted in increased expression of encoded insulin compared to the unmodified self-vector mINS-pBHTI (pBHT500). The supernatant from equal numbers of HEK293 cells transfected with 0.1 ug of each plasmid was collected 24 hours post transfection, and insulin protein levels were determined by ELISA according to manufacturer's specifications (Rat/mouse insulin ELISA kit, Linco Research Inc., St. Charles, MI). Background levels in supernatant collected from "no DNA" control wells were subtracted from the protein levels detected for each plasmid. As shown in Figure 4 the addition of an intron to generate the HESV pBHT561 resulted in an approximately 30-fold increase in the amount of insulin protein secreted by cells compared to the unmodified pBHT500 self-vector.

Treatment of female NOD mice began only after the mice became hyperglycemic with blood glucose levels reaching 190-250 mg/dl (typically at 15-18 weeks of age) as determined by plasma glucose measurements using the One Touch II meter (Johnson & Johnson, Milpitas, CA). Mice with such overt clinical pre-diabetes were then injected in each quadricep with 0.05 ml of 0.25% bupivicaine-HCL (Sigma, St. Louis, MO). Two days later the mice (n=10 per group) were administered intramuscularly substantially endotoxin-free 0.10 ml of PBS, pBHT1 empty vector, mINS-II-pBHT1, or mINS-II-HESV at 250 ug/ml in PBS with 0.9 mM calcium in each quadricep for a total of 50 ug/animal. DNA injections were continued weekly thereafter for a total of 12 weeks. As a positive control anti-CD3 antibodies were administered at 5 ug/animal by IV injection for 5 consecutive days. Mice were tested weekly for glucosuria by Chemstrip (Boehringer Mannheim Co., Indianapolis, IN) and diabetes was confirmed by plasma glucose measurement using the One Touch II meter (Johnson & Johnson, Milpitas, CA). Progression to diabetes was defined as two consecutive blood glucose measurements greater than 250 mg/dl.

The results shown in Figure 5 reveal that DNA vaccination with the modified self-vector mINS-II-HESV reduced the development of diabetes in hyperglycemic NOD mice with less mice progressing to diabetes after 14 weeks and with those that did taking longer to progress compared to PBS (p=0.007) and pBHT1 empty vector injected controls. Furthermore, the treatment efficiency of mINS-II-HESV was also significantly greater than that of the unmodified self-vector mINS-II-pBHT1 suggesting that higher expression levels of preproinsulin II associated with introduction of a chimeric intron into a self-vector can further delay disease progression. Importantly, mINS-II-HESV treatment did not differ significantly from anti-CD3 treated positive controls (p=0.47).

### Example 4

### Treatment of established hyperglycemia in NOD mice by DNA vaccination using a non-secreted self-vector (N-SSV) encoding proinsulin II

This study investigated whether modifying the preproinsulin II DNA self-vector described in Example 1 to a N-SSV affects treatment efficacy of DNA vaccination to modulate the disease by slowing or preventing progression to diabetes. A non-secreted self-vector (N-SSV) was constructed to encode a non-secreted version of preproinsulin II, proinsulin II, by removing the signal sequence. This modified self-vector was then used to vaccinate NOD mice with established hyperglycemia.

A N-SSV encoding proinsulin II was generated that differs from the self-vector encoding preproinsulin II (mINS-II-pBHT1) as described in Example 1 in that proinsulin II lacks the signal peptide sequence of preproinsulin II. As described above, full-length preproinsulin II was cloned into a modified pVAX1 plasmid vector, pBHT1, to generate the plasmid pBHT500. The proinsulin region of mouse preproinsulin II from pBHT500 was PCR amplified using the oligonucleotides INS2.5.Eco (ATTGAATTCAAGATGGCTTTTGTCAAGCAGCACACCTTTG) and INS2.3.Xho (AATTCTCGAGCTAGTTGCAGTAGTTGTCCAGCT). A methionine start codon was incorporated into the 5' oligo at the N-terminus of the proinsulin sequence to replace the start codon from the deleted signal sequence. The PCR fragment was digested with EcoR1 and Xho1 and ligated into the corresponding sites of pBHT1 to generate pBHT555 (mINS-II-N-SSV). The DNA was purified using Qiagen Endo-free Mega-preps (Qiagen, Valencia, CA) according to the manufacturer's protocol.

Next it was demonstrated that the removal of the signal sequence from preproinsulin II to generate mINS-II-N-SSV resulted in the production of an intracellular form of insulin. HEK293 cells were transfected with 2 ug of the insulin expressing plasmids mINS-II-pBHT1 (pBHT500) and mINS-II-N-SSV (pBHT555). Transfected cells were incubated for 48 hours and insulin protein levels in the supernatant and cell lysates were analyzed at 48 hrs by ELISA (Fig. 6A). Significant amounts of protein were detected in the supernatant from mINS-II-pBHT1 transfected cells, but no protein was detected in the supernatant of cells transfected with mINS-II-N-SSV. Alternatively transfected cells were incubated for 24 hrs in normal media and then for 24 hrs in the presence of the proteasome inhibitor lactacystin (5uM) to promote steady state levels of intracellular insulin. Insulin protein levels at 48 hrs were measured by ELISA (Fig. 6B). Again significant amounts of protein were detected in the supernatant from mINS-II-pBHT1 transfected cells, but no protein was detected in the supernatant of cells transfected with mINS-II-N-SSV rather the protein was found in the cell lysate indicating the protein was confined to the cytoplasm.

Treatment of female NOD mice began only after the mice became hyperglycemic with blood glucose levels reaching 190-250 mg/dl (typically at 15-18 weeks of age) as determined by plasma glucose measurements using the One Touch II meter (Johnson & Johnson, Milpitas, CA). Mice with such overt clinical pre-diabetes were then injected in each quadricep with 0.05 ml of 0.25% bupivicaine-HCL (Sigma, St. Louis, MO). Two days later the mice were administered intramuscularly substantially endotoxin-free 0.10 ml of PBS, pBHT1 empty vector, mINS-II-pBHT1, or mINS-II-N-SSV at 250 ug/ml in PBS with 0.9 mM calcium in each quadricep for a total of 50 ug/animal. DNA injections were continued weekly thereafter for a total of 12 weeks. As a positive control anti-CD3 antibodies were administered at 5 ug/animal by IV injection for 5 consecutive days. Mice were tested weekly for glucosuria by Chemstrip (Boehringer Mannheim Co., Indianapolis, IN) and diabetes was confirmed by plasma glucose measurement using the One Touch II meter (Johnson & Johnson, Milpitas, CA). Progression to diabetes was defined as two consecutive blood glucose measurements greater than 250 mg/dl.

The results shown in Figure 5 reveal that DNA vaccination with the modified self-vector mINS-II-N-SSV reduced the development of diabetes in hyperglycemic NOD mice with less mice progressing to diabetes after 14 weeks compared to PBS injected controls and with those that did taking longer to progress compared to PBS (p=0.02) and pBHT1 empty vector injected controls. Furthermore, the treatment efficiency of mINS-II-N-SSV was also significantly greater than that of mINS-II-pBHT1 suggesting that preventing secretion of a self-polypeptide can further delay disease progression. Importantly, mINS-II-N-SSV treatment did not differ significantly from the anti-CD3 treated controls (p=0.13).

### Example 5

### Dosing of DNA vaccines for treatment of diabetes using modified self-vectors

This study investigated the preferred dosing of DNA vaccines using modified self-vectors. Weekly versus bi-weekly treatment with the high expression self-vector encoding preproinsulin (mINS-II-HESV) and the non-secreted self-vector encoding proinsulin II (mINS-II-N-SSV) was examined for efficacy in slowing diabetes progression in NOD mice.

Treatment of female NOD mice began at 10 weeks of age. Mice (n=20 per group) were injected in each quadricep with 0.05 ml of 0.25% bupivicaine-HCL (Sigma, St. Louis, MO). Two days later the mice were administered intramuscularly substantially endotoxin-free 0.10 ml of PBS, mINS-II-HESV, or mINS-II-N-SSV at 250 ug/ml in PBS with 0.9 mM calcium in each quadricep for a total of 50 ug/animal. DNA injections were continued thereafter either weekly or bi-weekly. Mice were tested weekly for glucosuria by Chemstrip (Boehringer Mannheim Co., Indianapolis, IN) and diabetes was confirmed by plasma glucose measurement using the One Touch II meter (Johnson & Johnson, Milpitas, CA). Progression to diabetes was defined as two consecutive blood glucose measurements greater than 250 mg/dl. DNA vaccination with both mINS-11-HESV and mINS-II-N-SSV revealed a trend towards delaying diabetes onset with both weekly and bi-weekly treatments (Fig. 7A-D).

### Example 6

### Treatment of established hyperglycemia in NOD mice by DNA vaccination using a non-secreted high expression self-vector (N-SHESV) encoding proinsulin II

This study investigated the effect of combined high expression and non-secretion modifications to the preproinsulin II DNA self-vector act on treatment efficacy of DNA vaccination to slow or prevent progression to diabetes. A non-secreted, high expression self-vector (N-SHESV) encoding proinsulin II was constructed, and this modified self-vector was used to vaccinate NOD mice with established hyperglycemia.

A N-SHESV was constructed that contains a β-globin/Ig chimeric intron downstream of the promoter region and upstream of the start codon of a non-secreted version of preproinsulin II, proinsulin II. The chimeric β-globin/IgG intron was isolated from pBHT520 as a 280 bp HindIII-XhoI fragment that was then cloned into mINS-II-N-SSV (pBHT555) between the CMV promoter and the coding region for non-secreted proinsulin to generate mINS-II-N-SHESV (pBHT568).

First it was demonstrated that the lack of the signal sequence in proinsulin II encoded by mINS-II-N-SHESV resulted in the production of an intracellular form of insulin. HEK293 cells were transfected with 2 ug of the insulin expressing plasmids mINS-II-pBHT1 (pBHT500) and mINS-II-N-SHESV (pBHT568). Transfected cells were incubated for 48 hours and insulin protein levels in the supernatant and cell lysates were analyzed at 48 hrs by ELISA (Fig. 6A). Significant amounts of protein were detected in the supernatant from mINS-II-pBHT1 transfected cells, but no protein was detected in the supernatant of cells transfected with mINS-II-N-SHESV. Alternatively transfected cells were incubated for 24 hrs in normal media and then for 24 hrs in the presence of the proteasome inhibitor lactacystin (5uM) to promote steady state levels of intracellular insulin. Insulin protein levels at 48 hrs were measured by ELISA (Fig. 6B). Again significant amounts of protein were detected in the supernatant from mINS-II-pBHT1 transfected cells, but no protein was detected in the supernatant of cells transfected with mINS-II-N-SHESV, rather the protein was confined to the cell lysate.

Next it was demonstrated that the removal of the signal sequence in preproinsulin II and the introduction of an intron to generate the high expression non-secreted self-vector mINS-II-N-SHESV (pBHT568) resulted in the production of an intracellular form of insulin compared with the unmodified self-vector mINS-II-pBHT1 (pBHT500) and with increased expression compared to mINS-11-N-SSV (pBHT555). HEK293 cells were transfected with 2 ug of the insulin expressing plasmids mINS-II-pBHT1 (pBHT500), mINS-II-N-SSV (pBHT555), and mINS-II-N-SHESV (pBHT568). Transfected cells were incubated for 48 hours and insulin protein levels in the supernatant and cell lysates were analyzed at 48 hrs by ELISA (Fig. 6A). Significant amounts of protein were detected in the supernatant from mINS-II-pBHT1 transfected cells, but no protein was detected in the supernatant of cells transfected with mINS-II-N-SHESV. Alternatively transfected cells were incubated for 24 hrs in normal media and then for 24 hrs in the presence of the proteasome inhibitor lactacystin (5uM) to promote steady state levels of intracellular insulin. Insulin protein levels at 48 hrs were measured by ELISA (Fig. 6B). Again significant amounts of protein were detected in the supernatant from mINS-II-pBHT1 transfected cells, but no protein was detected in the supernatant of cells transfected with mINS-II-N-SHESV. Furthermore, cells transfected with mINS-II-N-SHESV showed a 2-fold increase in protein expression compared to mINS-II-N-SSV lacking an intron in the cell lysate.

Treatment of female NOD mice began after the mice became hyperglycemic with blood glucose levels reaching 190-250 mg/dl (typically at 15-18 weeks of age) as determined by plasma glucose measurements using the One Touch II meter (Johnson & Johnson, Milpitas, CA). Mice with such overt clinical pre-diabetes were injected in each quadricep with 0.05 ml of 0.25% bupivicaine-HCL (Sigma, St. Louis, MO). Two days later the mice (n=15 per group) were administered intramuscularly substantially endotoxin-free 0.10 ml of PBS , mINS-II-N-SSV, or mINS-II-N-SHESV at 250 ug/ml in PBS with 0.9 mM calcium in each quadricep for a total of 50 ug/animal. DNA injections are continued weekly, every other week, or every four weeks for a total of 25 weeks. As a positive control anti-CD3 antibodies were administered at 5ug/animal by IV injection for 5 consecutive days. Mice are tested weekly for glucosuria by Chemstrip (Boehringer Mannheim Co., Indianapolis, IN) and diabetes confirmed by plasma glucose measurement using the One Touch II meter (Johnson & Johnson, Milpitas, CA). Progression to diabetes is defined as two consecutive blood glucose measurements greater than 250 mg/dl.

Treatment with either mINS-II-N-SSV or mINS-II-N-SHESV significantly delayed IDDM onset under all treatment regimens (Fig. 8A, B). Furthermore, the combination of high expression and non-secretion modifications in mINS-II-N-SHESV increased treatment efficiency of DNA vaccination compared to the single modification of mINS-II-N-SSV (Fig. 8A, B).

### Example 7

### Treatment of established hyperglycemia in NOD mice by DNA vaccination using combinations of modified DNA self-vectors encoding preproinsulin II

This study investigated whether combined high expression and non-secretion modifications to the preproinsulin II DNA self-vector act additively to effect treatment efficacy of DNA vaccination to slow or prevent progression to diabetes. A combination of: 1) a HESV containing a β-globin/Ig chimeric intron 5' the encoded preproinsulin II (mINS-II-HESV) and 2) a N-SSV encoding proinsulin II lacking the signal sequence of preproinsulin II (mINS-II-N-SSV) as described above were used to vaccinate NOD mice with established hyperglycemia.

Treatment of female NOD mice began after the mice became hyperglycemic with blood glucose levels reaching 190-250 mg/dl (typically at 15-18 weeks of age) as determined by plasma glucose measurements using the One Touch II meter (Johnson & Johnson, Milpitas, CA). Mice with such overt clinical pre-diabetes were injected in each quadricep with 0.05 ml of 0.25% bupivicaine-HCL (Sigma, St. Louis, MO). Two days later the mice were administered intramuscularly 0.10 ml of PBS, mINS-II-HESV, mINS-II-N-SSV, or a combination of mINS-II-HESV and mINS-II-N-SSV at 250 ug/ml in PBS with 0.9 mM calcium in each quadricep for a total of 50 ug/animal. DNA injections were continued weekly for a total of 25 weeks. Mice were tested weekly for glucosuria by Chemstrip (Boehringer Mannheim Co., Indianapolis, IN) and diabetes was confirmed by plasma glucose measurement using the One Touch II meter (Johnson & Johnson, Milpitas, CA). Progression to diabetes was defined as two consecutive blood glucose measurements greater than 250 mg/dl.

Vaccination with a combination of mINS-II-HESV and mINS-II-N-SSV self-vectors resulted in a significant reduction in disease progression compared to vaccination with mINS-II-HESV alone (Fig. 9).

### Example 8

### Prevention of autoantibody production and insulitis in NOD mice by DNA vaccination using modified DNA self-vectors encoding preproinsulin II

Prior to diabetes onset, NOD mice produce autoantibodies to insulin and display insulitus, the infiltration of lymphocytes into pancreatic islets of Langerhans. This study investigated whether DNA vaccination with modified self-vectors encoding preproinsulin II affectively inhibits the production of insulin autoantibodies and insulitis in NOD mice.

Female NOD mice were treated at 5 weeks of age before signs of hyperglycemia. Mice were injected in each quadricep with 0.05 ml of 0.25% bupivicaine-HCL (Sigma, St. Louis, MO). Two days later the mice were administered intramuscularly substantially endotoxin-free 0.10 ml of PBS, pBHT1 non-coding vector, mINS-I-pBHT1, mINS-II-pBHT1, mINS-II-HESV, or mINS-II-N-SSV at 250 ug/ml in PBS with 0.9 mM calcium in each quadricep for a total of 50 ug/animal. The plasmid DNA was injected weekly for a total of 6 weeks. Anti-CD3 was administered substantially endotoxin-free by IV injection (5ug/animal) for 5 consecutive days. Two weeks after the last DNA injection, sera was collected and screened in a blinded fashion for antibodies to insulin by radioimmunoassay at the Barbara Davis Center for Diabetes. A mouse insulin autoantibody (mIAA) index of greater than 0.01 was considered positive according to well established criteria (Eisenbarth, et al.). The percentage of animals with insulin autoantibodies is shown in Figure 10. Statistical analysis (Fisher's Exact Test) revealed a statistically significant reduction in the percentage of animals with autoantibodies to insulin in the mINS-II-N-SSV treated group (p=0.01) and the anti-CD3 treated group (p=0.01)

Also examined was the presence of insulitis in NOD mice immunized as described above. Two weeks after the last DNA injection the pancreas of each treated mouse was harvested and fixed in formalin. Sections were stained with H&E and scored in a blinded fashion for the extent of insulitis by an expert in IDDM at the Barbara Davis Center for Diabetes. Treatment with INS-II-N-SSV provided statistically significant reduction in insulitis compared to vector alone (Fig. 11).

### Example 9

### Prevention of IDDM in NOD mice by DNA vaccination using modified DNA self vectors encoding preproinsulin II

This study investigates whether DNA vaccination with modified self-vectors encoding preproinsulin II prevents development of hyperglycemia and diabetes in NOD mice.

Female NOD mice are treated at 5 weeks of age before signs of hyperglycemia. Mice are injected in each quadricep with 0.05 ml of 0.25% bupivicaine-HCL (Sigma, St. Louis, MO). Two days later the mice are administered intramuscularly with substantially endotoxin-free 0.10 ml of pBHT1 non-coding vector, mINS-II-HESV, mINS-II-N-SSV, or mINS-II-N-SHESV at 250 ug/ml in PBS with 0.9 mM calcium in each quadricep for a total of 50 ug/animal. The plasmid DNA is injected weekly for 6 weeks. Anti-CD3 antibodies are administered by IV injection (5 ug/animal) for 5 consecutive days. Mice are tested weekly for greater than 30 weeks for glucosuria by Chemstrip (Boehringer Mannheim Co., Indianapolis, IN) and diabetes is confirmed by plasma glucose measurement using the One Touch II meter (Johnson & Johnson, Milpitas, CA). Animals having repeated plasma glucose levels greater than 250 mg/dl are considered diabetic.

### Example 10

### Treatment of established hyperglycemia in NOD mice by DNA vaccination using a high expression self-vector encoding proinsulin II formulated with increasing Ca++ concentrations

This study investigated whether DNA vaccination with a high expression self-vector encoding proinsulin II formulated with increasing concentrations of Ca++ decreased the development of diabetes in NOD mice with established hyperlyceria.

Treatment of female NOD mice began after the mice became hyperglycemic with blood glucose levels reaching 190-250 mg/dl (typically at 15-18 weeks of age) as determined by plasma glucose measurements using the One Touch II meter (Johnson & Johnson, Milpitas, CA). Mice with overt clinical pre-diabetes were injected in each quadricep with 0.05 ml of 0.25% bupivicaine-HCL (Sigma, St. Louis, MO). Two days later the mice (n=5 per group) were administered intramuscularly 0.10 ml of PBS or mINS-II-HESV (pBHT-3021) at 250 ug/ml in PBS with different final Ca++ concentrations including: 0.9 mM (1x), 2.7 mM (3x) and 5.4 mM (6x) in each quadricep for a total of 50 ug/animal. DNA preparations (0.2 ml) were formulated at 0.25 mg/ml or 1.5 mg/ml with calcium chloride concentrations ranging from 0.9 mM (1X) to 8.1 mM (9X). Samples were placed at -20° C approximately one hour after formulation and left overnight at -20° C. The samples were thawed at room temperature prior to injection. Separate samples were spun for 5 minutes in an eppendorf microfuge (13,000 rpm). Supernatants were removed and the pellets were resuspended in Tris-EDTA (TE) and OD₂₆₀ readings were taken to determine the amount of DNA in the pellet. DNA injections were continued weekly for a total of 4 weeks. Mice were tested weekly for glucosuria by Chemstrip (Boehringer Mannheim Co., Indianapolis, IN) and diabetes was confirmed by plasma glucose measurement using the One Touch II meter (Johnson & Johnson, Milpitas, CA). Progression to diabetes was defined as two consecutive blood glucose measurements greater than 250 mg/dl.

Vaccination with a mINS-II-HESV formulated with 6x Ca++ resulted in a significant reduction in disease progression compared to vaccination with mINS-II-HESV formulated with 3x or 1x Ca++ (Fig. 12A). Similar results were obtained when insulin was co-administered (Fig. 12B). Furthermore, addition of Markane revealed a slight increase in efficacy at 3x and 6x calcium formulations (Fig. 12C). Composite results for diabetic progression with different calcium formulations with or without Markane are summarized in Figure 12D.

In addition to reducing disease progression, DNA vaccination with a higher calcium formulation also reduced the percentage of mice that obtained blood glucose (BG) levels over 600 mg/dl. Post-diabetes onset, mice were tested for plasma glucose levels using the One Touch II meter (Johnson & Johnson, Milpitas, CA). Mice vaccinated with pBHT-3021 in 6x calcium showed a significant delay and reduction of high blood glucose levels compared to mice treated with the self-vector in 1x calcium formulations, results that mimicked those obtained with an anti-CD3 positive control (Fig. 12E). A similar reduction in the percentage of mice with high blood glucose levels obtained with 6x calcium formulation was also achieved with a 5 day injection protocol of pBHT-3021 with 1x calcium (Fig. 12F). Furthermore, both 6x calcium and 1x calcium injected for 5 days resulted in a reversion of 1/5 of animals with high blood glucose levels to non-diabetic status as compared to no reversion when animals were treated with 1x calcium or PBS control (Fig. 12G). Thus formulation of self-vector plasmids with higher concentrations of calcium significantly increases efficacy of DNA vaccination and can substitute for more frequent dosing regimes.

### Example 11

### Physical Analysts of High Calcium Formulations

Given the increased efficacy of DNA vaccination with a self-vector formulated with higher calcium concentrations as disclosed in the previous example, a physical analysis of different calcium formulations was undertaken.

Dynamic light scattering (DLS) was used to evaluate the size of DNA aggregates when incubate at room temperature for 0-3 hours after formulation and after freeze/thaw. DNA samples of BHT-3021 (2 mg/mL) were stored at -80°C. DLS analysis was performed at two different DNA concentrations (0.25 and 1.5 mg/ml diluted in phosphate buffered saline) alone or in the presence of four different concentrations of calcium chloride (0.9, 3.0, 5.4 and 8.0 mM). The hydrodynamic diameter of the DNA samples was measured at 20°C using a light scattering instrument (Brookhaven Instruments Corp, Holtszille, NY) equipped with a 50 mW diode-pumped laser (532 nm) incident upon a sample cell immersed in a bath of decalin. The scattered light was monitored by a PMT (EMI 9863) at 90° to the incident beam, and the autocorrelation function was generated by a digital correlator (BI-9000AT). Data were collected continuously for five 30-seconds intervals for each sample and averaged. Data were analyzed by a variety of methods to yield information about the polydispersity of the preparation and the relative sizes of the various components present. The autocorrelation function was fit by the method of cumulants to yield the average diffusion coefficient of the DNA and/or complexes. The effective hydrodynamic diameter was obtained from the diffusion coefficient by the Stokes-Einstein equation. In addition, the data was fit to a non-negatively constrained least squares algorithm to yield multi-modal distributions. Also, for a more complete analysis, these methods were employed using a number average and an intensity average of the population.

Formulations of plasmid self-vector DNA with no or low (0.9 mM) calcium contain plasmid monomers exclusively with an average diameter of ~70 nM regardless of the time after formulation or whether the solution has been subjected to a freeze/thaw cycle (Tables 2-4). In contrast, at calcium concentrations of 3.0 mM and above, micron-sized particles formed within one hour and increase in size as the solution was incubated at room temperature for 2-3 hours after formulation (Tables 2-4). The size of particles increases with increasing calcium and increasing DNA concentration. After freezing the particles were too large to measure by DLS analysis.

**Table 2. Dynamic Light scattering of plasmid formulations at 0-1 hour after preparation. (Based on intesity average lognormal size distribution)**

| **Label** | **Description** | **Diameter (nm)** | **Polydispersity index** |
|---|---|---|---|
| BHT3021 #1 | 0.25 mq/mIBHT3021, no calcium Chloride | 68.7±0.6 | 0.223±0.015 |
| BHT3021 #2 | 0.25 mg/mlBHT3021, 0.9mM calcium Chloride | 69.5±0.7 | 0.217±0.017 |
| BHT3021 #3 | 0.25 mg/mlBHT3021, 3.0mM calcium Chloride | 839.5±56.9 | 0.258±0.043 |
| BHT3021 #4 | 0.25 mgImIBHT3021, 5.4mM calcium Chloride | 1093.1181.2 | 0.290±0.037 |
| But3021 #5 | 0.25 mg/mlBHT3021, 8.0mM calcium Chloride | 3054±0.321 | 0.372±0.073 |
| BHT3021 #6 | 1.5 mg/mlBHT3021. no calcium Chloride | 56.9±0.3 | 0.240±0.004 |
| BHT3021 #7 | 1.5 mg/mlBHT3021, 0.9mM calcium Chloride | 59.1±0.8 | 0.225±0.011 |
| BHT3021 #8 | 1.5 mg/mlBHT3021, 3.0mM calcium Chloride | 706±69.7 | 0.407±0.056 |
| BHT3021 #9 | 1.5 mg/mlBHT3021, 5.4mM calcium Chloride | 724±145 | 0.373±0.077 |
| BHT3021 #10 | 1.5 mg/mlBHT3021, 8.0mM calcium Chloride | 1932.4±135 | 0.288±0.082 |

**Table 3. Dynamic Light scattering of plasmid formulations at 2-3 hours after preparation. (Based on intesity average lognormal size distribution)**

| **Label** | **Description** | **(Diameter (nm)** | **Polydispersity index** |
|---|---|---|---|
| BHT3021 #1 | 0.25 mg/mlBHT3021, no calcium Chloride | 69.5±0.6 | 0.212±0.017 |
| BHT3021 #2 | 0.25 mg/mlBHT3021, 0.9mM calcium Chloride | 69.4±0.3 | 0.236±0.032 |
| BHT3021 #3 | 0.25 mg/mlBHT3021, 3.0mM calcium Chloride | 1219.6±97.7 | 0.307±0.077 |
| BHT3021 #4 | 0.25 mg/mlBHT3021, 5.4mM calcium Chloride | 1304.0±101.8 | 0.343±0.028 |
| BHT3021 #5 | 0.25 mg/mlBHT3021, 8.0mM calcium Chloride | Out of range | nd |
| BHT3021 #6 | 1.5 mg/mlBHT3021, no calcium Chloride | 55.3±0.5 | 0.238±0.004 |
| BHT3021 #7 | 1.5 mg/mlBHT3021, 0.9mM calcium Chloride | 57.1±0.3 | 0.238±0.008 |
| BHT3021 #8 | 1.5 mg/mlBHT3021, 3.0mM calcium Chloride | 1665.4±120.5 | 0.342±0.032 |
| BHT3021 #9 | 1.5 mg/mlBHT3021, 5.4mM calcium Chloride | 1328.2±191.6 | 0.372±0.021 |
| BHT3021 #10 | 1.5 mg/mlBHT3021, 8.0mM calcium Chloride | 1530.2±182.6 | 0.201±0.068 |

**Table 4. Dynamic Light scattering of plasmid formulations after overnight freeze-thaw cycle. (Based on intesity average lognormal size distribution)**

| **Label** | **Description** | **Diameter (nm)** | **Polydispersity index** |
|---|---|---|---|
| BHT3021 #1 | 0.25 mg/mlBHT3021, no calcium Chloride | 69.3±1.2 | 0.223±0.031 |
| BHT3021 #2 | 0.25 mg/mIBHT3021, 0.9mM calcium Chloride | 79.5±2.4 | 0.271±0.005 |
| BHT3021 #3 | 0.25 mg/mlBHT3021, 3.0mM calcium Chloride | out of range | nd |
| BHT3021 #4 | 0.25 mg/mlBHT3021, 5.4mM calcium Chloride | out of range | nd |
| BHT3021 #5 | 0.25 mg/mlBHT3021, 8.0mM calcium Chloride | out of range | nd |
| BHT3021 #6 | 1.5 mg/mlBHT3021, no calcium Chloride | 57.5±0.3 | 0.225±0.006 |
| BHT3021 #7 | 1.5 mg/mlBHT3021, 0.9mM calcium Chloride | 59.1±0.4 | 0.235±0.007 |
| BHT3021 #8 | 1.5 mg/mIBHT3021, 3.0mM calcium Chloride | out of range | nd |
| BHT3021 #9 | 1.5 mg/mlBHT3021, 5.4mM calcium Chloride | out of range | nd |
| RHT3021 #10 | 1.5 mg/mlBHT3021, 8.0mM calcium Chloride | out of range | nd |

Since the DLS analysis indicated the formation of micron-sized particles in the presence of calcium chloride concentration of 3.0 mM and up, a Coulter Multisizer 3 (Beckman Coulter Inc.) with an overall sizing range of 0.4-1200 µm was employed to perform an analysis of the aggregation state of DNA/Ca-phosphate complexes. The Multisizer 3 coulter counter offers ultra-high resolution; multiple channel analysis and accuracy; and its response is not affected by particle color, shape, density, composition, or refractive index. Particles suspended in buffer were drawn through a small aperture, separating two electrodes that have an electric current flowing between them. The voltage applied across the aperture created a "sensing zone" so that as particles passed through they displaced their own volume of electrolyte, momentarily increasing the impedance of the aperture. This change in impedance produced a tiny but proportional current flow into an amplifier that converted the current fluctuation into a voltage pulse large enough to be measured accurately. Analyzing this pulse enables a size distribution to be acquired and displayed. For the present experiments, a 200 µm aperture tube was used to detect sizes in the range of 4-120 µm and a 560 µm aperture was used to detect particles in the range of 120-336 µm. Solutions for large particle analysis were prepared by 3 different regimens: 4 degrees Celsius overnight before freezing at -20 degrees Celsius; freezing at -20 degrees Celsius within 15 minutes after formulation; and freezing at -20 degrees Celsius after a 4 hour incubation at room temperature. A normal distribution of particles was seen after freezing with an average diameter of approximately 25 µm for a DNA concentration of 0.25 mg/mL and 5.4 mM calcium chloride.

To determine the amount of DNA associated with large particles, centrifugation experiments were performed. Solutions containing varying ratios of DNA (0.25 to 2.0 mg/ml) and calcium (6X to 80X) were generated and frozen overnight at -20 degrees Celsius. After thawing, the solutions were centrifuged for five minutes in a microcentrifuge, and the supernatants were analyzed for DNA content by measuring absorption (260 nm) using a spectrophotometer. For the 6X calcium samples ~70% of the DNA is associated with particles that are precipitated from solution by a short centrifugation using a microfuge. The amount of DNA that is precipitated at varying concentrations of DNA and calcium are shown in Table 5 below. At DNA concentrations of 1.5-2.0 mg/ml only ~50% of the DNA can be precipitated.

**Table 5.**

| Sample | % DNA in supernatant |
|---|---|
| 0.25 mg/mL + 6X | 70% |
| 0.5 mg/mL + 6X | 57% |
| 0.5 mg/mL + 20X | 12% |
| 0.5 mg/mL + 40X | 35% |
| 1.0 mg/mL + 6X | 82% |
| 1.0 mg/mL + 20X | 25% |
| 1.0 mg/mL + 40X | 50% |
| 1.5 mg/mL + 6X | 85% |
| 1.5 mg/mL + 20X | 68% |
| 1.5 mg/mL + 40X | 60% |
| 2.0 mg/mL + 6X | 79% |
| 2.0 mg/mL + 24X | 56% |
| 2.0 mg/mL + 48X | 46% |
| 2.0 mg/mL + 80X | 50% |

### Example 12

### Treatment of established hyperglycemia in NOD mice by DNA vaccination using modified DNA self-vectors encoding preproinsulin II in combination with immune modulatory sequences (IMS)

This study investigates whether treatment efficiency of DNA vaccination with modified self-vectors encoding preproinsulin II can be further enhanced by co-administration of IMS. IMS 22-mer oligodeoxynucleotides (IMS-ODN) containing a single 5'-AAGGTT-3' sequence are chemically synthesized with a phosphorothioate backbone to protect against nuclease degradation. These IMS-ODN are then co-administered with modified self-vectors to NOD mice with established hyperglycemia.

Treatment of female NOD mice begins after the mice become hyperglycemic with blood glucose levels reaching 190-250 mg/dl (typically at 15-18 weeks of age) as determined by plasma glucose measurements using the One Touch II meter (Johhson & Johnson, Milpitas, CA). Mice with such overt clinical pre-diabetes are injected in each quadricep with 0.05 ml of 0.25% bupivicaine-HCL (Sigma, St. Louis, MO). Two days later the mice are administered intramuscularly substantially endotoxin-free 0.10 ml of pBHT1 non-coding vector, mINS-II-pBHTI, mINS-II-HESV, mINS-II-N-SSV, a combination of mINS-II-HESV and mINS-II-N-SSV, or mINS-II-N-SHESV at 250 ug/ml in PBS with 0.9 mM calcium in each quadricep for a total of 50 ug/animal. DNA injections are continued weekly for a total of 12 weeks. At the same time as initial DNA vaccination, 50 ug IMS in a volume of 200 ul PBS are administered intraperitoneally and reinjected weekly for 12 weeks. Mice are tested weekly for glucosuria by Chemstrip (Boehringer Mannheim Co., Indianapolis, IN) and diabetes is confirmed by plasma glucose measurement using the One Touch II meter (Johnson & Johnson, Milpitas, CA). Progression to diabetes is defined as two consecutive blood glucose measurements greater than 250 mg/dl and indicates whether co-administration of IMS affects treatment efficacy.

### Example 13

### Treatment of established hyperglycemia in NOD mice by DNA vaccination using modified DNA self-vectors encoding_preproinsulin II with added immunostimulatory sequences (ISS)

This study investigates whether treatment efficiency of DNA vaccination with modified self-vectors encoding preproinsulin II can be further enhanced by co-administration of immunostimulatory sequences (ISS). A cluster of ISS containing unmethylated CpG sequences according to the optimal sequence 5'-AACGTT-3' were added to a non-secreted self-vector encoding proinsulin and are added to a high expression vector encoding preproinsulin then used treat NOD mice with established hyperglycemia.

Modified self-vectors are generated that contain an increased number of mouse optimal stimulatory CpG elements in the vector backbone. A cluster of five mouse optimal CpG elements of the sequence AACGTT was generated by annealing a pair of phosphorylated oligonucleotides (sense strand— AGCTCAACGTTTCTAACGTTTTAACGTTTCCAACGTTTTAACGTTTC and antisense strand—GAAACGTTAAAACGTTGGAAACGTTAAAACGTTAGAAACGTTGAGCT). The annealed sequences were ligated into the NruI site of mINS-II-N-SSV (pBHT555) immediately upstream of the CMV promoter to generate mINS-II-N-SSV-CpG. Similarly the annealed sequences are ligated into the NruI site of mINS-II-HESV to generate mINS-II-HESV-CpG.

Treatment of female NOD mice begins after the mice become hyperglycemic with blood glucose levels reaching 190-250 mg/dl (typically at 15-18 weeks of age) as determined by plasma glucose measurements using the One Touch II meter (Johnson & Johnson, Milpitas, CA). Mice with such overt clinical pre-diabetes are injected in each quadricep with 0.05 ml of 0.25% bupivicaine-HCL (Sigma, St. Louis, MO). Two days later the mice are administered intramuscularly substantially endotoxin-free 0.10 ml of pBHT1 non-coding vector, mINS-II-HESV, mINS-II-HESV-CpG, mINS-II-N-SSV, or mINS-II-N-SSV-CpG at 250 ug/ml in PBS with 0.9 mM calcium in each quadricep for a total of 50 ug/animal. DNA injections are continued weekly for a total of 12 weeks. Mice are tested weekly for glucosuria by Chemstrip (Boehringer Mannheim Co., Indianapolis, IN) and diabetes is confirmed by plasma glucose measurement using the One Touch II meter (Johnson & Johnson, Milpitas, CA). Progression to diabetes is defined as two consecutive blood glucose measurements greater than 250 mg/dl and indicates the effect of added CpG immunostimulatory sequences on treatment efficacy.

### Example 14

### Prevention of IDDM in NOD mice by DNA vaccination using high expression DNA self-vectors encoding preproinsulin, glutamic acid decarboxylase, and tyrosine phosphatase IA-2

This study investigates whether combined DNA vaccination with modified self-vectors encoding multiple self-proteins associated with IDDM prevents hyperglycemia and progression to overt diabetes. In this particular embodiment HESVs encoding the self-proteins preproinsulin II as described above, glutamic acid decarboxylase (GAD)-65 and -67, and tyrosine phosphatase IA-2 are tested for the ability to prevent development of hyperglycemia and diabetes in NOD mice.

HESVs encoding GAD-65, GAD-67, IGRP and tyrosine phosphatase IA-2 are constructed. cDNAs encoding full-length murine GAD-65, GAD-67, and tyrosine phosphatase IA-2 are isolated by PCR from a mouse pancreas cDNA library and cloned into pBHT1 containing a chimeric β-globin/Ig intron downstream of the promoter region and 5' to the starter methionine of each amplified cDNA. The DNA is purified using Qiagen Endo-free Mega-preps (Qiagen, Valencia, CA). These modified self-vectors are then used to vaccinate NOD mice.

Female NOD mice are treated at 5 weeks of age before signs of hyperglycemia. Mice are injected in each quadricep with 0.05 ml of 0.25% bupivicaine-HCL (Sigma, St. Louis, MO). Two days later the mice are administered intramuscularly substantially endotoxin-free 0.10 ml of pBHT1 non-coding vector, pINS-II-HESV, a HESV encoding GAD-65, a HESV encoding GAD-67, a HESV encoding tyrosine phosphatase IA-2, or a combination of the HESVs at 250 ug/ml in PBS with 0.9 mM calcium in each quadricep for a total of 50 ug/animal. The plasmid DNA is injected weekly for 6 weeks. Mice are tested weekly for greater than 30 weeks for glucosuria by Chemstrip (Boehringer Mannheim Co., Indianapolis, IN) and diabetes is confirmed by plasma glucose measurement using the One Touch II meter (Johnson & Johnson, Milpitas, CA). Animals having repeated plasma glucose levels greater than 250 mg/dl are considered diabetic.

### Example 15

### Treatment of human IDDM by DNA vaccination using a high expression DNA self-vector encoding preproinsulin

This study examines whether DNA vaccination with a modified self-vector encoding and capable of expressing a self-polypeptide that includes one or more autoantigenic epitopes associated with IDDM can treat human IDDM. In this particular embodiment DNA vaccination with a HESV containing a chimeric β-globin/Ig intron 5' to the encoded preproinsulin is investigated. A full-length human cDNA encoding preproinsulin is isolated by PCR from human pancreas cDNA library (Stratagene, La Jolla, CA) and cloned into pBHT1 modified to include a chimeric β-globin/Ig intron downstream of the promoter region to generate a HESV adapted for administration to humans.

A therapeutically effective amount of HESV encoding preproinsulin is administered to a human patient diagnosed with IDDM. Therapeutically effective amounts of a self-vector are in the range of about 0.001 ug to about 1 g. A most preferred therapeutic amount of a self-vector is in the range of about 0.025 mg to about 5 mg. The DNA therapy is delivered monthly for 6-12 months, and then every 3-12 months as a maintenance dose. Alternative treatment regimens may be developed and may range from daily, to weekly, to every other month, to yearly, to a one-time administration depending upon the severity of the disease, the age of the patient, the self-protein(s), -polypeptide(s) or peptide(s) being administered and such other factors as would be considered by the ordinary treating physician.

In the preferred embodiment the DNA is delivered by intramuscular injection. Alternatively, the DNA self-vector is inhaled or delivered intranasally, orally, subcutaneously, intradermally, intravenously, impressed through the skin, or attached to gold particles delivered by gene gun to or through the dermis. The DNA is formulated in phosphate buffered saline with physiologic levels of calcium (0.9 mM). Alternatively the DNA is formulated in solutions containing higher quantities of Ca++, between 1 mM and 2M. The DNA may be formulated with other cations such as zinc, aluminum, and others.

Human diabetes patients treated with a HESV encoding preproinsulin are monitored for disease activity based on decreased requirement for exogenous insulin, alterations in serum autoantibody profiles, decreases in glycosuria, and decreases in diabetes complications such as cataracts, vascular insufficiency, arthropathy, and neuropathy.

### Example 16

### Selection and maintenance of a high expression self-vector (HESV) by repressor titration

Antibiotics and antibiotic resistance genes are the most commonly used markers for the selection and maintenance of recombinant DNA plasmids in host cells, including bacterial hosts such as *E. coli.* Yet their use in gene therapy in which plasmids are directly injected into a patient is discouraged in order to avoid the spread of antibiotic resistance traits by horizontal transfer. Thus we describe methods for alternative antibiotic-free selection and maintenance of a HESV of the present invention.

A HESV derived from the parent pBHT1 vector containing a chimeric β-globin/Ig intron and encoding human preproinsulin as described above is modified to allow for antibiotic-free selection and maintenance using repressor titration. The kanamycin resistance gene is removed from the HESV using flanking restriction enzyme sites present in the parent pBHT1 vector or added by site-directed mutagenesis with standard recombinant DNA techniques. A 66 basepair synthetic *E. coli lactose* (*lac*) operon dimer operator (Genbank Acc. Num. K02913) is ligated into the HESV using the same restriction sites to replace the kanamycin resistance gene. Alternatively a HESV containing a chimeric β-globin/Ig intron is first modified to replace the kanamycin resistance gene with the synthetic *lac* operon and then the human preproinsulin coding region is cloned downstream of the chimeric intron. A HESV modified to contain a *lac* operon sequence is referred to as a HESV*lacO*. The HESV*lacO* vector is then transformed into genetically modified *E. coli* that contain the *dapD* essential gene under the control of the *lac* promoter (*lacOP*) such as DH1*lacdapD* or DH1*lacP2dapD* as described (Cranenburgh et al., 2001). Repressor titration allows transformed *E. coli* cells to survive and thus the propagation of the HESV*lacO* plasmid.

### Example 17

### Treatment of human IDDM by DNA vaccination using a non-secreted DNA self-vector encoding proinsulin

This study examines whether DNA vaccination with a modified self-vector encoding and capable of expressing a non-secreted self-polypeptide that includes one or more secreted autoantigens associated with IDDM can treat human IDDM. In this particular embodiment DNA vaccination with a N-SSV encoding a non-secreted version of proinsulin lacking the signal sequence is investigated. A full-length human cDNA encoding proinsulin but lacking the signal sequence is isolated by PCR from a human pancreas cDNA library (Stratagene, La Jolla, CA) using a 5' primer containing an in-frame starter methionine to replace the start codon of the removed signal sequence. The PCR amplified product is digested and ligated into pBHT1 adapted for administration to humans.

A therapeutically effective amount of about 0.025 mg to about 5 mg of the N-SSV encoding proinsulin is administered intramuscularly to a human patient diagnosed with IDDM. The DNA therapy is delivered monthly for 6-12 months, and then every 3-12 months as a maintenance dose. Patients treated with the N-SSV encoding proinsulin are monitored for disease activity based on decreased requirement for exogenous insulin, alterations in serum autoantibody profiles, decreases in glycosuria, and decreases in diabetes complications such as cataracts, vascular insufficiency, arthropathy, and neuropathy.

### Example 18

### Selection and maintenance of a non-secreted self-vector (N-SSV) by repressor titration

This example describes methods for alternative antibiotic-free selection and maintenance of N-SSVs of the present invention. AN-SSV derived from parent pBHT1 vector encoding preproinsulin lacking the signal sequence, proinsulin, as described above is modified to allow for antibiotic-free selection and maintenance using repressor titration. The kanamycin resistance gene is removed from the N-SSV using flanking restriction enzyme sites present in the parent pBHT1 vector or added by site-directed mutagenesis with standard recombinant DNA techniques. A 66 basepair synthetic *E. coli lactose* (*lac*) operon dimer operator (Genbank Acc. Num. K02913) is ligated into the N-SSV using the same restriction sites to replace the kanamycin resistance gene. Alternatively the parent pBHT1 vector is first modified to replace the kanamycin resistance gene with the synthetic *lac* operon and then the human preproinsulin coding region lacking the signal sequence, proinsulin, is cloned downstream of the promoter. A N-SSV modified to contain a *lac* operon sequence is referred to as a N-SSV*lacO*. The N-SSV*lacO* vector is then transformed into genetically modified *E*. *coli* that contain the *dapD* essential gene under the control of the *lac* promoter (*laeOP*) such as DH1*lacdapD* or DH1*lacP2dapD* as described (Cranenburgh et al., 2001). Repressor titration allows transformed *E*. *coli* cells to survive and thus the propagation of the N-SSV*lacO* plasmid.

### Example 19

### Treatment of human IDDM by DNA vaccination using high expression and non-secreted DNA self-vectors encoding multiple self-polypeptides

This study examines whether DNA vaccination with modified self-vectors encoding and capable of expressing multiple self-polypeptides associated with IDDM can treat human IDDM. In this particular embodiment DNA vaccination with a combination of a N-SSV encoding proinsulin and HESVs encoding proinsulin as described above; glutamic acid decarboxylase (GAD) -65 and -67; tyrosine phosphatase IA-2; and islet cell antigen 69 kD is investigated. Full-length human cDNAs encoding GAD-65, GAD-67, tyrosine phosphatase IA-2, and islet cell antigen 65 kD are isolated by PCR from a human pancreas cDNA library (Stratagene, La Jolla, CA) and cloned into pBHT1 modified to include a chimeric β-globin/Ig intron downstream of the promoter region to generate a HESV adapted for administration to humans.

A therapeutically effective amount of a combination of the N-SSV encoding proinsulin and the HESVs encoding proinsulin, GAD-65, GAD-67, tyrosine phosphatase IA-2, and islet cell antigen 65 kD is administered intramuscularly to a human patient diagnosed with IDDM. DNA therapy is delivered monthly for 6-12 months, and then every 3-12 months as a maintenance dose. Patients such treated are monitored for disease activity based on decreased requirement for exogenous insulin, alterations in serum autoantibody profiles, decreases in glycosuria, and decreases in diabetes complications such as cataracts, vascular insufficiency, arthropathy, and neuropathy.

### Example 20

### Prevention of experimental autoimmune encephalomyelitis (EAE) in mice by DNA vaccination using modified DNA self-vectors encoding proteolipid protein (PLP)

Experimental autoimmune encephalomyelitis (EAE) is a mouse model of multiple sclerosis (MS), an inflammatory, demyelinating autoimmune disease of the central nervous system. This study is designed to investigate whether modified self-vectors encoding murine PLP can treat EAE. In this specific embodiment a self-vector encoding PLP is compared to a HESV encoding full-length PLP for the ability to prevent induction of EAE in susceptible mice.

To generate the self-vectors, full-length murine PLP is isolated by PCR amplification from a mouse pancreas cDNA library and cloned into pBHT1 with or without a chimeric β-globin/Ig intron 5' to the start codon. Mice are injected in each quadricep with 0.05 ml of 0.25% bupivicaine-HCL (Sigma, St. Louis, MO) and two days later injected again with 0.10 ml non-coding vector, a self-vector encoding PLP, or a HESV that contains a chimeric intron and encodes PLP at 250 ug/ml in PBS with 0.9 mM calcium. A second injection of DNA is given after one week. Ten days later mice are challenge with EAE induction.

EAE is induced in control and experimental mice with the injection of a peptide fragment of murine PLP, the 139-151 peptide, dissolved in PBS at 2 mg/ml and emulsified with an equal volume of Incomplete Freund's Adjuvant supplemented with 4 mg/ml heat-killed mycobacterium tuberculosis H37Ra (Difco Laboratories, Detroit, MI). Mice are injected subcutaneously with 0.1 ml of the peptide emulsion and, on the same day and 48 h later, intravenously with 0.1 ml of 4 µg/ml Bordetella Pertussis toxin in PBS. Animals are monitored and scored weekly for up to 20 weeks as follows: 0 = no clinical disease; 1 = tail weakness or paralysis; 2 = hind limb weakness; 3 = hind limb paralysis; 4 = forelimb weakness or paralysis; 5 = moribund or dead animal.

Following resolution of the acute phase of the clinical disease, control and experimental animals are sacrificed and lymph node cells (LNC) proliferative responses and cytokine production are examined. Draining LNC are restimulated *in vitro* with the PLP139-151 self peptide and their proliferation assessed by tritiated thymidine incorporation. To evaluate the levels of cytokine mRNA in inflamed brains, a ribonuclease protection assay on mRNA isolated from brain tissue is used. Values are normalized using expression levels of the housekeeping gene, GAPDH.

### Example 21

### Treatment of experimental autoimmune encephalomyelitis (EAE) in mice by DNA vaccination using modified DNA self-vectors encoding proteolipid protein (PLP) formulated with increasing Ca++ concentrations.

This study is designed to investigate whether DNA vaccination with a modified self-vector encoding murine PLP more effectively treats EAE in mice when formulated with increasing concentrations of Ca++.

EAE is induced in control and experimental SJL mice with the injection of a peptide fragment of murine PLP, the 139-151 peptide, dissolved in PBS at 2 mg/ml and emulsified with an equal volume of Incomplete Freund's Adjuvant supplemented with 4 mg/ml heat-killed mycobacterium tuberculosis H37Ra (Difco Laboratories, Detroit, MI). Mice are injected subcutaneously with 0.1 ml of the peptide emulsion and. Animals are monitored and scored starting on day 10 as follows: 0 = no clinical disease; 1 = tail weakness or paralysis; 2 = hind limb weakness; 3 = hind limb paralysis; 4 = forelimb weakness or paralysis; 5 = moribund or dead animal.

At peak disease, mice are randomized into different treatment groups. Mice are then injected in each quadricep with 0.05 ml of 0.25% bupivicaine-HCL (Sigma, St. Louis, MO) and two days later injected again with 0.10 ml PBS or pBHT-PLP at 250 ug/ml in PBS with different final Ca++ concentrations including: 0.9 mM (1x), 2.7 mM (3x) and 5.4 mM (6x) in each quadricep for a total of 50 ug/animal. Animals are then monitored for changes in mean disease scores as described above.

### Example 22

### Treatment of experimental autoimmune encephalomyelitis (RAE) in mice by DNA vaccination using a secreted self-vector (SSV) encoding myelin oligodendrocyte glycoprotein (MOG)

Experimental autoimmune encephalomyelitis (EAE) is a mouse model of multiple sclerosis (MS), an inflammatory, demyelinating autoimmune disease of the central nervous system. This study was designed to investigate whether modified self-vectors encoding murine MOG can treat EAE. In this specific embodiment a self-vector encoding MOG was compared to a modified self-vector encoding a soluble form of the extracellular region of MOG for the ability to treat EAE in susceptible mice.

A SSV encoding murine MOG was constructed that differs from a self-vector encoding full length MOG in that the extracellular region of MOG is secreted in a soluble form lacking the transmembrane and intracellular domains. The nucleotide sequence encoding the signal peptide and extracellular domain of murine MOG (MOG 1-154) was PCR amplified from the plasmid mMOG-pBHT1 (pBHT503) that contains the full length MOG coding sequence. The oligonucleotides used were smMOG.5.Eco-CATTGAATTCAAGATGGCCTGTTTGTGGAGC and smMOG.3.Xho - CAATTCTCGAGTCAACCGGGGTTGACCCAATAGAAG with the smMOG.3.Xho oligo providing a stop codon for the secreted MOG protein. The amplified fragment was cloned into the EcoRI-XoI sites between the CMV promoter and the BGH polyadenylation signal of pBHT1 to generate mMOG-SSV (pBHT516).

EAE was induced in susceptible mice by injection of a peptide fragment of murine MOG, the 35-55 peptide, dissolved in PBS at 2 mg/ml and emulsified with an equal volume of Incomplete Freund's Adjuvant supplemented with 4 mg/ml heat-killed mycobacterium tuberculosis H37Ra (Difco Laboratories, Detroit, MI). Mice were injected subcutaneously with 0.1 ml of the peptide emulsion and, on the same day and 48 h later, intravenously with 0.1 ml of 4 µg/ml Bordetella Pertussis toxin in PBS. Animals were monitored and scored daily for up to 14 weeks as follows: 0 = no clinical disease; 1 = tail weakness or paralysis; 2 = hind limb weakness; 3 = hind limb paralysis; 4 = forelimb weakness or paralysis; 5 = moribund or dead animal.

At day 16 mice were randomized into different treatment groups based on their disease score so that all groups had equal mean disease scores. Mice were injected in each quadricep with 0.05 ml of 0.25% bupivicaine-HCL (Sigma, St. Louis, MO) and two days later injected again with 0.10 ml PBS, mMOG-pBHT1, or mMOG-SSV at 250 ug/ml in PBS with 0.9 mM calcium in each quadricep for a total of 50 ug/animal. DNA injections were given bi-weekly for a total of five injections. As a positive control the steroid depromedrol was injected in MOG immunized mice at 1 mg/kg weekly.

At the conclusion of the study, the immune response of DNA vaccinated and control animals to the extracellular domain of MOG was examined. Sera from treated mice was collected and analyzed by ELISA for IgG1 anti-MOG specific antibodies.

The results shown in Figures 13 reveal that animals vaccinated with MOG self-vectors showed reductions in their mean disease scores compared to vehicle controls. Furthermore, animals vaccinated with mMOG-SSV had a lower mean disease score than animals vaccinated with the unmodified MOG self-vector. The presence of antibodies recognizing MOG paralleled the treatment response (Fig. 14). Animals treated with MOG self-vectors had lower mean optimal density ELISA scores than PBS injected controls with mMOG-SSV treated animals trending lower than animals treated with the unmodified MOG self-vector.

### Example 23

### Treatment of human multiple sclerosis (MS) by DNA vaccination using a high expression DNA self-vector encoding myelin basic protein (MBP)

This study examines whether DNA vaccination with a modified self-vector encoding and capable of expressing a self-polypeptide that includes one or more autoantigenic epitopes associated with MS can treat human MS. In this particular embodiment DNA vaccination with a HESV containing a β-globin/Ig chimeric intron 5' to the start codon of human MBP is used. Full-length human MBP cDNA is isolated by PCR amplification from a human brain cDNA library (Stratagene, La Jolla, CA). The PCR amplification product is digested with restriction enzymes and ligated into pBHT1 containing a β-globin/Ig chimeric intron 5' to the starter methionine to generate a HESV suitable for administration to humans.

Therapeutically effective amounts of about 0.025 mg to 5 mg of the HESV encoding MBP is administered intramuscularly to a human patient diagnosed with MS. The polynucleotide therapy is delivered monthly for 6-12 months, and then every 3-12 months as a maintenance dose. Human MS patients treated with HESVs encoding MBP are monitored for disease activity based on the number of clinical relapses and MRI monitoring for both the number of new gadolinium-enhancing lesions and the volume of the enhancing lesions.

### Example 24

### Treatment of human multiple sclerosis (MS) by DNA vaccination using a high expression DNA self-vector encoding combinations of myelin self-polypeptides

This study examines whether DNA vaccination with modified self-vectors encoding and capable of expressing multiple self-polypeptide associated with MS can treat human MS. In this particular embodiment DNA vaccination with HESVs encoding multiple myelin self-polypeptides is used. In one embodiment each myelin self-polypeptide is encoded in a distinct HESV. In another embodiment, several myelin self-polypeptides are encoded sequentially in a single HESV utilizing internal ribosomal re-entry sequences (IRESs) or other methods to express multiple proteins from a single plasmid DNA. Full-length human MBP, PLP, myelin-associated oligodendrocytic basic protein (MOBP), myelin oligodendrocyte glycoprotein (MOG), and myelin-associated glycoprotein (MAG) cDNAs are isolated by PCR from human brain cDNA library (Stratagene, La Jolla, CA) and cloned into pBHT1 containing a chimer β-globin/Ig intron 5' to the starter methionine to generate HESVs.

Therapeutically effective amounts of about 0.025 mg to 5 mg of HESVs encoding myelin-associated self-proteins are administered to a human patient diagnosed with MS. The polynucleotide therapy is delivered monthly for 6-12 months, and then every 3-12 months as a maintenance dose. Human MS patients are treated and monitored for disease activity based on the number of clinical relapses and MRI monitoring for both the number of new gadolinium-enhancing lesions and the volume of the enhancing lesions.

### Example 25

### Prevention and treatment of collagen-induced arthritis in mice by DNA vaccination using modified DNA self-vectors encoding type II collagen

Rheumatoid arthritis (RA) is a chronic autoimmune disease characterized by inflammatory synovitis that causes erosive joint destruction. RA is mediated by autoreactive T cells that recognize self-proteins present in synovial joints. Collagen-induced arthritis (CIA) in mice is a model of T cell-mediated autoimmunity that shares many features with rheumatoid arthritis including histologically similar synovitis and bony erosions. Furthermore, a relapsing model of CIA has clinical remissions and relapses of inflammatory erosive synovitis much like those observed in human RA patients (Malfait et al., 2000). CIA is induced by immunizing genetically susceptible mouse strains with type II collagen. This study is designed to investigate if modified self-vectors encoding full-length murine type II collagen is better able than a similar non-modified self-vector to prevent the development of CIA in mice.

A self-vector, HESV, N-SSV, and N-SHESV encoding type II collagen are constructed. Full-length type II collagen is isolated by PCR from a mouse cDNA library and cloned into pBHT1 alone or with a chimeric β-globin/Ig intron 5' to the start codon to generate a unmodified self-vector and a HESV, respectively. Type II collagen lacking the signal sequence is amplified from the pBHT1 vector encoding type II collagen using a 5' primer that contains an in-frame starter methionine and re-cloned into pBHT1 alone or with a chimeric β-globin/Ig intron to generate a N-SSV or a N-SHESV, respectively. Self-vectors encoding additional synovial self-proteins such as collagens type IV and IX may be similarly derived. DNA is purified using Qiagen Endo-free Mega-preps (Qiagen, Valencia, CA) according to the manufacturer's protocol.

Male DBA/1LacJ (H-2^{q}) mice between 6-9 weeks of age are used for this study. 100 ug of either unmodified self-vector or modified vectors encoding type II collagen are injected intramuscularly into the tibialis anterior muscle once every week for three weeks prior to the induction of disease or following onset of clinical CIA to treat relapsing CIA. After DNA vaccination, mice are challenged with CIA induction by intradermally injection at the base of the tail with 100 ug purified bovine type II collagen protein in complete Freund's adjuvant (CFA).

Injected mice are followed daily for 12 weeks for clinical evidence of CIA based on the visual scoring system: 0, no evidence of erythema and swelling; 1, erythema and mild swelling confined to the mid-foot (tarsals) or ankle joint; 2, erythema and mild swelling extending from the ankle to the mid-foot; 3, erythema and moderate swelling extending from the ankle to the metatarsal joints; and 4 erythema and severe swelling encompassing the ankle, foot and digits (Coligan et al., John Wiley and Sons, Inc 15.5.1-15.5.24, 1994). The clinical score for each animal is the sum of the visual score for each of its four paws. Histological analysis is performed on joints from mice that develop clinical arthritis. The first paw from the limb with the highest visual score is decalcified, sectioned, and stained with hematoxylin and eosin, and the stained sections are examined for lymphocytic infiltration, synovial hyperplasia, and erosions (Williams et al, 1994).

### Example 26

### Treatment of human rheumatoid arthritis (RA) and other autoimmune diseases targeting joints by DNA vaccination using modified DNA self-vectors encoding combinations of synovial self-polypeptides

This study examines whether DNA vaccination with modified self-vectors encoding and capable of expression self-polypeptides that includes one or more autoantigenic epitopes associated with RA can treat human RA. In particular embodiments DNA vaccination with combinations of N-SSVs encoding type II and type IV collagen proteins is envisioned. In other embodiments DNA vaccination with HESVs encoding BiP, gp39, and /or glucose-6-phosphate isomerase is used. Also envisioned is DNA vaccination using a combination of HESVs encoding BiP, gp39, and/or glucose-6-phosphate isomerase and N-SHESVs encoding type II collagen, type IV collagen, and/or fibrin. Human cDNAs for BiP, gp39, and glucose-6-phosphate are isolated by PCR amplification from a human cDNA library and cloned into a pBHT1 containing a chimeric β-globin/Ig intron upstream of the start codon of each encoded self-polypeptide. Human cDNAs for type II collagen, type IV collagen, and fibrin lacking signal sequences are isolated by PCR with a 5' primer containing an in-frame start codon and cloned into pBHT1 to generate N-SSVs or into a pBHT1 containing a chimeric β-globin/Ig intron to generate N-SHESVs.

Humans with new-onset or ongoing RA are diagnosed based on the American College of Rheumatology Criteria. Four out of 7 of the following criteria are required for diagnosis: (i) symmetrical polyarthritis, (ii) involvement of the MCPs, PIPs, or wrists, (iii) involvement of more than 3 different joint areas, (iv) joint erosions on X rays of hands or feet, (v) positive rheumatoid factor test, (iv) greater than 1 hour of morning stiffness, and (vii) nodules on extensor surfaces. Patients diagnosed with RA are treated with therapeutically effective amounts of about 0.025 mg to 5 mg of a combination of HESVs encoding BiP, gp39, and/or glucose-6-phosphate isomerase and/or N-SHESVs encoding type II collagen, type IV collagen, and/or fibrin. The polynucleotide therapy is delivered monthly for 6-12 months, and then every 3-12 months as a maintenance dose. The efficacy of the DNA therapy is monitored based on the fraction of patients with a reduction in their tender and swollen joint count by greater than 20% (an American College of Rheumatology 20% Response, ACR20), 50% (ACR50), and 70% (ACR70). Additional measures for human RA include reduction in inflammatory markers (including ESR and CRP), reduction in steroid usage, reduction in radiographic progression (including erosions and joint space narrowing), and improvement in disability status scores (such as the Health Assessment Questionnaire - HAQ). Changes in autoantibody titers and profiles are monitored. An identical approach can be used for related arthritides such as psoriatic arthritis, reactive arthritis, Reiter's syndrome, Ankylosing spondylitis, and polymyalgia rheumatica.

### Example 27

### Prevention and treatment of Myasthenia gravis in mice (rats) by DNA vaccination using modified DNA self-vectors encoding the nicotinic acetylcholine receptor alpha chain

This study examines whether DNA vaccination with modified self-vectors encoding the nicotinic acetylcholine receptor alpha chain (CHRNA1) can treat experimental autoimmune Myasthenia gravis (EAMG) in rats. Three distinct vectors are constructed using standard recombinant DNA technology to encode CHRNA1: a HESV, a N-SHESV and SHESV. To generate a HESV a full-length coding sequence of CHRNA1 is isolated by RT-PCR from a rat muscle library and ligated into pBHT520 containing a β-globin/Ig chimeric intron (rAchR-HESV). To generate a N-SHESV the extracellular domain (amino acids 21-230) of rat CHRNA1 lacking both the signal sequence and the transmembrane domains is isolated by RT-PCR and ligated into pBHT520 containing a β-globin/Ig chimeric intron (rAchR-N-SHESV). And finally to generate a SHESV the first 230 amino acids of the rat CHRNA1 is isolated by RT-PCR and ligated into pBHT520 containing a β-globin/Ig chimeric intron (rAchR-SHESV).

Myasthenia gravis is induced by immunizating rats with native muti-subunit acetylcholine receptor protein purified from the electric organs of the eel Torpedo californica mixed with complete Freund's adjuvant. Both prevention and treatment therapeutic regimens are employed. For disease prevention animals receive a minimum of 4 weekly injections of the modified self-vectors described above prior to immunization. For disease treatment animals receive weekly injections following immunization and at the beginning of disease onset. The muscle strength is assessed by the ability of rats to grasp and lift repeatedly a 300-g rack from the table while suspended manually by the base of the tail for 30 s. Animals are scored daily for clinical signs of disease based on the following scales:
0 normal;
1 no abnormalilites before testing, but reduced strength at the end;
2 clinical signs present before testing, i.e. tremor, head down, hunched posture, and weak grip;
3 severe clinical signs present before testing, no grip, and moribund
4 death
(Baggi et al., JI 172:2697, 2004) to examine the ability of DNA immunization with AchR alpha chain modified self-vectors to reduce and/or reverse disease severity.

### Sequence Listing

SEQ ID NO: 1
human proinsulin coding sequence
atggccctttgtgaaccaacacctgtgcggctcacacctggtggaagctctctacctagtgtg
cggggaacgaggcttcttctacacacccaagacccgccgggaggcagaggacctgcaggtgg
ggcaggtggagctgggcgggggccctggtgcaggcagcctgcagcccttggccctggagggg
tccctgcagaagcgtggcattgtggaacaatgctgtaccagcatctgctccctctaccagct
ggagaactactgcaactag

SEQ ID NO: 2
human proinsulin amino acid sequence
MAFVNQHLCGSHLVEALYLVCGERGFFYTPKTRREAEDLQVGQVELGGGPGAGSLQPLALEG
SLQKRGIVEQCCTSICSLYQLENYCN

## Claims

1. A high expression self-vector comprising in operative combination:
(a) a CMV promoter and early enhancer;
(b) a β-globin/Ig chimeric intron;
(c) a polynucleotide encoding one or more autoantigens targeted in insulin dependent diabetes mellitus (IDDM), said autoantigen being selected from insulin, proinsulin and preproinsulin;
(d) an immune modulatory sequence selected from the group consisting of 5'-Purine-Pyrimidine-[X]-[Y]-Pyrimidine-Pyrimidine-3' or 5'-Purine-Purine-[X]-[Y]-Pyrimidine-Pyrimidine-3',
wherein X and Y are any naturally occurring or synthetic nucleotide, except that X and Y cannot be cytosine-guanine; and
(e) a bovine growth hormone polyadenylation signal sequence and transcription terminator,
for use in a method of treating IDDM in a subject with clinical or diagnostic symptoms of IDDM.

2. A high expression self-vector for use according to claim 1, wherein the polynucleotide encodes proinsulin and results in the production of an intracellular form of insulin.

3. A high expression self-vector for use according to claim 1, wherein the polynucleotide encodes human proinsulin as shown in SEQ ID NO: 2.

4. A high expression self-vector for use according to any one of the preceding claims, wherein the high expression self-vector is formulated with calcium at a concentration between about 0.05 mM to about 2 M.

5. A high expression self-vector for use according to any one of the preceding claims, wherein the immune modulatory sequence is 5'-purine-pyrimidine-[X]-[Y]-pyrimidine-pyrimidine-3' and -[X]-[Y]- are GG.

6. A high expression self-vector for use according to any one of the preceding claims, wherein the immune modulatory sequence comprises a flanking sequence TGACTGTG and AGAGATGA 5' and 3' respectively.

7. A high expression self-vector for use according to any one of the preceding claims, wherein the subject is hyperglycemic.

8. A high expression self-vector for use according to any one of the preceding claims, wherein the high expression self-vector is formulated with a non-toxic inorganic base, a non-toxic organic base, phosphate buffered saline, a cationic polymer, (or a liposome), or is incorporated in a viral vector or a viral particle.

## Patentansprüche

1. Expressionsstarker Eigenvektor, der Folgendes in operativer Kombination umfasst:
(a) einen CMV-Promotor und einen frühen Enhancer;
(b) ein chimäres β-Globin/Ig-Intron,
(c) ein Polynucleotid, das für ein oder mehrere Autoantigene kodiert, die das Ziel bei insulinabhängigem Diabetes mellitus (IDDM) sind, wobei das Autoantigen aus Insulin, Proinsulin und Präproinsulin ausgewählt ist;
(d) eine immunmodulierende Sequenz, die aus der aus 5'-Purin-Pyrimidin-[X]-[Y]-Pyrimidin-Pyrimidin-3' und 5'-Purin-Purin-[X]-[Y]-Pyrimidin-Pyrimidin-3' ausgewählt ist, worin X und Y beliebige, natürlich vorkommende oder synthetische Nucleotide sind, mit der Ausnahme, dass X und Y nicht Cytosin-Guanin sein können; und
(e) eine(n) Rinderwachstumshormon-Polyadenylierungssignalsequenz und -Transkriptionsterminator,
zur Verwendung in einem Verfahren zur Behandlung von IDDM in einem Individuum mit klinischen oder diagnostischen IDDM-Symptomen.

2. Expressionsstarker Eigenvektor zur Verwendung nach Anspruch 1, wobei das Polynucleotid für Proinsulin kodiert und zur Produktion einer intrazellulären Form von Insulin führt.

3. Expressionsstarker Eigenvektor nach Anspruch 1, wobei das Polynucleotid für menschliches Proinsulin gemäß Seq.-ID Nr. 2 kodiert.

4. Expressionsstarker Eigenvektor zur Verwendung nach einem der vorangegangenen Ansprüche, wobei der expressionsstarke Eigenvektor zusammen mit Calcium in einer Konzentration zwischen etwa 0,05 mM bis etwa 2 M formuliert ist.

5. Expressionsstarker Eigenvektor nach einem der vorangegangenen Ansprüche, wobei die immunmodulierende Sequenz 5'-Purin-Pyrimidin-[X]-[Y]-Pyrimidin-Pyrimidin-3' ist und -[X]-[Y]- GG ist.

6. Expressionsstarker Eigenvektor zur Verwendung nach einem der vorangegangenen Ansprüche, wobei die immunmodulierende Sequenz die flankierenden 5'- bzw. 3'-Sequenzen TGACTGTG und AGAGATGA umfasst.

7. Expressionsstarker Eigenvektor zur Verwendung nach einem der vorangegangenen Ansprüche, wobei das Individuum hyperglykämisch ist.

8. Expressionsstarker Eigenvektor zur Verwendung nach einem der vorangegangenen Ansprüche, wobei der expressionsstarke Eigenvektor zusammen mit einer nichttoxischen anorganischen Base, einer nichttoxischen organischen Base, phosphatgepufferter Kochsalzlösung, einem kationischen Polymer oder einem Liposom formuliert ist oder in einen viralen Vektor oder ein Viruspartikel eingebaut ist.

## Revendications

1. Auto-vecteur d'expression élevée comprenant en combinaison fonctionnelle:
(a) un promoteur de CMV et un amplificateur précoce;
(b) un intron de β-globine/Ig chimérique;
(c) un polynucléotide codant pour un ou plusieurs auto-antigènes ciblés dans le diabète insulinodépendant (IDDM), ledit auto-antigène étant sélectionné parmi l'insuline, la proinsuline et la préproinsuline;
(d) une séquence modulatoire immune sélectionnée dans le groupe consistant en
5'-Purine-Pyrimidine-[X]-[Y]-Pyrimidine-Pyrimidine-3'
ou
5'-Purine-Purine-[X]-[Y]-Pyrimidine-Pyrimidine-3',
où X et Y sont n'importe quel nucléotide se produisant naturellement ou synthétique, excepté que X et Y ne peuvent pas être cytosine-guanidine; et
(c) une séquence de signaux de polyadénylation de l'hormone de croissance bovine et un terminateur de transcription,
destiné à être utilisé dans une méthode de traitement du IDDM chez un sujet présentant des symptômes cliniques ou diagnostics de IDDM.

2. Auto-vecteur d'expression élevée pour utilisation selon la revendication 1, où le polynucléotide code pour la proinsuline et se traduit par la production d'une forme intracellulaire d'insuline.

3. Auto-vecteur d'expression élevée pour utilisation selon la revendication 1, où le polynucléotide code pour la proinsuline humaine, comme représenté dans la SEQ ID NO: 2.

4. Auto-vecteur d'expression élevée pour utilisation selon l'une quelconque des revendications précédentes, où l'auto-vecteur d'expression élevée est formulée avec du calcium à une concentration entre environ 0,05 mM à environ 2 M.

5. Auto-vecteur d'expression élevée pour utilisation selon l'une quelconque des revendications précédentes, où la séquence modulatoire immune est 5'-purine-pyrmidine-[X]-[Y]-pyrimidine-pyrimidine-3' et [X]-[Y]- sont GG.

6. Auto-vecteur d'expression élevée pour utilisation selon l'une quelconque des revendications précédentes, où la séquence modulatoire immune comprend une séquence flanquante TGACTGTG et AGAGATGA 5' et 3' respectivement.

7. Auto-vecteur d'expression élevée pour utilisation selon l'une quelconque des revendications précédentes, où le sujet est hyperglycémique.

8. Auto-vecteur d'expression élevée pour utilisation selon l'une quelconque des revendication précédentes, où l'auto-vecteur d'expression élevée est formulé avec une base inorganique non toxique, une base organique non toxique, de l'eau salée tamponnée au phosphate, un polymère cationique ou un liposome, ou bien il est incorporé dans un vecteur viral ou une particule virale.
